(19) Europäisches Patentamt
European Patent Office
Office européen des brevets

(11) EP 4 755 372 A1

(12) **EUROPEAN PATENT APPLICATION**
published in accordance with Art. 153(4) EPC

(43) Date of publication:
**10.06.2026 Bulletin 2026/24**

(21) Application number: **24849160.7**

(22) Date of filing: **30.07.2024**

(51) International Patent Classification (IPC):
***A61K 9/10*** *(2006.01)* ***A61K 47/34*** *(2017.01)*
***A61K 51/12*** *(2006.01)* ***A61P 35/00*** *(2006.01)*
***A61K 103/34*** *(2006.01)*

(52) Cooperative Patent Classification (CPC):
**A61K 9/10; A61K 47/34; A61K 51/12; A61P 35/00;**
A61K 51/00

(86) International application number:
**PCT/JP2024/027098**

(87) International publication number:
**WO 2025/028509 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.07.2023 JP 2023124868**

(71) Applicant: **INSTITUTE OF SCIENCE TOKYO**
**Tokyo 152-8550 (JP)**

(72) Inventors:
- **MIURA Yutaka**
**Tokyo 152-8550 (JP)**
- **NISHIYAMA Nobuhiro**
**Tokyo 152-8550 (JP)**
- **GAO Shan**
**Tokyo 152-8550 (JP)**
- **OHNO Satoshi**
**Tokyo 152-8550 (JP)**
- **OGATA Keisuke**
**Tokyo 152-8550 (JP)**
- **ISHIDA Shota**
**Tokyo 152-8550 (JP)**
- **AOKI Ichio**
**Chiba-shi, Chiba 263-8555 (JP)**
- **OSADA Kensuke**
**Chiba-shi, Chiba 263-8555 (JP)**
- **SUMIYOSHI Akira**
**Chiba-shi, Chiba 263-8555 (JP)**

(74) Representative: **AWA Sweden AB**
**Box 5117**
**200 71 Malmö (SE)**

(54) **POLYMERIC MICELLE COMPRISING SINGLE POLYMER CHAIN OF SELF-AGGREGATING TYPE**

(57) The present invention addresses the problem that many conventional nano-sized contrast agents are aggregates of a plurality of constituent molecules and, under diluted conditions resulting from prolonged blood circulation, dissociate by reaching a critical micelle concentration (CMC) or critical aggregate concentration (CAC) or lower, such that it is difficult to control in vivo kinetics thereof up to the discharge phase. The present invention provides a polymeric micelle comprising a single polymer chain of a self-aggregating type, said single polymer chain containing one or more gadolinium complexes and having a particle size of not more than 10 nm in water.

Fig. 1

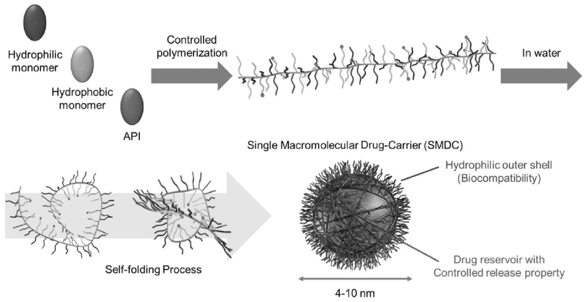


EP 4 755 372 A1

## Description

FIELD

The present invention relates to a novel polymeric micelle, and specifically it relates to a self-aggregating polymeric micelle formed by folding of a single polymer chain, as well as to their use and production for magnetic resonance imaging and radiation cancer treatment such as neutron capture therapy. The polymeric micelle of the invention can simultaneously maintain the positive contrast effect of gadolinium (Gd) complexes and their high longitudinal relaxivity during magnetic resonance imaging by self-aggregation of the single polymer chain, while also allowing treatment of tumors by radiation therapy such as neutron capture therapy.

BACKGROUND

Image diagnosis of tumors using magnetic resonance imaging (MRI) is an important method of diagnosis that allows visual contrast and noninvasive discernment of tumors in a patient's body [NPLs 1 and 2]. Contrast agents (CA), such as paramagnetic metal complexes, are generally useful for controlling the nuclear relaxation rate (relaxation time) of protons in water molecules in the longitudinal direction (T1) or transverse direction (T2), and it is known that polymers bonding to such metal complexes promote increase in rotational correlation time and augment molecular relaxivity [NPLs 3 and 4]. The additivity for changes in relaxation properties and the resulting relaxivity when introducing complexes into polymers have not been commonly established for current molecular designs, but introducing contrast agents into polymers is a commonly known method for enhancing image contrast [NPL 5].

In a drug delivery system (DDS) via systemic administration, nanosize DDS carriers are known to exhibit a stealth function allowing long-term blood circulation and selective accumulation in solid cancers [NPLs 6 and 7]. Most DDS carriers such as liposomes, polymer-type micelles and nanoparticles are also treated as effective carriers for delivery of contrast agents to cancer sites [NPLs 8 to 10]. Since the signal-to-noise ratio (S/N) in MRI depends on the balance between the concentration in blood and target tissue and the contrast ratio between lesions and normal tissue, it is extremely important to increase the S/N even when using a nanosize contrast agent (NCA) [NPL 11]. When an NCA is administered, therefore, control of both blood clearance and accumulation in the target tissue affects the precision of diagnosis by MRI. Diagnosis accuracy is also affected by the contrast ratio between lesions and normal tissue after accumulation in the target tissue, i.e. the positive contrast effect. However, most of the currently known nanosize contrast agents are aggregates of multiple structural molecules, and because they reach and dissociate below the critical micelle concentration (CMC) or critical aggregation concentration (CAC) under dilution conditions produced by prolonged blood circulation [NPLs 12 and 13], it is difficult to control their pharmacokinetic properties up to the point of their excretion. In addition to this, with most nanosize contrast agents the negative contrast effect produced by shortening of T2 in the body due to increased hydrophobicity and local magnetic field disturbance lowers the contrast ratio between lesions and normal tissue.

[CITATION LIST]

[NON PATENT LITERATURE]

[NPL 1] Stabile, A. et al., Nat. Rev. Urol., 17, 41-61 (2020)
[NPL 2] Sclafani, F. et al., Br. J. Cancer, 117, 1478-1485 (2017)
[NPL 3] Wahsner, J. et al., Chem. Rev., 119, 957-1057 (2018)
[NPL 4] Jeon, M. et al., Adv. Mater., 33, 1906539 (2021)
[NPL 5] Tang, J. et al., Prog. Polym. Sci., 38, 462-502 (2013)
[NPL 6] Wang, A. Z. et al., Annu. Rev. Med., 63, 185-198 (2012)
[NPL 7] Cheng, C. J. & Saltzman, W. M., Nat. Nanotechnol., 7, 346-347 (2012)
[NPL 8] Kanasty, R. et al., Nat. Mater., 12, 967-977 (2013)
[NPL 9] Nishiyama, N. et al., Cancer Sci., 107, 867-874 (2016)
[NPL 10] Cho, K. et al., Clin. Cancer Res., 14, 1310-1316 (2008)
[NPL 11] Versilst, P. et al., Chem. Soc. Rev., 44, 1791-1806 (2015)
[NPL 12] Cabral, H. et al., Nat. Nanotechnol., 6, 815-823 (2011)
[NPL 13] Lu, Y. et al., Nat. Biomed. Eng., 2, 318-325 (2018)


SUMMARY

[TECHNICAL PROBLEM]

No molecular designs have yet been reported which, for introduction of contrast agents into polymers, are able to improve relaxivity and further enhance the positive contrast effect. Most conventional nanosize contrast agents are aggregates of multiple structural molecules, and under dilution conditions resulting from prolonged blood circulation, this has been a problem in that dissociation occurs to below the critical micelle concentration (CMC) or critical aggregation concentration (CAC), making it difficult to control the pharmacokinetics up to the point of their excretion. It is an object of the present invention to solve this problem.

[SOLUTION TO PROBLEM]

Definitions

Unless otherwise specified, the terms used throughout the present specification have the same meanings as the technical and scientific terms commonly used by those skilled in the art. The terms defined below are used herein in the sense most suitable for describing the invention.

As used herein, the term "single polymer chain" refers to a polymer composed of a single-chain, such as a polymer having different monomer segments linked together, and the term may be used in the same sense as and interchangeable with "single polymer". According to the invention, the polymer of the invention spontaneously folds in water to form micelles with diameters of 10 nm or smaller.

Also as used herein, the term "association number" refers to the number of single polymer chains of the invention that form a single micelle in water. The term "association number" may also be used herein in the same sense as "cluster number". According to the invention, the DA (degree of association) value representing the degree of molecular association may be used as the association number of single polymer chains according to the invention. The association number can be calculated by $N_{agg} = M_{w,MALS}$ (PBS)/$M_{w,MALS}$ (chloroform), for example, as described below. DA = 1.0 represents ideal intramolecular association, i.e. SMDC formation, but according to the invention, SMDC is defined as DA < 1.5. A high DA (DA ≥ 1.5) represents multimolecular association. A low DA (DA < 1.0) also means SMDC formation in water, although the SMDC are reversed micelles in organic solvents.

As a result of much diligent research directed toward solving the problems described above, the present inventors have completed this invention upon finding that a polymer having a specified structure, upon self-aggregation, can maintain a positive contrast effect due to higher longitudinal relaxivity produced by restricted molecular motion of Gd complexes during magnetic resonance imaging, and simultaneously maintaining hydrophilicity during self-aggregation to avoid T2 shortening, and further finding that this allows tumors to be treated by neutron capture therapy.

Specifically, the aspects of the present invention include, but are not limited to, the following.

[1] A polymeric micelle composed of a self-aggregating single polymer chain, comprising one to several gadolinium complexes, and spontaneously folding in water to form a micelle with a diameter of 10 nm or smaller.

[2] The polymeric micelle composed of the self-aggregating single polymer chain according to [1], wherein:

(A) a side chain containing polyethylene glycol (PEG) or a PEG derivative;
(B) a side chain containing a hydrophobic group; and
(C) a side chain containing a gadolinium complex;

are bonded to the main backbone either via a linker or directly.

[3] The polymeric micelle composed of the self-aggregating single polymer chain according to [2], wherein

(D) a side chain containing a carboxylic acid group
is also bonded to the main backbone either via a linker or directly.

[4] The polymeric micelle composed of the self-aggregating single polymer chain according to [2] or [3], wherein the PEG or PEG derivative in the side chain (A) has the following structure:

[Chemical Formula 1]

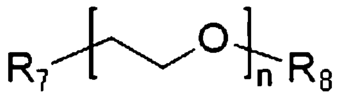

; or

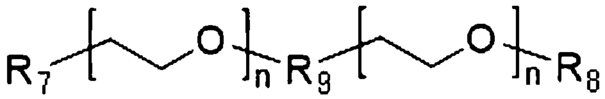

(where

$R_7$ is a methoxy, ethoxy, acetyl or benzyl group;
$R_8$ is an amide bond, ether bond or ester bond, being the portion that binds to the main backbone;
$R_9$ is a biodegradable linker; and
n is 1 to 10).

[5] The polymeric micelle composed of the self-aggregating single polymer chain according to any one of [2] to [4], wherein the hydrophobicity (cLogP value) of the hydrophobic groups in the side chain (B) is 1 to 10.
[6] The polymeric micelle composed of the self-aggregating single polymer chain according to any one of [2] to [5], wherein the hydrophobic group in the side chain (B) is selected from the group consisting of phenyl, naphthyl, pyrenyl, anthracene, cholesterol, steroid derivatives, 1-pyrenemethylamine, 1-ethyl-3-(3-dimethylaminopropyl)carbodiimide hydrochloride, 1-naphthylmethylamine, DL-$\alpha$-tocopherol, nerol, farnesol and anticancer agents.
[7] The polymeric micelle composed of the self-aggregating single polymer chain according to any one of [1] to [6], wherein the gadolinium complex is one or more selected from the group consisting of gadoteridol, meglumine gadoterate and gadobutrol.
[8] The polymeric micelle composed of the self-aggregating single polymer chain according to any one of [2] to [7], wherein when the numbers of repeating units of the side chains (A) to (D) in a single polymer chain are represented as a, b, c and d respectively, the following conditions are satisfied:

(I) when the cLogP value is less than 1.5 and the total degree of polymerization (DP) of a + b + c + d is 100 to 500,

a is 50% or greater of the total DP;
b is 40% or lower of the total DP;
c is 10% or lower of the total DP; and
d is 10% or lower of the total DP;

with the proviso that c and d make up $1\% \leq c + d \leq 20\%$ of the total DP;
(II) when the cLogP value is 1.5 or greater and less than 4.5 and the total degree of polymerization (DP) of a + b + c + d is less than 200,

a is 50% or greater of the total DP;
b is 30% or lower of the total DP;
c is 5% or lower of the total DP; and
d is 15% or lower of the total DP;

with the proviso that c and d make up $1\% \leq c + d \leq 20\%$ of the total DP;
(III) when the cLogP value is 1.5 or greater and less than 4.5 and the total degree of polymerization (DP) of a + b + c + d is 200 to 500,

a is 50% or greater of the total DP;
b is 40% or lower of the total DP;
c is 5% or lower of the total DP; and
d is 15% or lower of the total DP,

with the proviso that c and d make up $1\% \leq c + d \leq 20\%$ of the total DP;
(IV) when the cLogP value is 4.5 or greater and less than 5.5 and the total degree of polymerization (DP) of a + b + c

+ d is 200 to 500,

a is 50% or greater of the total DP;
b is 30% or lower of the total DP;
c is 5% or lower of the total DP; and
d is 15% or lower of the total DP, with the proviso that c and d make up 1% ≤ c + d ≤ 20% of the total DP; or

(V) when the cLogP value is 5.5 or greater and the total degree of polymerization (DP) of a + b + c + d is 200 to 500,

a is 60% or greater of the total DP;
b is 20% or lower of the total DP;
c is 2% or lower of the total DP; and
d is 8% or lower of the total DP, with the proviso that c and d make up 1% ≤ c + d ≤ 10% of the total DP.

[9] The polymeric micelle composed of the self-aggregating single polymer chain according to any one of [1] to [8], wherein the association number of the single polymer chain in water or buffer is less than 1.5, as calculated from the ratio of $M_{w,MALS}$ (PBS)/$M_{w,MALS}$ (chloroform).

[10] The polymeric micelle composed of self-aggregating single polymer chain according to any one of [1] to [9], wherein the main chain of the single polymer chain is selected from the group consisting of polyethers, polyesters, polyalkylenes, polycarbonates, poly(dioxanone), polyacid anhydrides, polyhydroxy acids, polyfumarate, polycaprolactone, polyamides, polyacetals, poly(orthoesters), polyhydroxybutyrate, polyvinyl alcohol, polyurethanes, polyphosphazenes, polyacrylates, polymethacrylates, polycyanoacrylates, polyureas, polystyrene, polyamines, poly(arylates), polycarbonates, poly(propylene fumarate), polyhydroxyalkanoates, polyketals, polyesteramides, polyhydroxyvalerates, polyorthocarbonates, poly(vinylpyrrolidone), polyalkylene oxalates, polyalkylene succinates, polylactic acid, poly(malic acid), poly(methylvinyl ether) and poly(maleic anhydride).

[11] The polymeric micelle composed of self-aggregating single polymer chain, comprising one to several gadolinium complexes, spontaneously folding in water to form micelles with diameters of 10 nm or smaller, and having the following structure:

[Chemical Formula 2]

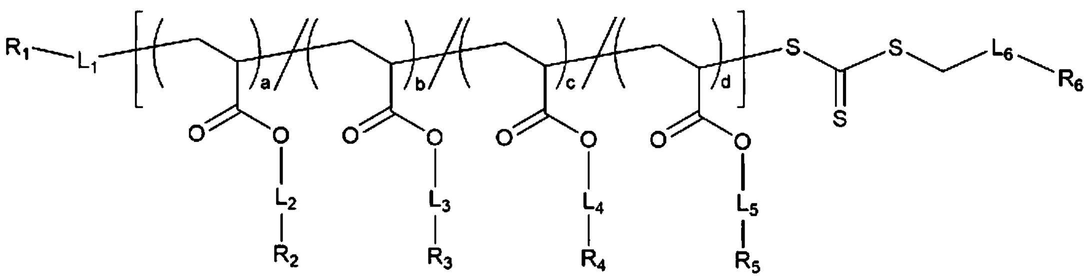

(1)

or

[Chemical Formula 3]

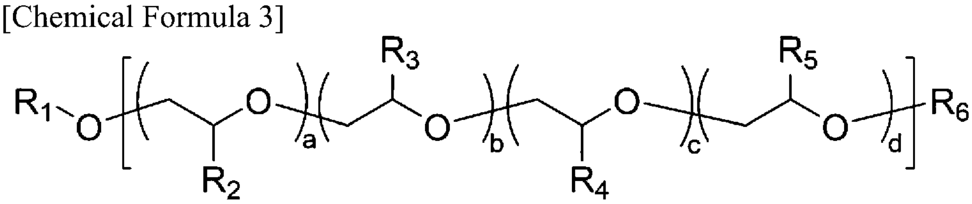

(2)

(where

$R_1$ and $R_6$ are each independently a methoxy, ethoxy, acetyl or benzyl group;
$R_2$ is PEG or a PEG derivative;
$R_3$ is a hydrophobic group;
$R_4$ is a gadolinium complex;

$R_5$ is a carboxylic acid group;
$L_1$ to $L_6$ are each independently not present or a linker; and
a, b, c and d satisfy any of the following conditions (I) to (V):

(I) when the cLogP value is less than 1.5 and the total degree of polymerization (DP) of a + b + c + d is 100 to 500,

a is 50% or greater of the total DP;
b is 40% or lower of the total DP;
c is 10% or lower of the total DP; and
d is 10% or lower of the total DP, with the proviso that c and d make up 1% ≤ c + d ≤ 20% of the total DP;

(II) when the cLogP value is 1.5 or greater and less than 4.5 and the total degree of polymerization (DP) of a + b + c + d is less than 200,

a is 50% or greater of the total DP;
b is 30% or lower of the total DP;
c is 5% or lower of the total DP; and
d is 15% or lower of the total DP, with the proviso that the c and d make up 1% ≤ c + d ≤ 20% of the total DP;

(III) when the cLogP value is 1.5 or greater and less than 4.5 and the total degree of polymerization (DP) of a + b + c + d is 200 to 500,

a is 50% or greater of the total DP;
b is 40% or lower of the total DP;
c is 5% or lower of the total DP; and
d is 15% or lower of the total DP, with the proviso that c and d make up 1% ≤ c + d ≤ 20% of the total DP;

(IV) when the cLogP value is 4.5 or greater and less than 5.5 and the total degree of polymerization (DP) of a + b + c + d is 200 to 500,

a is 50% or greater of the total DP;
b is 30% or lower of the total DP;
c is 5% or lower of the total DP; and
d is 15% or lower of the total DP, with the proviso that c and d make up 1% ≤ c + d ≤ 20% of the total DP;

(V) when the cLogP value is 5.5 or greater and the total degree of polymerization (DP) of a + b + c + d is 200 to 500,

a is 60% or greater of the total DP;
b is 20% or lower of the total DP;
c is 2% or lower of the total DP; and
d is 8% or lower of the total DP, with the proviso that c and d make up 1% ≤ c + d ≤ 10% of the total DP.

[12] The polymeric micelle composed of self-aggregating single polymer chain according to [11], wherein the PEG or PEG derivative may have, but is not limited to, the following structure:

[Chemical Formula 4]

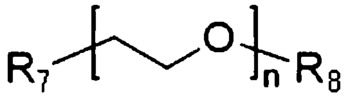

; or

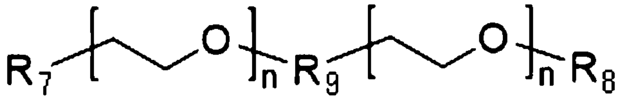

(where

$R_7$ is a methoxy, ethoxy, acetyl or benzyl group;
$R_8$ is an amide bond, ether bond or ester bond, being the portion that binds to the main backbone;
$R_9$ is a biodegradable linker; and
n is 1 to 10).

[13] The polymeric micelle composed of self-aggregating single polymer chain according to [11] or [12], wherein the hydrophobicity (cLogP value) of the hydrophobic groups in the side chain (B) is 1 to 10.
[14] The polymeric micelle composed of self-aggregating single polymer chain according to any one of [11] to [13], wherein the hydrophobic group is selected from the group consisting of phenyl, naphthyl, pyrenyl, anthracene, cholesterol, steroid derivatives, 1-pyrenemethylamine, 1-ethyl-3-(3-dimethylaminopropyl)carbodiimide hydrochloride, 1-naphthylmethylamine, DL-$\alpha$-tocopherol, nerol, farnesol and anticancer agents.
[15] The polymeric micelle composed of self-aggregating single polymer chain according to any one of [11] to [14], wherein the gadolinium complex is one or more selected from the group consisting of gadoteridol, meglumine gadoterate and gadobutrol.
[16] The polymeric micelle composed of self-aggregating single polymer chain according to any one of [11] to [15], wherein the association number of the single polymer chain in water or buffer is less than 1.5, as calculated from the ratio of $M_{w,MALS}$ (PBS)/$M_{w,MALS}$ (chloroform).
[17] The polymeric micelle composed of self-aggregating single polymer chain according to any one of [1] to [16], wherein when used in magnetic resonance imaging (MRI) for detection or diagnosis of cancer or a tumor, r1 is 20 $s^{-1}mM^{-1}$ or greater while maintaining longitudinal relaxivity (r1)/transverse relaxivity (r2) $\geq$ 0.70.
[18] A pharmaceutical composition for treatment of cancer or a tumor by neutron capture therapy, comprising polymeric micelles composed of self-aggregating single polymer chain according to any one of [1] to [16].

[ADVANTAGEOUS EFFECTS OF INVENTION]

In magnetic resonance imaging it becomes possible to maintain high longitudinal relaxivity of Gd complexes and simultaneously a positive contrast effect, and to carry out tumor treatment by neutron capture therapy.

BRIEF DESCRIPTION OF DRAWINGS

Fig. 1 shows an overview of formation of a Single-Molecular Drug Carrier (SMDC).

Fig. 2 shows the boundary for unimolecular association of poly-2a in aqueous solution. Shown are $N_{agg} \leq 1.0$ (black circles); $1.0 < N_{agg} \leq 1.5$ (white circles); $1.5 < N_{agg} \leq 2.0$ (black squares); $2.0 < N_{agg} \leq 5.0$ (black/white squares); $5.0 < N_{agg}$ (white squares); and x: $N_{agg}$ could not be determined, or precipitation caused by multimolecular association. The dotted line represents the threshold between intermolecular self-assembly and intramolecular self-assembly.

Fig. 3 shows the boundary for unimolecular association of poly-2b in aqueous solution. Shown are $N_{agg} \leq 1.0$ (black circles); $1.0 < N_{agg} \leq 1.5$ (white circles); $1.5 < N_{agg} \leq 2.0$ (black squares); $2.0 < N_{agg} \leq 5.0$ (black/white squares); $5.0 < N_{agg}$ (white squares); and x: $N_{agg}$ could not be determined, or precipitation caused by multimolecular association. The dotted line represents the threshold between intermolecular self-assembly and intramolecular self-assembly.

Fig. 4 shows the boundary for unimolecular association of poly-2c in aqueous solution. Shown are $N_{agg} \leq 1.0$ (black circles); $1.0 < N_{agg} \leq 1.5$ (white circles); $1.5 < N_{agg} \leq 2.0$ (black squares); $2.0 < N_{agg} \leq 5.0$ (black/white squares); $5.0 < N_{agg}$ (white squares); and x: $N_{agg}$ could not be determined, or precipitation caused by multimolecular association. The dotted line represents the threshold between intermolecular self-assembly and intramolecular self-assembly.

Fig. 5 shows the boundary for unimolecular association of poly-2d in aqueous solution. Shown are $N_{agg} \leq 1.0$ (black circles); $1.0 < N_{agg} \leq 1.5$ (white circles); $1.5 < N_{agg} \leq 2.0$ (black squares); $2.0 < N_{agg} \leq 5.0$ (black/white squares); $5.0 < N_{agg}$ (white squares); and x: $N_{agg}$ could not be determined, or precipitation caused by multimolecular association. The dotted line represents the threshold between intermolecular self-assembly and intramolecular self-assembly.

Fig. 6 shows the boundary for unimolecular association of poly-2e in aqueous solution. Shown are $N_{agg} \leq 1.0$ (black circles); $1.0 < N_{agg} \leq 1.5$ (white circles); $1.5 < N_{agg} \leq 2.0$ (black squares); $2.0 < N_{agg} \leq 5.0$ (black/white squares); 5.0 <

$N_{agg}$ (white squares); and x: $N_{agg}$ could not be determined, or precipitation caused by multimolecular association. The dotted line represents the threshold between intermolecular self-assembly and intramolecular self-assembly.

Fig. 7 shows the boundary for unimolecular association of poly-2f in aqueous solution. Shown are $N_{agg} \leq 1.0$ (black circles); $1.0 < N_{agg} \leq 1.5$ (white circles); $1.5 < N_{agg} \leq 2.0$ (black squares); $2.0 < N_{agg} \leq 5.0$ (black/white squares); $5.0 < N_{agg}$ (white squares); and x: $N_{agg}$ could not be determined, or precipitation caused by multimolecular association. The dotted line represents the threshold between intermolecular self-assembly and intramolecular self-assembly.

Fig. 8 shows the boundary for unimolecular association of poly-2g in aqueous solution. Shown are $N_{agg} \leq 1.0$ (black circles); $1.0 < N_{agg} \leq 1.5$ (white circles); $1.5 < N_{agg} \leq 2.0$ (black squares); $2.0 < N_{agg} \leq 5.0$ (black/white squares); $5.0 < N_{agg}$ (white squares); and x: $N_{agg}$ could not be determined, or precipitation caused by multimolecular association. The dotted line represents the threshold between intermolecular self-assembly and intramolecular self-assembly.

Fig. 9 shows an SEC chromatogram for TP1.

Fig. 10 shows the NMR spectrum for TP4.

Fig. 11 is a diagram for determining universal boundary conditions for unimolecular association of poly-2 in aqueous solution. $N_{agg} \leq 1.0$ (black spheres); $1.0 < N_{agg} \leq 1.5$ (gray spheres); $1.5 < N_{agg} \leq 2.0$ (black cubes); $2.0 < N_{agg} \leq 5.0$ (dark gray cubes); $5.0 < N_{agg}$ (light gray cubes). The curved surface represents the threshold between intermolecular self-assembly and intramolecular self-assembly.

Fig. 12 shows a Kratky plot in an aqueous solution.

Fig. 13 shows a Kratky plot in DMF.

Fig. 14 shows a TEM image of SMDC-$Gd_4$. The scale bar represents 100 nm.

Fig. 15 shows a TEM image of SMDC-$Gd_4$, with a scale bar of 100 nm.

Fig. 16 shows a particle size analysis example using DLS.

Fig. 17 shows zeta potential measurement.

Fig. 18 shows a comparison of blood retentivity.

Fig. 19 shows a comparison of tumor accumulation.

Fig. 20 shows a comparison of accumulation in the kidneys.

Fig. 21 shows a comparison of accumulation in the liver.

Fig. 22 shows a comparison of accumulation in the spleen.

Fig. 23 shows a comparison of accumulation in the brain.

Fig. 24 shows a comparison of accumulation in muscle.

Fig. 25 shows a comparison of accumulation in the pancreas.

Fig. 26 shows evaluation of cytotoxicity.

Fig. 27 shows changes in WBC after administration of SMDC-Gd.

Fig. 28 shows changes in RBC after administration of SMDC-Gd.

Fig. 29 shows changes in HGB after administration of SMDC-Gd.

Fig. 30 shows changes in HCT after administration of SMDC-Gd.

Fig. 31 shows changes in PLT after administration of SMDC-Gd.

Fig. 32 shows changes in BUN after administration of SMDC-Gd.

Fig. 33 shows changes in CRE after administration of SMDC-Gd.

Fig. 34 shows changes in GPT after administration of SMDC-Gd.

Fig. 35 shows changes in GOT after administration of SMDC-Gd.

Fig. 36 shows changes in ALP after administration of SMDC-Gd.

Fig. 37 shows changes in TP after administration of SMDC-Gd.

Fig. 38 shows time-dependent change in a quantitative mapping image of longitudinal relaxation rate (Gd conc. 0.1 mmol/kg).

Fig. 39 shows time-dependent change in the tumor/muscle ratio of the longitudinal relaxation rate (Gd conc. 0.1 mmol/kg).

Fig. 40 shows time-dependent change in the longitudinal relaxation rate in tumor (Gd conc. 0.1 mmol/kg).

Fig. 41 shows a quantitative mapping image of the longitudinal relaxation rate 24 hours after administration.

Fig. 42 shows time-dependent change in the longitudinal relaxation rate of a tumor 24 hours after administration.

Fig. 43 shows signal changes in bladder and kidney in a $T_1$-weighted image.

Fig. 44 shows time-dependent change in the MR signal for kidney in a $T_1$-weighted image.

Fig. 45 shows time-dependent change in the MR signal for bladder in a $T_1$-weighted image.

Fig. 46 shows a comparison of organ distribution with 3 administrations and single administration of SMDC-$Gd_4$.

Fig. 47 shows an organ distribution comparison after 3 administrations and single administration of Gd-DOTA.

Fig. 48 shows an antitumor effect by Gd-NCT.

Fig. 49 shows changes in weight after carrying out Gd-NCT.

DESCRIPTION OF EMBODIMENTS

The present invention relates to a self-aggregating polymeric micelle comprising a drug or a gadolinium complex. The polymeric micelle is characterized by folding of single polymer chain in water and can be used as MRI contrast agents or gadolinium formulations for neutron capture therapy, the invention providing a polymeric micelle composed of a single polymer chain with improved relaxivity due to folding of the single polymer chain. The present invention will now be explained in greater detail.

The polymeric micelle of the invention are random copolymers having on a side chain a hydrophilic group and a hydrophobic group as the driving force for folding, and gadolinium complexes functioning as MRI contrast agents and neutron capture therapy agents chemically bonded on the side chains. The polymers have particle diameters of 10 nm or smaller due to their self-folding ability in water, forming a single-molecular drug carrier (SMDC). (see Fig. 1).

Due to folding in water, the polymeric micelle have a form with shells composed of hydrophilic side chains and cores composed of hydrophobic side chains, enclosing the gadolinium complexes. This folding causes the gadolinium complexes to be present in a more crowded environment than when they are simply introduced into comb polymers. As a result, the polymeric micelle increase the rotational correlation time of the gadolinium complexes, functioning as a polymeric micelle that increase relaxivity.

The polymeric micelle exhibit a behavior similar to an EPR (Enhanced Permeability and Retention) effect, allowing delivery of a tumor-selective drug. In addition, since sizes of 10 nm and smaller can be precisely controlled, it is possible to adjust the clearance speed out of the body, such as by renal excretion. As a result, it is possible to improve tumor diagnosis, therapeutic effect and therapeutic advantages, and particularly to lower the background signal in a target organ or blood during use as a diagnostic agent such as a nano-contrast agent, thus allowing data to be obtained with a more satisfactory S/N ratio.

The polymeric micelle of the invention will now be explained in greater detail. The polymeric micelle of the invention comprise one to several (for example, 1 to 10) gadolinium complexes each, with the polymer single-chain folding in water to spontaneously form particles of 10 nm or smaller.

According to one embodiment, the polymer of the invention is a self-aggregating micelle having

(A) a side chain containing polyethylene glycol (PEG) or a PEG derivative;
(B) a side chain containing a hydrophobic group;
(C) a side chain containing a gadolinium complex; and
(D) a side chain containing a carboxylic acid group,

bonded to the main backbone either directly or via a linker.

The side chain (A) is the portion that is situated on the outer shell of the particle when a polymeric micelle has been formed, and that has a PEG or PEG derivative which is expected to inhibit biocompatibility and interaction with blood proteins, the PEG or PEG derivative having the following structure:

[Chemical Formula 5]

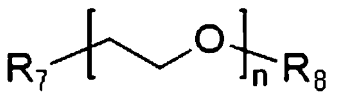

; or

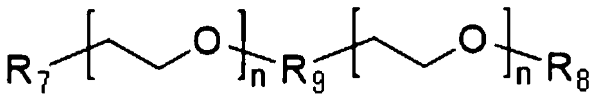

(where

$R_7$ is a methoxy, ethoxy, acetyl or benzyl group;
$R_8$ is an amide bond, ether bond or ester bond that binds to the main backbone;
$R_9$ is a biodegradable linker; and
n is 1 to 10).

The side chain (B) is the section with a hydrophobic group serving as the driving force for folding in water. The hydrophobicity (cLogP value) of the hydrophobic group of the side chain (B) is 1 to 10. Examples of hydrophobic groups include, but are not limited to, phenyl, naphthyl, pyrenyl, anthracene, cholesterol, steroid derivatives, 1-pyrenemethylamine, 1-ethyl-3-(3-dimethylaminopropyl)carbodiimide hydrochloride, 1-naphthylmethylamine, DL-$\alpha$-tocopherol, nerol, farnesol and anticancer agents.

The side chain (C) is the portion with a gadolinium complex that contributes to MR contrast or neutron capture therapy. Examples of gadolinium complexes include gadoteridol, meglumine gadoterate and gadobutrol.

The side chain (D) is composed of a portion with a carboxylic acid in order to provide an overall negative charge when the polymeric micelle has been formed.

The polymer of the invention is a structural material characterized by being controlled by the number of side chains (A) and the hydrophobicity (cLogP value) of the side chains (B) and also by the total number of repeating molecules of the side chains (A) to (D), and it has an association number of less than 1.5 in water or buffer solution.

The following conditions (I) to (V) are preferably satisfied, where a, b, c and d are the numbers of repeating units of side chains (A) to (D), respectively, in the single polymer chain.

(I) When the cLogP value is less than 1.5 and the total degree of polymerization (DP) of a + b + c + d is 100 to 500,

a is 50% or greater of the total DP;
b is 40% or lower of the total DP;

c is 10% or lower of the total DP; and
d is 10% or lower of the total DP; with the proviso that c and d make up 1% ≤ c + d ≤ 20% of the total DP.

(II) When the cLogP value is 1.5 or greater and less than 4.5 and the total degree of polymerization (DP) of a + b + c + d is less than 200,

a is 50% or greater of the total DP;
b is 30% or lower of the total DP;
c is 5% or lower of the total DP; and
d is 15% or lower of the total DP; with the proviso that c and d make up 1% ≤ c + d ≤ 20% of the total DP.

(III) When the cLogP value is 1.5 or greater and less than 4.5 and the total degree of polymerization (DP) of a + b + c + d is 200 to 500,

a is 50% or greater of the total DP;
b is 40% or lower of the total DP;
c is 5% or lower of the total DP; and
d is 15% or lower of the total DP; with the proviso that c and d make up 1% ≤ c + d ≤ 20% of the total DP.

(IV) When the cLogP value is 4.5 or greater and less than 5.5 and the total degree of polymerization (DP) of a + b + c + d is 200 to 500,

a is 50% or greater of the total DP;
b is 30% or lower of the total DP;
c is 5% or lower of the total DP; and
d is 15% or lower of the total DP; with the proviso that c and d make up 1% ≤ c + d ≤ 20% of the total DP.

(V) When the cLogP value is 5.5 or greater and the total degree of polymerization (DP) of a + b + c + d is 200 to 500,

a is 60% or greater of the total DP;
b is 20% or lower of the total DP;
c is 2% or lower of the total DP; and
d is 8% or lower of the total DP; with the proviso that c and d make up 1% ≤ c + d ≤ 10% of the total DP.

The polymeric micelle defined by any of the above has an association number of less than 1.5 for the single polymer chain in water or buffer.

The polymeric micelle of the invention is characterized by side chains (A) to (D) that are bonded to the main backbone either directly or via a linker, where the main backbone (main chain) is not particularly restricted and examples include polyethers, polyesters, polyalkylenes (such as polyethylene), polycarbonates, poly(dioxanone), polyacid anhydrides, polyhydroxy acids, polyfumarate, polycaprolactone, polyamides, polyacetals, poly(orthoesters), polyhydroxybutyrate, polyvinyl alcohol, polyurethanes, polyphosphazenes, polyacrylates, polymethacrylates, polycyanoacrylate, polyureas, polystyrene, polyamines, poly(arylates), polycarbonates, poly(propylene fumarate), polyhydroxyalkanoates, polyketals, polyesteramides, polyhydroxyvalerates, polyorthocarbonates, poly(vinylpyrrolidone), polyalkylene oxalates, polyalkylene succinates, polylactic acid, poly(malic acid), poly(methylvinyl ether) and poly(maleic anhydride). The polymer of the invention specified for this embodiment may be a single random copolymer molecule.

According to another embodiment, the polymer of the invention is a polymeric micelle comprising a gadolinium complex (optionally a drug) composed of a single random copolymer molecule, and while the main chain of the random copolymer is not restricted, it is preferably a backbone with high flexibility, such as a hydrocarbon-based or polyether-based backbone without unsaturated bonds, as in the following formula (1) or formula (2).

[Chemical Formula 6]

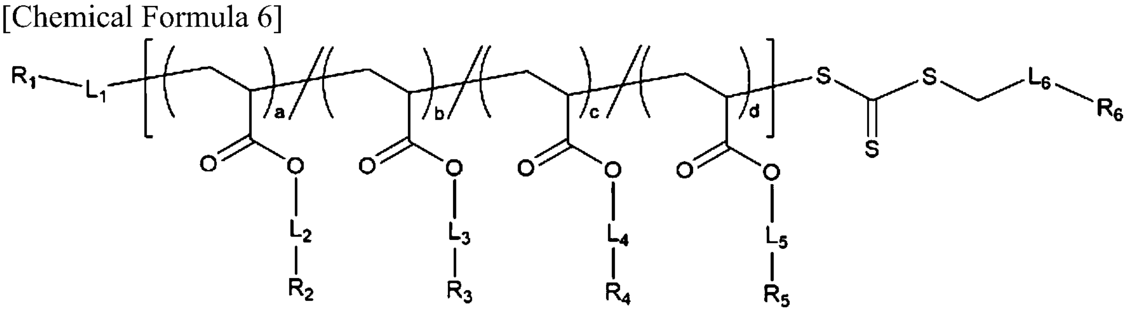

(1)

[Chemical Formula 7]

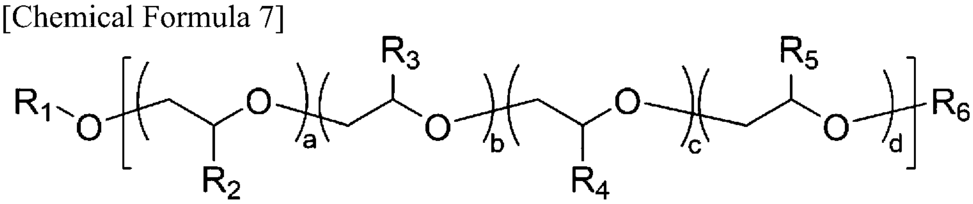

(2)

$R_1$ and $R_6$ are each independently a methoxy, ethoxy, acetyl or benzyl group.

$R_2$ is a portion having a PEG or PEG derivative that is situated on the outer shells of the particles when the polymeric micelle has been formed, and that is expected to inhibit biocompatibility and interaction between blood proteins. For example, the PEG or PEG derivative may have, but is not limited to, the following structure:

[Chemical Formula 8]

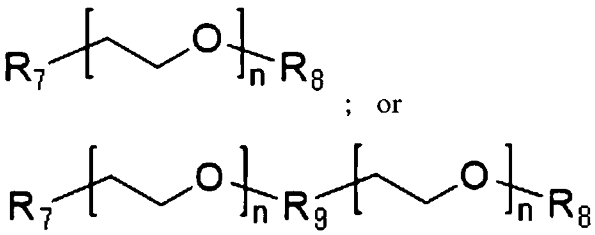

(where

$R_7$ is a methoxy, ethoxy, acetyl or benzyl group;
$R_8$ is an amide bond, ether bond or ester bond, being the portion that bonds to the main backbone;
$R_9$ is a biodegradable linker; and
n is 1 to 10).

$R_3$ is the portion with a hydrophobic group which acts as the driving force for folding in water.

$R_4$ is the portion with a gadolinium complex that contributes to MR contrast or neutron capture therapy.

$R_5$ is composed of a portion with an carboxylic acid in order to provide an overall negative charge when the polymeric micelle has been formed.

The polymer of the invention is a structural material characterized by being controlled by the number of $R_2$ groups, the hydrophobicity (cLogP value) of $R_3$, and the total number of repeating molecules of $R_2$ to $R_5$, and it has an association number of less than 1.5 in water or buffer solution.

According to one embodiment, when the cLogP value of $R_3$ in the polymer of the invention is less than 1.5 and the total degree of polymerization (DP) of a + b + c + d is 100 to 500,

a is 50% or greater of the total DP;
b is 40% or lower of the total DP;
c is 10% or lower of the total DP; and
d is 10% or lower of the total DP; with the proviso that c and d make up $1\% \leq c + d \leq 20\%$ of the total DP.

According to another embodiment, when $R_3$ in the polymer of the invention has a cLogP value of 1.5 or greater

and less than 4.5 and the total DP of a + b + c + d is less than 200,

a is 50% or greater of the total DP;
b is 30% or lower of the total DP;
c is 5% or lower of the total DP; and
d is 15% or lower of the total DP; with the proviso that c and d make up 1% ≤ c + d ≤ 20% of the total DP.

According to another embodiment, when $R_3$ in the polymer of the invention has a cLogP value of 1.5 or greater and less than 4.5 and the total DP of a + b + c + d is 200 to 500,

a is 50% or greater of the total DP;
b is 40% or lower of the total DP;
c is 5% or lower of the total DP; and
d is 15% or lower of the total DP; with the proviso that c and d make up 1% ≤ c + d ≤ 20% of the total DP.

According to yet another embodiment, when $R_3$ in the polymer of the invention has a cLogP value of 4.5 or greater and less than 5.5 and the total DP of a + b + c + d is 200 to 500,

a is 50% or greater of the total DP;
b is 30% or lower of the total DP;
c is 5% or lower of the total DP; and
d is 15% or lower of the total DP; with the proviso that c and d make up 1% ≤ c + d ≤ 20% of the total DP.

According to yet another embodiment, when $R_3$ in the polymer of the invention has a cLogP value of 5.5 or greater and the total DP of a + b + c + d is 200 to 500,

a is 60% or greater of the total DP;
b is less than 20% of the total DP;
c is less than 2% of the total DP; and
dis less than 8% of the total DP; with the proviso that c and d make up 1% ≤ c + d ≤ 10% of the total DP.

For all of the embodiments described above, $L_1$ to $L_6$ are each independently either not present or linkers. Examples of linkers include, but are not limited to, cleavable linkers and biodegradable linkers.

For all of the embodiments described above, when the polymeric micelle of the invention is used in magnetic resonance imaging (MRI) for detection or diagnosis of cancer or tumor, r1 is 20 $s^{-1}mM^{-1}$ or greater while maintaining longitudinal relaxivity (r1)/transverse relaxivity (r2) ≥ 0.70.

The polymeric micelle of the invention is characterized by having gadolinium complexes chemically bonded in the molecules, and the presence of a drug makes possible cancer or tumor diagnosis and/or treatment. Examples of cancer or tumors include, but are not limited to, neuroblastoma, liver cancer, malignant melanoma, uterine cancer, bladder cancer, biliary tract cancer, esophageal cancer, osteosarcoma, testicular cancer, thyroid cancer, acute myeloid leukemia, brain tumor, prostate cancer, pancreatic cancer, head and neck squamous cell carcinoma, mesothelioma, lung cancer, colorectal cancer, kidney cancer, ovarian cancer and breast cancer.

According to the invention there is provided a pharmaceutical composition for treatment of cancer or tumor by neutron capture therapy, comprising a polymeric micelle of the invention.

<Contrast agent>

The polymeric micelle of the invention may comprise at least one gadolinium contrast agent as shown in Table 1. Two or more gadolinium contrast agents may also be used in combination, but they preferably contain only one type.

[Table 1-1]

| Table 1. Gadolinium contrast agent | | | |
|---|---|---|---|
| Trade name (chemical name) | Structure | Uses | Chemical structure |
| Magnevist$^{R}$ (gadopentetic acid, Gd-DTPA) | Linear ionic | For MRI of central nervous system (brain, spinal cord, connective tissue) and extracranial and extraspinal tissue (head and neck) | 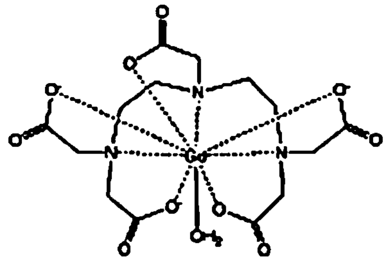 |
| MultiHanc$^{R}$ (gadobenic acid, Gd-BOPTA) | Linear ionic | For MRI of central nervous system, or obstructive vascular disease of kidney or aorta iliac femoral region | 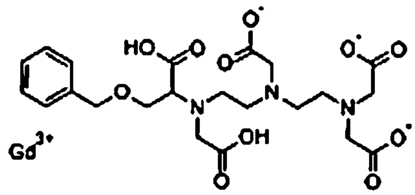 |
| Omniscan™ (gadodiamide, Gd-DTPA-BMA) | Linear nonionic | Used for MRI of lesions in central nervous system, thoracic cavity, abdominal cavity, cavity of pelvis and retroperitoneal space | 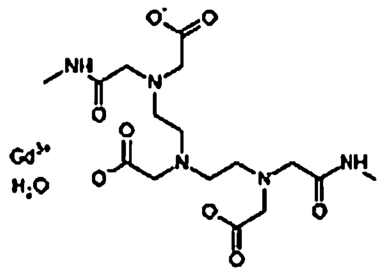 |
| Dotarem$^{R}$/Clariscan™ (gadoteric acid, Gd-DOTA) | Cyclic ionic | Used for MRI of central nervous system | 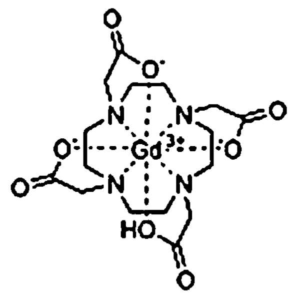 |

[Table 1-2]

| | | | |
|---|---|---|---|
| ProHance$^{R}$ (gadoteridol, Gd-BP-DO3A) | Cyclic nonionic | Used for MRI of central nervous system, extracranial and extraspinal tissue | 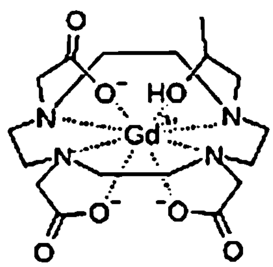 |

(continued)

| | | | |
|---|---|---|---|
| Gadavist$^R$ (gadobutrol, Gd-BT-DO3A) | Cyclic nonionic | Used for MRI of central nervous system, breast malignancy, supra-aortic artery disease and renal artery disease | 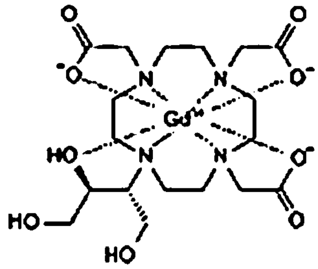 |
| Eovist$^R$/ Primovisth$^R$ (gadoxetic acid, Gd-EOB-DTPA) | Linear ionic | Used for MRI of liver | 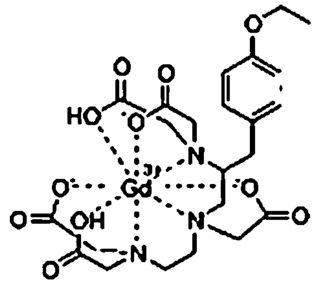 |
| Vueway™/ Elucirem™ (gadopiclenol) | Cyclic nonionic | Used for MRI of lesions in central nervous system and body (head and neck, thorax, abdominal region, pelvis and musculoskeletal system). | 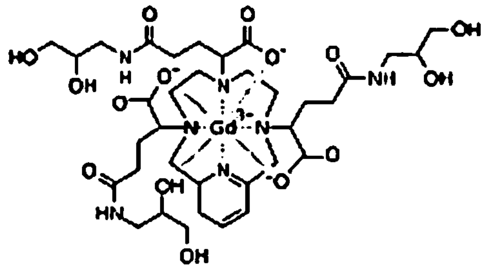 |

The polymeric micelle comprising the gadolinium contrast agent of the invention preferably has the following structure.

[Chemical Formula 9]

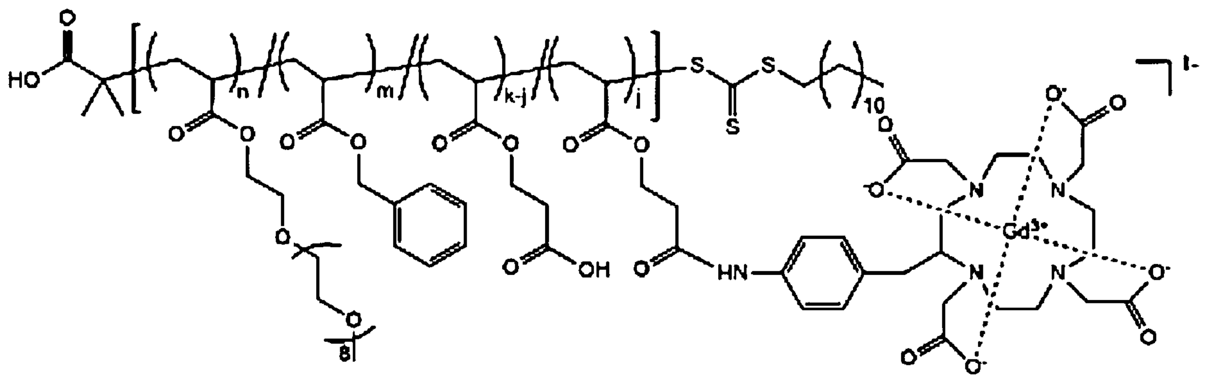

The polymeric micelle comprising the gadolinium contrast agent of the invention may contain a salt derived from an inorganic or organic acid and a base. Specific examples of salts include acetic acid salts, adipic acid salts, alginic acid salts, aspartic acid salts, benzoic acid salts, benzenesulfonic acid salts, bisulfuric acid salts, butyric acid salts, citric acid salts, camphoric acid salts, camphorsulfonic acid salts, cyclopentanepropionic acid salts, digluconic acid salts, dodecylsulfuric acid salts, ethanesulfonic acid salts, fumaric acid salts, glucoheptanoic acid salts, glycerophosphoric acid salts, hemisulfuric acid salts, heptanoic acid salts, hexanoic acid salts, hydrochlorides, hydrobromic acid salts, hydroiodic acid salts, 2-hydroxyethanesulfonic acid salts, lactic acid salts, maleic acid salt, methanesulfonic acid salts, 2-naphthalenesulfonic acid salts, nicotinic acid salts, oxalic acid salts, pamoates, pectinic acid salts, persulfuric acid salts, 3-phenylpropionic acid salts, picric acid salts, pivalic acid salts, propionic acid salts, succinic acid salts, tartaric acid salts, thiocyanic acid salts, tosylic acid salts, undecanoic acid salts, ammonium salts, alkali metal salts (such as sodium and potassium salts), alkaline earth metal salts (such as calcium, magnesium and zinc salts), salts with organic bases (such as dicyclohexylamine salts and N-methyl-D-glucamine), and salts with amino acids (such as arginine and lysine).

The polymeric micelle comprising the gadolinium contrast agent of the invention may comprise any carrier, adjuvant or vehicle. Carriers, adjuvants and vehicles that may be used in the polymeric micelle of the invention include, but are not limited to, ion-exchange substances, alumina, aluminum stearate, lecithin, serum proteins (such as human serum albumin), buffering substances (such as phosphates), glycine, sorbic acid, potassium sorbate, TRIS (tris(hydroxymethyl) aminomethane), partial glyceride mixtures of vegetable saturated fatty acids, water, salts or electrolytes (such as protamine sulfate, disodium hydrogen phosphate, potassium hydrogen phosphate, sodium chloride and zinc salts), colloidal silica, magnesium trisilicate, polyvinylpyrrolidone, cellulose-based substances, polyethylene glycol, carboxymethylcellulose sodium, polyacrylate, wax, polyethylene-polyoxypropylene-block copolymer, polyethylene glycol and lanolin.

The polymeric micelle comprising the gadolinium contrast agent of the invention may be in the form of an injectable aseptic compound medicine (for example, an injectable aseptic or oily suspension). Such a suspension can be formulated using a suitable dispersing agent and/or moistening agent and/or suspending agent, according to a technique known in the technical field. An injectable aseptic compound medicine may also be an aseptic injectable solution or suspension (for example, a solution in 1,3-butanediol) in a nontoxic diluent or solvent that can be received parenterally. Receivable vehicles and solvents that may be used are water, a linker solution and isotonic saline. Aseptic non-volatile oils are also used as solvents or suspension media. For this purpose, any non-irritating non-volatile oil that comprises a synthesized mono- or diglyceride may be used. Fatty acids (for example, oleic acid and its glyceride derivatives) are useful for preparation of injectable substances, and especially their polyoxyethylated forms, similar to natural pharmaceutically acceptable oils (such as olive oil or castor oils).

When the polymeric micelle comprising a gadolinium contrast agent of the invention are to be used as a contrast agent for MRI, the preferred route is parenteral administration, and specifically intravenous administration. When the polymeric micelle is to be intravenously administered, the dose concentration is preferably 5 mg/kg (body weight), for example.

The gadolinium contrast agent of the invention can also be utilized as a gadolinium formulation for neutron capture therapy for cancer or tumor. For a common general explanation of gadolinium formulations, see Ichikawa et al., "Frontiers: Creation of gadolinium formulation for neutron capture therapy for malignant tumors" (Pharmacia, vol. 56, No. 5, pp.396-400, 2020). Incidentally, it is demonstrated herein in Example 4 (Fig. 14) that the polymeric micelle of the invention can be used as neutron capture therapy.

The dosage (amount or concentration) for contrast depends on the sensitivity of the diagnostic imaging device and the composition of the contrast agent. For MRI imaging, for example, the gadolinium contrast agent of the invention will generally require a lower dosage than a contrast agent comprising a paramagnetic substance with a lower magnetic moment, such as iron(III). The dosage is preferably about 0.001 to 1 mmol/kg (body weight) per administration.

It should also be understood that specific dosage formulations for specific patients are dependent on a variety of factors (such as their age, body weight, health condition, gender and drug combinations, as well as the judgment of the attending physician).

According to the invention, MRI imaging is carried out after administration of an appropriate dosage of the gadolinium contrast agent of the invention. Selection of pulse sequence (inversion recovery (IR); spin echo (SE); echo planar imaging (EPI); time-of-flight (TOF); turbo flash; gradient echo (GE)) and imaging parameter values (echo time (TE); inversion time (TI); repetition time (TR); flip angle)

is governed by the required diagnostic information. When it is desired to obtain a T1-weighted image, TE should generally be less than 30 milliseconds (or the minimum) in order to maximize the T1 weighting. Conversely, when T2 measurement is desired, the TE should be greater than 30 milliseconds in order to minimize the competitive T1 effect. TI and TR are kept essentially the same for both T1 weighted and T2 weighted images. TI and TR are generally on the order of about 5 to 1000 milliseconds and 2 to 1000 milliseconds, respectively.

<Production Examples>

The method for producing the gadolinium contrast agent of the invention is as described below.

Synthesis of poly{[poly(ethylene glycol)monomethyl etheracrylate]-stat-(2-carboxyethyl acrylate)}(poly-1)

To a 10 mL volumetric flask in a glove box (Miwa Works, DBO-1.5KH-TSO, argon atmosphere) there were added poly(ethylene glycol)methyl etheracrylate (PEGA, ethylene glycol, mean number of repeats = 9) (3.01 g, 6.27 mmol, 55.8

equivalents), 2-carboxyethyl acrylate (CEA) (990 mg, 6.87 mmol, 61.1 equivalents), dehydrated/deoxygenated THF (6.3 mL), 2-(dodecylthiocarbonothioylthio)-2-methylpropionic acid (DDMAT) (41.0 mg, 112 μmol, 1.0 equivalents) and 2,2'-azobis(isobutyronitrile) (AIBN) (51.2 mM THF solution, 436 μL, 22.3 μmol, 0.2 equivalent). The flask was removed from the glove box, and after blowing argon gas into the reaction mixture for 30 minutes, the reactor was placed in an oil bath at 60°C and stirred for 2 hours. The reaction mixture was thawed with liquid nitrogen to suspend polymerization, and the reaction mixture was added dropwise into a large amount of ice-cooled diethyl ether. The precipitate was separated out and washed 3 times with ice-cooled diethyl ether, after which it was dissolved in methanol. The methanol was then completely removed by distillation under reduced pressure and vacuum drying, and the non-volatilizing components were redissolved in distilled water and freeze-dried to obtain 1.58 g of yellow viscous liquid poly-1-1.

The polymerization degree of each monomer and the number-average molecular weight ($M_{n,NMR}$) of the obtained copolymer were calculated by $^1$H-NMR, determining the polymerization degree of PEGA as 45, the polymerization degree of CEA as 65, and the $M_{n,NMR}$ as 31,000. The molecular weight dispersity ($M_w/M_n$) of the obtained copolymer was calculated by GPC to be 1.46.

[Chemical Formula 10]

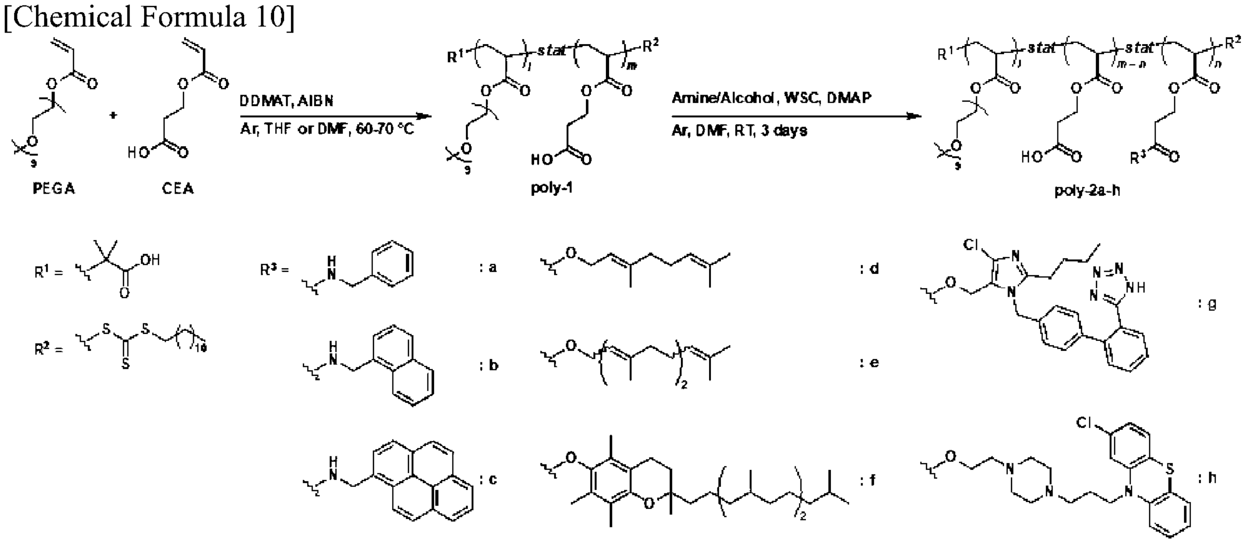


PEGA CEA poly-1 poly-2a-h

[Measuring apparatus and conditions]

(1) $^1$H-NMR measurement

Apparatus: Bruker Avance III (400 MHz)/Bruker
Solvent: 0.03% tetramethylsilane-containing chloroform-d/Kanto Chemical Co., Inc.
Measuring temperature: room temperature
Number of scans: 256
Results: δ(ppm) 4.71-4.18 ($-COOCH_2CH_2O-$, $COOCH_2CH_2COOH$), 3.88-3.32 ($-CH_2(OCH_2CH_2)_8CH_3$, $-CH_2(CH_2)_{10}CH_3$), 2.75-2.65 ($-COOCH_2CH_2COOH$), 2.32-1.64 (main chain), 1.30-1.09 (DDMAT), 0.88 (3H,- $CH_2(CH_2)_{10}CH_3$)

(2) GPC measurement

Apparatus: JASCO Extrema HPLC system/JASCO Corp. (CO-4060, AS-4050, PU-4180)
Detector: RI-4030 differential refractometer/JASCO Corp.
Column: TSKgel SuperAW-L guard column/Tosoh Corp. (4.6 mm × 35 mm), TSKgel SuperAW3000/Tosoh Corp. (6.0 mm × 150 mm; particle size, 4 μm), TSKgel SuperAW4000/Tosoh Corp. (6.0 mm × 150 mm; particle size, 6 μm)
Mobile phase: N,N-dimethylformamide (DMF) containing 50 mmol/L lithium bromide
Temperature: 40°C

Flow rate: 0.2 mL/min
Standard substance: Shodex STANDARD M-75/Resonac Corp.

The charging amounts, solvents, reaction temperatures and polymerization times for the reagents used with poly-1-1 (PEGA, CEA, DDMAT and AIBN) were changed as appropriate, and the same methods were used to synthesize polymers having different copolymer compositions and average molecular weights, as shown in the following tables.

[Table 2]

Table 2[a]

| Entry | $[PEGA]_0/[CEA]_0/[DDMAT]_0/[AIBN]_0$ | Solvent | Temperature (°C) | Time (h) |
|---|---|---|---|---|
| poly-1-1 | 56.3/61.1/1.0/0.2 | THF | 60 | 2 |
| poly-1-2 | 68.8/50.0/1.0/0.2 | THF | 60 | 2 |
| poly-1-3 | 50.0/66.7/1.0/0.2 | THF | 60 | 2 |
| poly-1-4 | 75.0/44.4/1.0/0.2 | THF | 60 | 2 |
| poly-1-5 | 62.5/55.6/1.0/0.2 | THF | 60 | 2 |
| poly-1-6 | 168.8/183.3/1.0/0.2 | THF | 60 | 2 |
| poly-1-7 | 225.0/133.3/1.0/0.2 | THF | 60 | 2 |
| poly-1-8 | 206.3/150.0/1.0/0.2 | THF | 60 | 2 |
| poly-1-9 | 87.5/33.3/1.0/0.2 | THF | 60 | 2 |
| poly-1-10 | 71.4/285.7/1.0.5 | DMF | 70 | 0.67 |
| poly-1-11 | 100.0/133.3/1.0/0.2 | THF | 60 | 2 |
| poly-1-12 | 35.7/321.4/1.0/0.5 | DMF | 70 | 0.67 |
| poly-1-13 | 187.5/166.7/1.0/0.2 | THF | 60 | 2 |
| poly-1-14 | 125.0/111.1/1.0/0.2 | THF | 60 | 2 |
| poly-1-15 | 262.5/100.0/1.0/0.2 | THF | 60 | 2 |
| poly-1-16 | 175.0/66.7/1.0/0.2 | THF | 60 | 2 |
| poly-1-17 | 250.0/222.2/1.0/0.2 | THF | 60 | 4 |
| poly-1-18 | 350.0/133.3/1.0/0.2 | THF | 60 | 4 |
| poly-1-19 | 112.5/122.2/1.0/0.2 | THF | 60 | 2 |
| poly-1-20 | 150.0/200.0/1.0/0.2 | THF | 60 | 2 |
| poly-1-21 | 137.5/100.0/1.0/0.2 | THF | 60 | 2 |
| poly-1-22 | 150.0/88.9/1.0/0.2 | THF | 60 | 2 |
| poly-1-23 | 200.0/266.7/1.0/0.2 | THF | 60 | 4 |
| poly-1-24 | 300.0/177.8/1.0/0.2 | THF | 60 | 4 |
| poly-1-25 | 75.0/44.4/1.0/0.2 | THF | 60 | 18 |
| poly-1-26 | 68.8/50.0/1.0/0.2 | THF | 60 | 18 |
| poly-1-27 | 62.5/55.6/1.0/0.2 | THF | 60 | 18 |
| poly-1-28 | 50.0/450.0/1.0/0.5 | DMF | 70 | 1 |
| poly-1-29 | 275.0/200.0/1.0/0.2 | THF | 60 | 4 |
| poly-1-30 | 225.0/244.4/1.0/0.2 | THF | 60 | 4 |
| poly-1-31 | 100.0/400.0/1.0/0.5 | DMF | 70 | 1 |
| poly-1-32 | 56.3/61.1/1.0/0.2 | THF | 60 | 18 |
| poly-1-33 | 87.5/33.3/1.0/0.2 | THF | 60 | 18 |
| poly-1-34 | 50.0/66.7/1.0/0.2 | THF | 60 | 18 |

[a] Argon atmosphere; [Monomer]o = 1.0 mol $L^{-1}$

[Table 3]

Table 3[a]

| Entry | $I/m_{obsd}$[b] | DP[b] | CEA (mol%)[b] | $M_{n,NMR}$ (g $mol^{-1}$)[b] | $M_{n,SEC}$ (g $mol^{-1}$)[c] | $M_{w,SEC}$ (g $mol^{-1}$)[c] | $M_w/M_n$[c] |
|---|---|---|---|---|---|---|---|
| poly-1-1 | 45/65 | 110 | 59 | 31,000 | 10,000 | 14,000 | 1.46 |
| poly-1-2 | 60/60 | 120 | 50 | 38,000 | 12,000 | 16,000 | 1.35 |

(continued)

Table 3[a]

| Entry | $I/m_{obsd}$[b] | DP[b] | CEA (mol%)[b] | $M_{n,NMR}$ (g $mol^{-1}$)[b] | $M_{n,SEC}$ (g $mol^{-1}$)[c] | $M_{w,SEC}$ (g $mol^{-1}$)[c] | $M_w/M_n$[c] |
|---|---|---|---|---|---|---|---|
| poly-1-3 | 60/100 | 160 | 63 | 44,000 | 10,000 | 15,000 | 1.46 |
| poly-1-4 | 90/80 | 170 | 47 | 55,000 | 13,000 | 18,000 | 1.32 |
| poly-1-5 | 80/90 | 170 | 53 | 52,000 | 12,000 | 16,000 | 1.34 |
| poly-1-6 | 65/105 | 170 | 62 | 47,000 | 14,000 | 20,000 | 1.42 |
| poly-1-7 | 95/80 | 175 | 46 | 57,000 | 14,000 | 22,000 | 1.55 |
| poly-1-8 | 90/90 | 180 | 50 | 57,000 | 14,000 | 22,000 | 1.50 |
| poly-1-9 | 140/70 | 210 | 33 | 78,000 | 11,000 | 18,000 | 1.61 |
| poly-1-10 | 40/170 | 210 | 81 | 44,000 | 23,000 | 33,000 | 1.40 |
| poly-1-11 | 80/140 | 220 | 64 | 59,000 | 12,000 | 18,000 | 1.48 |
| poly-1-12 | 20/205 | 225 | 91 | 40,000 | 21,000 | 29,000 | 1.42 |
| poly-1-13 | 105/125 | 230 | 54 | 69,000 | 13,000 | 22,000 | 1.65 |
| poly-1-14 | 120/135 | 255 | 53 | 77,000 | 12,000 | 18,000 | 1.53 |
| poly-1-15 | 185/95 | 280 | 34 | 103,000 | 15,000 | 26,000 | 1.70 |
| poly-1-16 | 195/90 | 285 | 32 | 107,000 | 14,000 | 21,000 | 1.53 |
| poly-1-17 | 130/155 | 285 | 54 | 85,000 | 16,000 | 24,000 | 1.49 |
| poly-1-18 | 190/100 | 290 | 34 | 106,000 | 17,000 | 27,000 | 1.62 |
| poly-1-19 | 120/170 | 290 | 59 | 82,000 | 12,000 | 18,000 | 1.53 |
| poly-1-20 | 105/190 | 295 | 64 | 78,000 | 15,000 | 21,000 | 1.46 |
| poly-1-21 | 155/145 | 300 | 48 | 96,000 | 13,000 | 20,000 | 1.56 |
| poly-1-22 | 180/135 | 315 | 43 | 106,000 | 14,000 | 20,000 | 1.51 |
| poly-1-23 | 110/210 | 320 | 66 | 83,000 | 14,000 | 22,000 | 1.58 |
| poly-1-24 | 180/150 | 330 | 45 | 108,000 | 17,000 | 30,000 | 1.74 |
| poly-1-25 | 210/140 | 350 | 40 | 121,000 | 10,000 | 18,000 | 1.68 |
| poly-1-26 | 200/150 | 350 | 43 | 118,000 | 9,000 | 16,000 | 1.72 |
| poly-1-27 | 190/180 | 370 | 49 | 118,000 | 10,000 | 16,000 | 1.70 |
| poly-1-28 | 35/345 | 380 | 90 | 67,000 | 22,000 | 33,000 | 1.50 |
| poly-1-29 | 190/200 | 390 | 51 | 120,000 | 17,000 | 26,000 | 1.57 |
| poly-1-30 | 160/235 | 395 | 59 | 111,000 | 15,000 | 24,000 | 1.61 |
| poly-1-31 | 80/335 | 415 | 81 | 87,000 | 24,000 | 38,000 | 1.56 |
| poly-1-32 | 200/230 | 430 | 53 | 130,000 | 9,000 | 16,000 | 1.67 |
| poly-1-33 | 320/130 | 450 | 29 | 173,000 | 10,000 | 18,000 | 1.68 |
| poly-1-34 | 200/270 | 470 | 57 | 135,000 | 9,000 | 15,000 | 1.66 |

[a] Argon atmosphere; Solvent: THF or DMF; Temperature: 60 or 70°C; $[\text{monomer}]_O$ = 1.0 mol $L^{-1}$
[b] Determined by $^1H$ NMR in $CDCl_3$
[c] Determined by SEC in DMF containing 0.01 M LiBr, with PMMA as standard

Synthesis of poly-2a-i

After adding poly-1-1 (183 mg, 5.90 μmol, 1.0 equivalents), 1-(3-dimethylaminopropyl)-3-ethylcarbodiimide hydrochloride (WSC) (140 mg, 732 μmol, 124 equivalents), DMF (4.74 mL), 4-dimethylaminopyridine (DMAP) (753 mM DMF solution, 760 μL, 572 μmol, 97 equivalents) and benzylamine (BnA) (2.02 M DMF solution, 284 μL, 575 μmol, 98 equivalents) to a test tube in a glove box and capping, the test tube was removed from the glove box and stirred for 3 days at room temperature. The reaction mixture was dialyzed in methanol to suspend the reaction, and then purified (MWCO = 3.5 kDa). The solution in the dialysis membrane was distilled off under reduced pressure and vacuum dried to completely remove the methanol. The non-volatilizing components were then redissolved in distilled water and freeze-dried to obtain 188 mg of yellow viscous liquid poly-2a-1.

The polymerization degree of each monomer unit and the number-average molecular weight ($M_{n,NMR}$) of the obtained copolymer were calculated by $^1$H-NMR, determining the polymerization degree of PEGA as 45, the polymerization degree of CEA as 20, the polymerization degree of CEA-BnA as 45, and the $M_{n,NMR}$ as 35,000.

[Measuring apparatus and conditions]

(1) $^1$H-NMR measurement

Apparatus: Bruker Avance III (400 MHz)/Bruker
Solvent: 0.03% tetramethylsilane-containing chloroform-d/Kanto Chemical Co., Inc.
Measuring temperature: room temperature
Number of scans: 256
Results: poly-2a$\delta$(ppm) 7.53-7.03 (Aromatic), 4.71-3.89 ($-COOCH_2CH_2O-$, $-COOCH_2CH_2CO-$, $-CONHCH_2Ph$), 3.87-3.37 ($-CH_2(OCH_2CH_2)_8CH_3$, $-CH_2(CH_2)_{10}CH_3$), 3.02-1.32 (main chain, $-COOCH_2CH_2CO-$), 1.26-1.10 (DDMAT), 0.90-0.86 ($-CH_2(CH_2)_{10}CH_3$)

Changing the reagent BnA used with poly-2-1 to 1-naphthylmethylamine, 1-pyrenemethylamine, 2-ethylhexylamine, nerol, farnesol, DL-$\alpha$-tocopherol, losartan or fluphenazine, and using the charging amounts of WSC and DMAP at 1.5 equivalents with respect to the carboxy groups in poly-1, the same method described above was used to synthesize polymers having different copolymer compositions and average molecular weights, as shown in the following tables.

[Table 4]

Table 4[a]

| Entry | Precursor | $l/m$ - $n/n_{obsd}$ [b] | DP [b] | Hydrophobics (mol%) [b] | $M_{n,NMR}$ (g mol$^{-1}$) [b] |
|---|---|---|---|---|---|
| poly-2a-1 | poly-1-1 | 45/20/45 | 110 | 41 | 35,000 |
| poly-2a-2 | poly-1-2 | 60/25/35 | 120 | 29 | 41,000 |
| poly-2a-3 | poly-1-3 | 60/45/55 | 160 | 35 | 49,000 |
| poly-2a-4 | poly-1-4 | 90/45/35 | 170 | 20 | 58,000 |
| poly-2a-5 | poly-1-5 | 80/55/35 | 170 | 22 | 55,000 |
| poly-2a-6 | poly-1-6 | 65/70/35 | 170 | 22 | 50,000 |
| poly-2a-7 | poly-1-9 | 140/45/25 | 210 | 12 | 80,000 |
| poly-2a-8 | poly-1-10 | 40/70/100 | 210 | 49 | 53,000 |
| poly-2a-9 | poly-1-11 | 80/65/75 | 220 | 35 | 66,000 |
| poly-2a-10 | poly-1-12 | 20/120/85 | 225 | 38 | 48,000 |
| poly-2a-11 | poly-1-13 | 105/80/45 | 230 | 20 | 73,000 |
| poly-2a-12 | poly-1-14 | 120/40/95 | 255 | 37 | 86,000 |
| poly-2a-13 | poly-1-16 | 195/40/50 | 285 | 17 | 111,000 |
| poly-2a-14 | poly-1-19 | 120/65/105 | 290 | 37 | 92,000 |
| poly-2a-15 | poly-1-20 | 105/115/75 | 295 | 25 | 85,000 |
| poly-2a-16 | poly-1-21 | 155/75/100 | 300 | 33 | 105,000 |
| poly-2a-17 | poly-1-22 | 180/55/80 | 315 | 25 | 113,000 |
| poly-2a-18 | poly-1-26 | 200/90/60 | 350 | 17 | 123,000 |
| poly-2a-19 | poly-1-28 | 35/195/150 | 380 | 40 | 80,000 |
| poly-2a-20 | poly-1-30 | 160/125/110 | 395 | 28 | 121,000 |
| poly-2a-21 | poly-1-31 | 80/160/175 | 415 | 42 | 103,000 |
| poly-2a-22 | poly-1-34 | 200/100/170 | 470 | 36 | 151,000 |

[a] Argon atmosphere; Solvent: DMF; Temperature: RT; $[\text{poly-1}]_O$ = 1.0 mmol$^{-1}$
[b] Determined by $^1$H NMR in $CDCl_3$

[Table 5]

Table 5[a]

| Entry | Precursor | $l/m$ - $n/n_{obsd}$ [b] | DP [b] | $F_{\text{Hydrophobic}}$ (mol%) [b] | $M_{n,NMR}$ (g mol$^{-1}$) [b] |
|---|---|---|---|---|---|
| poly-2b-1 | poly-1-1 | 45/30/35 | 110 | 33 | 36,000 |
| poly-2b-2 | poly-1-2 | 60/30/30 | 120 | 24 | 42,000 |

(continued)

Table 5[a]

| Entry | Precursor | $I/m$ - $n/n_{obsd}$ [b] | DP [b] | $F_{Hydrophobic}$ (mol%) [b] | $M_{n,NMR}$ (g mol$^{-1}$) [b] |
|---|---|---|---|---|---|
| poly-2b-3 | poly-1-3 | 60/35/65 | 160 | 40 | 53,000 |
| poly-2b-4 | poly-1-4 | 90/40/40 | 170 | 22 | 60,000 |
| poly-2b-5 | poly-1-5 | 80/50/40 | 170 | 22 | 57,000 |
| poly-2b-6 | poly-1-9 | 140/45/25 | 210 | 12 | 81,000 |
| poly-2b-7 | poly-1-10 | 40/70/100 | 210 | 48 | 58,000 |
| poly-2b-8 | poly-1-11 | 80/80/60 | 220 | 27 | 67,000 |
| poly-2b-9 | poly-1-11 | 80/40/100 | 220 | 45 | 73,000 |
| poly-2b-10 | poly-1-12 | 20/105/100 | 225 | 45 | 54,000 |
| poly-2b-11 | poly-1-13 | 105/50/75 | 230 | 33 | 80,000 |
| poly-2b-12 | poly-1-14 | 120/80/55 | 255 | 22 | 86,000 |
| poly-2b-13 | poly-1-14 | 120/35/100 | 255 | 39 | 91,000 |
| poly-2b-14 | poly-1-16 | 195/55/35 | 285 | 11 | 112,000 |
| poly-2b-15 | poly-1-17 | 130/75/80 | 285 | 28 | 96,000 |
| poly-2b-16 | poly-1-19 | 120/100/70 | 290 | 23 | 92,000 |
| poly-2b-17 | poly-1-20 | 105/50/140 | 295 | 47 | 98,000 |
| poly-2b-18 | poly-1-21 | 155/85/60 | 300 | 19 | 104,000 |
| poly-2b-19 | poly-1-22 | 180/80/55 | 315 | 18 | 114,000 |
| poly-2b-20 | poly-1-23 | 110/50/160 | 320 | 51 | 106,000 |
| poly-2b-21 | poly-1-25 | 210/45/95 | 350 | 28 | 135,000 |
| poly-2b-22 | poly-1-28 | 35/160/185 | 380 | 49 | 93,000 |
| poly-2b-23 | poly-1-30 | 160/85/150 | 395 | 38 | 132,000 |
| poly-2b-24 | poly-1-31 | 80/65/270 | 415 | 66 | 125,000 |
| poly-2b-25 | poly-1-32 | 200/65/165 | 430 | 39 | 153,000 |
| poly-2b-26 | poly-1-33 | 320/70/60 | 450 | 13 | 181,000 |
| poly-2b-27 | poly-1-34 | 200/55/215 | 470 | 45 | 165,000 |

[a] Argon atmosphere; Solvent: DMF; Temperature: RT; $[\text{poly-1}]_O$ = 1.0 mmol$^{-1}$
[b] Determined by $^1$H NMR in $CDCl_3$

[Table 6]

Table 6[a]

| Entry | Precursor | $I/m$ - $n/n_{obsd}$ [b] | DP [b] | $F_{Hydrophobic}$ (mol%) [b] | $M_{n,NMR}$ (g mol$^{-1}$) [b] |
|---|---|---|---|---|---|
| poly-2c-1 | poly-1-1 | 45/25/40 | 110 | 36 | 40,000 |
| poly-2c-2 | poly-1-2 | 60/25/35 | 120 | 30 | 46,000 |
| poly-2c-3 | poly-1-3 | 60/30/70 | 160 | 44 | 59,000 |
| poly-2c-4 | poly-1-4 | 90/65/45 | 170 | 25 | 65,000 |
| poly-2c-5 | poly-1-5 | 80/45/45 | 170 | 25 | 61,000 |
| poly-2c-6 | poly-1-9 | 140/25/45 | 210 | 22 | 88,000 |
| poly-2c-7 | poly-1-11 | 80/45/95 | 220 | 44 | 80,000 |
| poly-2c-8 | poly-1-14 | 120/50/85 | 255 | 33 | 95,000 |
| poly-2c-9 | poly-1-16 | 195/50/40 | 285 | 14 | 115,000 |
| poly-2c-10 | poly-1-19 | 120/45/125 | 290 | 43 | 109,000 |
| poly-2c-11 | poly-1-20 | 105/100/90 | 295 | 30 | 97,000 |
| poly-2c-12 | poly-1-21 | 155/40/105 | 300 | 34 | 118,000 |
| poly-2c-13 | poly-1-22 | 180/40/95 | 315 | 31 | 127,000 |
| poly-2c-14 | poly-1-23 | 110/15/195 | 320 | 60 | 97,000 |
| poly-2c-15 | poly-1-24 | 180/100/50 | 330 | 15 | 119,000 |
| poly-2c-16 | poly-1-25 | 210/90/50 | 350 | 15 | 133,000 |

(continued)

Table 6[a]

| Entry | Precursor | $I/m$ - $n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2c-17 | poly-1-27 | 190/120/60 | 370 | 16 | 130,000 |
| poly-2c-18 | poly-1-29 | 190/120/80 | 390 | 21 | 138,000 |
| poly-2c-19 | poly-1-30 | 160/30/205 | 395 | 52 | 162,000 |
| poly-2c-20 | poly-1-32 | 200/35/195 | 430 | 46 | 133,000 |
| poly-2c-21 | poly-1-33 | 320/95/35 | 450 | 7 | 180,000 |
| poly-2c-22 | poly-1-34 | 200/150/120 | 470 | 26 | 162,000 |

[a] Argon atmosphere; Solvent: DMF; Temperature: RT; $[poly-1]_O$ = 1.0 $mmol^{-1}$
[b] Determined by $^1H$ NMR in $CDCl_3$

[Table 7]

Table 7[a]

| Entry | Precursor | $I/m$ - $n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2d-1 | poly-1-1 | 45/30/35 | 110 | 30 | 35,000 |
| poly-2d-2 | poly-1-2 | 60/35/25 | 120 | 21 | 41,000 |
| poly-2d-3 | poly-1-3 | 60/40/60 | 160 | 36 | 50,000 |
| poly-2d-4 | poly-1-4 | 90/35/45 | 170 | 27 | 60,000 |
| poly-2d-5 | poly-1-5 | 80/40/50 | 170 | 30 | 58,000 |
| poly-2d-6 | poly-1-8 | 90/45/45 | 180 | 25 | 62,000 |
| poly-2d-7 | poly-1-9 | 140/35/35 | 210 | 16 | 81,000 |
| poly-2d-8 | poly-1-10 | 40/5/165 | 210 | 77 | 62,000 |
| poly-2d-9 | poly-1-11 | 80/60/80 | 220 | 37 | 68,000 |
| poly-2d-10 | poly-1-12 | 20/165/40 | 225 | 30 | 45,000 |
| poly-2d-11 | poly-1-13 | 105/50/75 | 230 | 32 | 77,000 |
| poly-2d-12 | poly-1-14 | 120/75/95 | 255 | 30 | 86,000 |
| poly-2d-13 | poly-1-16 | 195/45/45 | 285 | 16 | 112,000 |
| poly-2d-14 | poly-1-19 | 120/75/95 | 290 | 32 | 93,000 |
| poly-2d-15 | poly-1-21 | 155/85/60 | 300 | 20 | 103,000 |
| poly-2d-16 | poly-1-22 | 180/55/80 | 315 | 25 | 115,000 |
| poly-2d-17 | poly-1-24 | 180/70/80 | 330 | 24 | 117,000 |
| poly-2d-18 | poly-1-26 | 200/65/85 | 350 | 24 | 127,000 |
| poly-2d-19 | poly-1-28 | 35/160/185 | 380 | 49 | 87,000 |
| poly-2d-20 | poly-1-29 | 190/100/100 | 390 | 25 | 131,000 |
| poly-2d-21 | poly-1-30 | 160/95/140 | 395 | 35 | 126,000 |
| poly-2d-22 | poly-1-31 | 80/145/190 | 415 | 46 | 108,000 |

[a] Argon atmosphere; Solvent: DMF; Temperature: RT; $[poly-1]_O$ = 1.0 $mmol^{-1}$
[b] Determined by $^1H$ NMR in $CDCl_3$

[Table 8]

Table 8[a]

| Entry | Precursor | $I/m$ - $n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2e-1 | poly-1-1 | 45/50/15 | 110 | 16 | 34,000 |
| poly-2e-2 | poly-1-3 | 60/70/30 | 160 | 18 | 48,000 |
| poly-2e-3 | poly-1-4 | 90/55/25 | 170 | 15 | 59,000 |
| poly-2e-4 | poly-1-5 | 80/60/30 | 170 | 17 | 56,000 |
| poly-2e-5 | poly-1-9 | 140/55/15 | 210 | 6 | 80,000 |
| poly-2e-6 | poly-1-10 | 40/105/65 | 210 | 31 | 53,000 |

(continued)

Table 8[a]

| Entry | Precursor | $I/m - n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2e-7 | poly-1-11 | 80/90/50 | 220 | 23 | 66,000 |
| poly-2e-8 | poly-1-12 | 20/150/60 | 225 | 26 | 48,000 |
| poly-2e-9 | poly-1-14 | 120/100/35 | 255 | 14 | 82,000 |
| poly-2e-10 | poly-1-16 | 195/70/20 | 285 | 7 | 110,000 |
| poly-2e-11 | poly-1-19 | 120/115/55 | 290 | 19 | 90,000 |
| poly-2e-12 | poly-1-21 | 155/90/55 | 300 | 19 | 104,000 |
| poly-2e-13 | poly-1-22 | 180/90/45 | 315 | 14 | 112,000 |
| poly-2e-14 | poly-1-24 | 180/95/55 | 330 | 16 | 116,000 |
| poly-2e-15 | poly-1-26 | 200/85/65 | 350 | 19 | 127,000 |
| poly-2e-16 | poly-1-27 | 190/110/70 | 370 | 18 | 127,000 |
| poly-2e-17 | poly-1-28 | 35/210/135 | 380 | 36 | 86,000 |
| poly-2e-18 | poly-1-30 | 160/110/125 | 395 | 32 | 128,000 |
| poly-2e-19 | poly-1- 31 | 80/190/145 | 415 | 35 | 107,000 |

[a] Argon atmosphere; Solvent: DMF; Temperature: RT; $[poly-1]_O$ = 1.0 $mmol^{-1}$
[b] Determined by $^1H$ NMR in $CDCl_3$

[Table 9]

Table 9[a]

| Entry | Precursor | $I/m - n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2f-1 | poly-1-1 | 45/50/15 | 110 | 15 | 35,000 |
| poly-2f-2 | poly-1-2 | 60/45/15 | 120 | 11 | 41,000 |
| poly-2f-3 | poly-1-3 | 60/70/30 | 160 | 18 | 50,000 |
| poly-2f-4 | poly-1-4 | 90/60/20 | 170 | 11 | 59,000 |
| poly-2f-5 | poly-1-5 | 80/70/20 | 170 | 13 | 56,000 |
| poly-2f-6 | poly-1-9 | 140/55/15 | 210 | 6 | 81,000 |
| poly-2f-7 | poly-1-11 | 80/95/45 | 220 | 21 | 69,000 |
| poly-2f-8 | poly-1-14 | 120/85/50 | 255 | 20 | 88,000 |
| poly-2f-9 | poly-1-16 | 195/70/20 | 285 | 7 | 111,000 |
| poly-2f-10 | poly-1-19 | 120/105/65 | 290 | 22 | 96,000 |
| poly-2f-11 | poly-1-21 | 155/95/50 | 300 | 16 | 106,000 |
| poly-2f-12 | poly-1-22 | 180/105/30 | 315 | 10 | 113,000 |
| poly-2f-13 | poly-1-24 | 180/105/45 | 330 | 14 | 118,000 |
| poly-2f-14 | poly-1-26 | 210/100/40 | 350 | 11 | 130,000 |
| poly-2f-15 | poly-1-27 | 190/120/60 | 370 | 17 | 130,000 |
| poly-2f-16 | poly-1-30 | 160/100/135 | 395 | 34 | 139,000 |
| poly-2f-17 | poly-1-32 | 200/130/100 | 430 | 23 | 150,000 |
| poly-2f-18 | poly-1-33 | 320/90/40 | 450 | 9 | 181,000 |

[a] Argon atmosphere; Solvent: DMF; Temperature: RT; $[poly-1]_O$ = 1.0 $mmol^{-1}$
[b] Determined by $^1H$ NMR in $CDCl_3$

[Table 10]

Table 10[a]

| Entry | Precursor | $I/m - n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2g-1 | poly-1-1 | 45/55/10 | 110 | 9 | 36,000 |
| poly-2g-2 | poly-1-2 | 60/50/10 | 120 | 6 | 41,000 |
| poly-2g-3 | poly-1-3 | 60/80/20 | 160 | 11 | 51,000 |

(continued)

Table 10[a]

| Entry | Precursor | $I/m$ - $n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2g-4 | poly-1-4 | 90/65/15 | 170 | 8 | 61,000 |
| poly-2g-5 | poly-1-5 | 80/75/15 | 170 | 8 | 58,000 |
| poly-2g-6 | poly-1-9 | 140/60/10 | 210 | 4 | 82,000 |
| poly-2g-7 | poly-1-11 | 80/115/25 | 220 | 11 | 69,000 |
| poly-2g-8 | poly-1-14 | 120/125/10 | 255 | 5 | 83,000 |
| poly-2g-9 | poly-1-16 | 195/80/10 | 285 | 4 | 112,000 |
| poly-2g-10 | poly-1-19 | 120/140/30 | 290 | 10 | 95,000 |
| poly-2g-11 | poly-1-21 | 155/120/25 | 300 | 8 | 106,000 |
| poly-2g-12 | poly-1-22 | 180/120/15 | 315 | 5 | 113,000 |
| poly-2g-13 | poly-1-24 | 180/130/20 | 330 | 6 | 117,000 |
| poly-2g-14 | poly-1-26 | 200/120/30 | 350 | 8 | 130,000 |
| poly-2g-15 | poly-1-27 | 190/145/35 | 370 | 9 | 132,000 |
| poly-2g-16 | poly-1-29 | 190/165/35 | 390 | 10 | 136,000 |
| poly-2g-17 | poly-1-32 | 200/190/40 | 430 | 10 | 147,000 |
| poly-2g-18 | poly-1-33 | 320/110/20 | 450 | 4 | 180,000 |
| poly-2g-19 | poly-1-34 | 200/210/60 | 470 | 13 | 160,000 |

[a] Argon atmosphere; Solvent: DMF; Temperature: RT; $[poly\text{-}1]_O$ = 1.0 $mmol^{-1}$
[b] Determined by $^1H$ NMR in $CDCl_3$

[Table 11]

Table 11[a]

| Entry | Precursor | $I/m$ - $n/n_{obsd}$ [b] | DP [b] | $F_{Hydrophobic}$ (mol%) [b] | $M_{n,NMR}$ (g $mol^{-1}$) [b] |
|---|---|---|---|---|---|
| poly-2h-1 | poly-1-11 | 80/40/100 | 220 | 45 | 99,000 |
| poly-2h-2 | poly-1-14 | 120/85/50 | 255 | 19 | 98,000 |
| poly-2h-3 | poly-1-16 | 195/45/45 | 285 | 15 | 125,000 |
| poly-2h-4 | poly-1-18 | 120/45/125 | 290 | 43 | 133,000 |
| poly-2h-5 | poly-1-21 | 155/50/95 | 300 | 32 | 135,000 |
| poly-2h-6 | poly-1-22 | 180/40/95 | 315 | 30 | 145,000 |
| poly-2i-1 | poly-1-11 | 80/85/55 | 220 | 25 | 81,000 |
| poly-2i-2 | poly-1-14 | 120/85/50 | 255 | 19 | 96,000 |
| poly-2i-3 | poly-1-16 | 195/70/20 | 285 | 8 | 116,000 |
| poly-2i-4 | poly-1-18 | 120/100/70 | 290 | 24 | 110,000 |
| poly-2i-5 | poly-1-21 | 155/100/45 | 300 | 14 | 113,000 |
| poly-2i-6 | poly-1-22 | 180/95/40 | 315 | 13 | 122,000 |

[a] Argon atmosphere; Solvent: DMF; Temperature: RT; $[poly\text{-}1]_O$ = 1.0 $mmol^{-1}$
[b] Determined by $^1H$ NMR in $CDCl_3$

Determination of unimolecular association boundary conditions in poly-2a to poly-2g aqueous solutions

The results of evaluating the association behavior in the poly-2a to poly-2g aqueous solutions were used to determine the boundary conditions for unimolecular association in the poly-2a to poly-2g aqueous solutions. The association behavior in each of the aqueous solutions of poly-2a to poly-2g was evaluated using the association number $N_{agg}$ calculated from the ratio of the absolute weight-average molecular weight $M_{w,MALS}$, obtained using a system utilizing size exclusion chromatography of poly-2a to poly-2g in aqueous solution (PBS) obtained as described above, and an organic solvent (chloroform), and a multi-angle light scattering detector (SEC-MALS). $N_{agg}$ is calculated by the following formula: $N_{agg} = M_{w,MALS}$ (PBS)/$M_{w,MALS}$ (chloroform). It was shown that the corresponding polymers formed a micelle as single molecules in aqueous solution when $N_{agg}$ was a value of less than 1.5, and that the corresponding polymers associated as multimolecules in aqueous solution when $N_{agg}$ was a value of 1.5 or greater. The boundary conditions for the

polymerization degree and the proportion of hydrophobic side chains allowing the polymer to associate as single molecules in aqueous solution were determined for each of poly-2a to poly-2g, based on the calculated $N_{agg}$ and the degree of polymerization of the polymer, and on the proportion of hydrophobic side chains in the polymer. The boundary conditions are the dashed curves in Figs. 2 to 8.

[Measuring apparatus and conditions]

(1) SEC-MALS measurement (PBS)

Apparatus: JASCO Extrema HPLC system/JASCO Corp. (CO-4060, AS-4050, PU-4180)
Detector: RI-4030 differential refractometer/JASCO Corp., DAWN 8 multi-angle light scattering detector/Wyatt Technology Corporation
Column: Shodex OHpak SB-G 6B/ Resonac Corp. (6.0 mm × 50 mm), Shodex OHpak SB-806 M HQ/ Resonac Corp. (8.0 mm × 300 mm; particle diameter, 13 μm), Shodex OHpak SB-804 HQ/ Resonac Corp. (8.0 mm × 300 mm; particle diameter, 10 μm)
Mobile phase: 137 mM NaCl, 2.7 mM KCl, 11.9 mM phosphate buffer (PBS), pH 7.4
Temperature: 40°C
Flow rate: 1 mL/min
Sample concentration : 3 mg/mL
Results: Tables 12 to 19

(2) SEC-MALS measurement (chloroform)

Apparatus: High-speed GPC apparatus/Tosoh Corp. (HLC-8420GPC)
Detector: DAWN-HELEOS II Multi-angle light scattering detector/Wyatt Technology Corporation, Optilab T-rEX differential refractometer/Wyatt Technology Corporation
Column: Shodex LF-G/Resonac Corp. (4.6 mm × 10 mm, particle size, 6 μm), Shodex LF-804/Resonac Corp. (8.0 mm × 300 mm, particle size, 6 μm)
Mobile phase: chloroform
Temperature: 40°C
Flow rate: 1 mL/min
Sample concentration : 3 mg/mL
Results: Tables 12 to 19

[Table 12]

Table 12

| Entry | DP[a] | $F_{Hydrophobic}$ [a] (mol%) | $M_{w,MALS}$ (Da) | | $M_w/M_n$ [c] | $N_{agg}$ [d] |
|---|---|---|---|---|---|---|
| | | | PBS[b] | $CHCl_3$ [c] | | |
| poly-2a-1 | 110 | 41 | 21,700 | 24,000 | 1.22 | 0.90 |
| poly-2a-2 | 120 | 29 | 18,600 | 29,300 | 1.40 | 0.63 |
| poly-2a-3 | 160 | 35 | 22,400 | 19,500 | 1.16 | 1.15 |
| poly-2a-4 | 170 | 20 | 21,800 | 23,500 | 1.16 | 0.93 |
| poly-2a-5 | 170 | 22 | 21,600 | 17,000 | 1.09 | 1.27 |
| poly-2a-6 | 170 | 22 | 24,400 | 23,500 | 1.12 | 1.04 |
| poly-2a-7 | 210 | 12 | 21,900 | 28,200 | 1.19 | 0.78 |
| poly-2a-8 | 210 | 49 | 72,700 | 47,000 | 1.05 | 1.55 |
| poly-2a-9 | 220 | 35 | 20,900 | 28,900 | 1.23 | 0.72 |
| poly-2a-10 | 225 | 38 | 1,136,100 | 41,800 | 1.02 | 27.18 |
| poly-2a-11 | 230 | 20 | 26,800 | 26,500 | 1.16 | 1.01 |

(continued)

| Entry | DP[a] | $F_{Hydrophobic}$ [a] (mol%) | $M_{w,MALS}$ (Da) | | $M_w/M_n$ [c] | $N_{agg}$ [d] |
|---|---|---|---|---|---|---|
| | | | PBS[b] | $CHCl_3$ [c] | | |
| poly-2a-12 | 255 | 37 | 24,900 | 23,900 | 1.14 | 1.04 |
| poly-2a-13 | 285 | 17 | 32,800 | 39,200 | 1.33 | 0.84 |
| poly-2a-14 | 290 | 37 | 30,600 | 20,200 | 1.09 | 1.51 |
| poly-2a-15 | 295 | 25 | 26,300 | 22,500 | 1.12 | 1.17 |
| poly-2a-16 | 300 | 33 | 29,300 | 32,800 | 1.23 | 0.89 |
| poly-2a-17 | 315 | 25 | 26,000 | 36,100 | 1.19 | 0.72 |
| poly-2a-18 | 350 | 17 | 20,000 | 19,100 | 1.26 | 1.05 |
| poly-2a-19 | 380 | 40 | 1,309,300 | 52,600 | 1.09 | 24.89 |
| poly-2a-20 | 395 | 28 | 28,700 | 28,600 | 1.11 | 1.00 |
| poly-2a-21 | 415 | 42 | 46,000 | 61,200 | 1.05 | 0.75 |
| poly-2a-22 | 470 | 36 | 20,000 | 17,500 | 1.21 | 1.14 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)

[Table 13]

Table 13

| Entry | DP[a] | $F_{Hydrophobic}$ (mol%) [a] | $M_{w,MALS}$ (Da) | | $M_w/M_n$ [c] | $N_{agg}$[d] |
|---|---|---|---|---|---|---|
| | | | PBS[b] | $CHCl_3$ [c] | | |
| poly-2b-1 | 110 | 33 | 32,000 | 16,600 | 1.10 | 1.93 |
| poly-2b-2 | 120 | 24 | 22,700 | 16,200 | 1.13 | 1.40 |
| poly-2b-3 | 160 | 40 | 32,300 | 16,800 | 1.11 | 1.92 |
| poly-2b-4 | 170 | 22 | 18,900 | 20,800 | 1.14 | 0.91 |
| poly-2b-5 | 170 | 22 | 22,700 | 19,100 | 1.17 | 1.19 |
| poly-2b-6 | 210 | 12 | 24,300 | 21,100 | 1.16 | 1.15 |
| poly-2b-7 | 210 | 48 | 2,618,400 | 42,300 | 1.18 | 61.90 |
| poly-2b-8 | 220 | 27 | 25,700 | 22,600 | 1.15 | 1.14 |
| poly-2b-9 | 220 | 45 | 30,100 | 36,800 | 1.16 | 0.82 |
| poly-2b-10 | 225 | 45 | n.d. [e] | 44,100 | 1.07 | n.d. [e] |
| poly-2b-11 | 230 | 33 | 31,200 | 28,100 | 1.22 | 1.11 |
| poly-2b-12 | 255 | 22 | 26,000 | 24,400 | 1.16 | 1.07 |
| poly-2b-13 | 255 | 39 | 27,900 | 40,100 | 1.43 | 0.70 |
| poly-2b-14 | 285 | 11 | 28,900 | 24,900 | 1.12 | 1.16 |
| poly-2b-15 | 285 | 28 | 34,100 | 30,500 | 1.18 | 1.12 |
| poly-2b-16 | 290 | 23 | 22,600 | 22,800 | 1.18 | 0.99 |
| poly-2b-17 | 295 | 47 | 46,300 | 33,500 | 1.14 | 1.38 |
| poly-2b-18 | 300 | 19 | 27,400 | 24,700 | 1.09 | 1.11 |
| poly-2b-19 | 315 | 18 | 38,000 | 46,000 | 1.19 | 0.83 |
| poly-2b-20 | 320 | 51 | 33,600 | 29,500 | 1.20 | 1.14 |
| poly-2b-21 | 350 | 28 | 25,600 | 20,900 | 1.26 | 1.22 |
| poly-2b-22 | 380 | 49 | n.d. [e] | 19,500 | 1.09 | n.d. [e] |
| poly-2b-23 | 395 | 38 | 37,300 | 45,600 | 1.31 | 0.82 |
| poly-2b-24 | 415 | 66 | n.d. [e] | 26,900 | 1.34 | n.d. [e] |
| poly-2b-25 | 430 | 39 | 24,400 | 20,200 | 1.27 | 1.21 |

(continued)

| Entry | DP[a] | $F_{Hydrophobic}$ (mol%) [a] | $M_{w,MALS}$ (Da) | | $M_w/M_n$ [c] | $N_{agg}$[d] |
|---|---|---|---|---|---|---|
| | | | PBS[b] | $CHCl_3$ [c] | | |
| poly-2b-26 | 450 | 13 | 22,500 | 22,600 | 1.21 | 1.00 |
| poly-2b-27 | 470 | 45 | 27,100 | 26,500 | 1.33 | 1.02 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)
[e] Calculation not possible due to insolubility in PBS

[Table 14]

Table 14

| Entry | DP[a] | $F_{Hydrophobic}$ (mol%) [a] | $M_{w,MALS}$ (Da) | | $M_w/M_n$ [c] | $N_{agg}$ [d] |
|---|---|---|---|---|---|---|
| | | | PBS[b] | $CHCl_3$ [c] | | |
| poly-2c-1 | 110 | 36 | 70,700 | 34,300 | 1.02 | 2.06 |
| poly-2c-2 | 120 | 30 | 44,600 | 33,800 | 1.00 | 1.32 |
| poly-2c-3 | 160 | 44 | 131,000 | 33,800 | 1.01 | 3.88 |
| poly-2c-4 | 170 | 25 | 33,900 | 33,100 | 1.00 | 1.02 |
| poly-2c-5 | 170 | 25 | 33,300 | 34,200 | 1.02 | 0.97 |
| poly-2c-6 | 210 | 22 | 32,400 | 34,500 | 1.02 | 0.94 |
| poly-2c-7 | 220 | 44 | 103,600 | 25,100 | 1.11 | 4.13 |
| poly-2c-8 | 255 | 33 | 56,400 | 26,700 | 1.14 | 2.11 |
| poly-2c-9 | 285 | 14 | 37,500 | 30,500 | 1.19 | 1.23 |
| poly-2c-10 | 290 | 43 | 128,100 | 29,600 | 1.11 | 4.33 |
| poly-2c-11 | 295 | 30 | 66,500 | 58,400 | 1.03 | 1.14 |
| poly-2c-12 | 300 | 34 | 55,200 | 31,100 | 1.14 | 1.77 |
| poly-2c-13 | 315 | 31 | 38,000 | 31,500 | 1.17 | 1.21 |
| poly-2c-14 | 320 | 60 | 216,900 | 49,200 | 1.18 | 4.41 |
| poly-2c-15 | 330 | 15 | 43,900 | 62,600 | 1.05 | 0.70 |
| poly-2c-16 | 350 | 15 | 25,800 | 46,500 | 1.02 | 0.55 |
| poly-2c-17 | 370 | 16 | 23,100 | 25,300 | 1.29 | 0.91 |
| poly-2c-18 | 390 | 21 | 46,400 | 58,700 | 1.28 | 0.79 |
| poly-2c-19 | 395 | 52 | 197,600 | 48,400 | 1.02 | 4.08 |
| poly-2c-20 | 430 | 46 | 98,800 | 31,000 | 1.12 | 3.19 |
| poly-2c-21 | 450 | 7 | 25,000 | 42,200 | 1.07 | 0.59 |
| poly-2c-22 | 470 | 26 | 33,900 | 49,500 | 1.06 | 0.68 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)

[Table 15]

Table 15

| Entry | DP[a] | $F_{Hydrophobic}$ (mol%) [a] | $M_{w,MALS}$ (Da) | | $M_w/M_n$ [c] | $N_{agg}$ [d] |
|---|---|---|---|---|---|---|
| | | | PBS[b] | $CHCl_3$ [c] | | |
| poly-2d-1 | 110 | 30 | 26,800 | 14,600 | 1.26 | 1.84 |
| poly-2d-2 | 120 | 21 | 13,400 | 15,800 | 1.22 | 1.04 |
| poly-2d-3 | 160 | 36 | 29,600 | 17,200 | 1.23 | 1.72 |
| poly-2d-4 | 170 | 27 | 26,900 | 14,100 | 1.14 | 1.91 |
| poly-2d-5 | 170 | 30 | 20,900 | 12,900 | 1.16 | 1.62 |
| poly-2d-6 | 180 | 25 | 31,000 | 38,200 | 1.38 | 0.81 |
| poly-2d-7 | 210 | 16 | 15,600 | 14,900 | 1.71 | 1.05 |
| poly-2d-8 | 210 | 77 | 111,700 | 39,800 | 1.11 | 2.81 |
| poly-2d-9 | 220 | 37 | 57,100 | 35,300 | 1.43 | 1.62 |
| poly-2d-10 | 225 | 30 | n.d. [e] | 44,100 | 1.07 | n.d. [e] |
| poly-2d-11 | 230 | 32 | 25,600 | 25,400 | 1.46 | 1.01 |
| poly-2d-12 | 255 | 30 | 30,100 | 39,600 | 1.53 | 0.76 |
| poly-2d-13 | 285 | 16 | 21,200 | 39,400 | 1.55 | 0.53 |
| poly-2d-14 | 290 | 32 | 34,200 | 37,500 | 1.52 | 0.91 |
| poly-2d-15 | 300 | 20 | 23,200 | 40,600 | 1.64 | 0.57 |
| poly-2d-16 | 315 | 25 | 27,400 | 39,200 | 1.49 | 0.70 |
| poly-2d-17 | 330 | 24 | 30,400 | 26,100 | 1.48 | 1.16 |
| poly-2d-18 | 350 | 24 | 22,600 | 31,100 | 1.31 | 0.73 |
| poly-2d-19 | 380 | 49 | n.d. [e] | 47,200 | 1.03 | n.d. [e] |
| poly-2d-20 | 390 | 25 | 36,400 | 34,100 | 1.41 | 1.07 |
| poly-2d-21 | 395 | 35 | 26,600 | 27,800 | 1.46 | 0.96 |
| poly-2d-22 | 415 | 46 | 965,800 | 40,900 | 1.23 | 23.60 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)
[e] Calculation not possible due to insolubility in PBS

[Table 16]

Table 16

| Entry | DP[a] | $F_{Hydrophobic}$ (mol%) [a] | $M_{w,MALS}$ (Da) | | $M_w/M_n$ [c] | $N_{agg}$[d] |
|---|---|---|---|---|---|---|
| | | | PBS[b] | $CHCl_3$ [c] | | |
| poly-2e-1 | 110 | 16 | 21,400 | 22,600 | 1.68 | 0.95 |
| poly-2e-2 | 160 | 18 | 31,200 | 20,300 | 1.81 | 1.54 |
| poly-2e-3 | 170 | 15 | 24,500 | 21,300 | 1.66 | 1.15 |
| poly-2e-4 | 170 | 17 | 26,300 | 20,300 | 1.58 | 1.30 |
| poly-2e-5 | 210 | 6 | 22,000 | 17,900 | 1.54 | 1.23 |
| poly-2e-6 | 210 | 31 | 122,800 | 38,800 | 1.03 | 3.16 |
| poly-2e-7 | 220 | 23 | 26,100 | 33,700 | 1.79 | 0.77 |
| poly-2e-8 | 225 | 26 | 532,300 | 34,700 | 1.02 | 15.30 |
| poly-2e-9 | 255 | 14 | 23,300 | 16,300 | 1.26 | 1.43 |
| poly-2e-10 | 285 | 7 | 27,500 | 29,000 | 1.37 | 0.95 |
| poly-2e-11 | 290 | 19 | 27,100 | 31,600 | 1.97 | 0.86 |
| poly-2e-12 | 300 | 19 | 27,500 | 28,400 | 1.51 | 0.97 |
| poly-2e-13 | 315 | 14 | 25,400 | 18,400 | 1.31 | 1.38 |

(continued)

| Entry | DP[a] | $F_{Hydrophobic}$ (mol%) [a] | $M_{w,MALS}$ (Da) | | $M_w/M_n$ [c] | $N_{agg}$[d] |
|---|---|---|---|---|---|---|
| | | | PBS[b] | $CHCl_3$ [c] | | |
| poly-2e-14 | 330 | 16 | 40,100 | 50,200 | 1.63 | 0.80 |
| poly-2e-15 | 350 | 19 | 33,000 | 30,700 | 1.56 | 1.07 |
| poly-2e-16 | 370 | 18 | 27,900 | 39,300 | 1.56 | 0.71 |
| poly-2e-17 | 380 | 36 | n.d. [e] | 44,700 | 1.02 | n.d. [e] |
| poly-2e-18 | 395 | 32 | 59,200 | 64,600 | 1.56 | 0.92 |
| poly-2e-19 | 415 | 35 | 1,501,500 | 49,100 | 1.02 | 30.60 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)
[e] Calculation not possible due to insolubility in PBS

[Table 17]

Table 17

| Entry | DP[a] | $F_{Hydrophobic}$ (mol%) [a] | $M_{w,MALS}$ (Da) | | $M_w/M_n$ [c] | $N_{agg}$ [d] |
|---|---|---|---|---|---|---|
| | | | PBS[b] | $CHCl_3$ [c] | | |
| poly-2f-1 | 110 | 15 | 31,400 | 14,700 | 1.43 | 2.14 |
| poly-2f-2 | 120 | 11 | 26,000 | 14,000 | 1.39 | 1.86 |
| poly-2f-3 | 160 | 18 | 43,300 | 11,400 | 1.51 | 3.80 |
| poly-2f-4 | 170 | 11 | 25,500 | 13,300 | 1.30 | 1.92 |
| poly-2f-5 | 170 | 13 | 27,000 | 14,200 | 1.55 | 1.90 |
| poly-2f-6 | 210 | 6 | 23,900 | 14,000 | 1.27 | 1.71 |
| poly-2f-7 | 220 | 21 | 37,900 | 15,500 | 1.68 | 2.45 |
| poly-2f-8 | 255 | 20 | 47,700 | 18,300 | 1.92 | 2.61 |
| poly-2f-9 | 285 | 7 | 28,500 | 26,900 | 2.14 | 1.06 |
| poly-2f-10 | 290 | 22 | 49,900 | 16,400 | 1.46 | 3.04 |
| poly-2f-11 | 300 | 16 | 29,900 | 16,500 | 1.87 | 1.81 |
| poly-2f-12 | 315 | 10 | 27,200 | 28,000 | 2.30 | 0.97 |
| poly-2f-13 | 330 | 14 | 41,600 | 51,600 | 1.44 | 0.81 |
| poly-2f-14 | 350 | 11 | 21,000 | 23,200 | 1.71 | 0.91 |
| poly-2f-15 | 370 | 17 | 23,700 | 32,900 | 1.64 | 0.72 |
| poly-2f-16 | 395 | 34 | 137,900 | 55,200 | 1.47 | 2.50 |
| poly-2f-17 | 430 | 23 | 58,900 | 34,800 | 1.52 | 1.69 |
| poly-2f-18 | 450 | 9 | 19,600 | 19,800 | 2.04 | 0.99 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)

[Table 18]

Table 18

| Entry | DP[a] | $F_{Hydrophobic}$ (mol%) [a] | $M_{w,MALS}$ (Da) PBS[b] | $M_{w,MALS}$ (Da) $CHCl_3$ [c] | $M_w/M_n$ [c] | $N_{agg}$ [d] |
|---|---|---|---|---|---|---|
| poly-2g-1 | 110 | 9 | 148,600 | 17,000 | 1.09 | 8.74 |
| poly-2g-2 | 120 | 6 | 32,000 | 20,400 | 1.10 | 1.57 |
| poly-2g-3 | 160 | 11 | 176,200 | 21,100 | 1.27 | 8.35 |
| poly-2g-4 | 170 | 8 | 41,800 | 20,600 | 1.09 | 2.03 |
| poly-2g-5 | 170 | 8 | 124,200 | 17,900 | 1.07 | 6.94 |
| poly-2g-6 | 210 | 4 | 34,400 | 21,700 | 1.14 | 1.59 |
| poly-2g-7 | 220 | 11 | 65,800 | 19,300 | 1.19 | 3.41 |
| poly-2g-8 | 255 | 5 | 25,300 | 12,300 | 1.15 | 2.06 |
| poly-2g-9 | 285 | 4 | 31,900 | 26,700 | 1.22 | 1.19 |
| poly-2g-10 | 290 | 10 | 104,100 | 22,800 | 1.29 | 4.57 |
| poly-2g-11 | 300 | 8 | 75,700 | 24,200 | 1.22 | 3.13 |
| poly-2g-12 | 315 | 5 | 29,200 | 21,400 | 1.14 | 1.36 |
| poly-2g-13 | 330 | 6 | 52,200 | 37,400 | 1.09 | 1.40 |
| poly-2g-14 | 350 | 8 | 68,000 | 21,100 | 1.34 | 3.22 |
| poly-2g-15 | 370 | 9 | 93,400 | 21,400 | 1.24 | 4.36 |
| poly-2g-16 | 390 | 10 | 275,400 | 36,300 | 1.13 | 7.59 |
| poly-2g-17 | 430 | 10 | 104,000 | 22,100 | 1.16 | 4.71 |
| poly-2g-18 | 450 | 4 | 34,400 | 37,300 | 1.36 | 0.92 |
| poly-2g-19 | 470 | 13 | 158,400 | 19,900 | 1.25 | 7.96 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)

[Table 19]

Table 19

| Entry | DP [a] | $F_{Hydrophobic}$ (mol%) [a] | $M_{w,MALS}$ (Da) PBS[b] | $M_{w,MALS}$ (Da) $CHCl_3$ [c] | $M_w/M_n$ [c] | $N_{agg}$[d] |
|---|---|---|---|---|---|---|
| poly-2h-1 | 220 | 45 | 71,500 | 26,100 | 1.29 | 2.74 |
| poly-2h-2 | 255 | 19 | 20,000 | 20,000 | 1.48 | 1.00 |
| poly-2h-3 | 285 | 15 | 31,300 | 29,800 | 1.54 | 1.05 |
| poly-2h-4 | 290 | 43 | 39,500 | 27,500 | 1.37 | 1.44 |
| poly-2h-5 | 300 | 32 | 33,100 | 27,800 | 1.49 | 1.19 |
| poly-2h-6 | 315 | 30 | 42,300 | 45,300 | 1.42 | 0.93 |
| poly-2i-1 | 220 | 25 | 56,500 | 31,600 | 1.24 | 1.79 |
| poly-2i-2 | 255 | 19 | 42,100 | 37,300 | 1.44 | 1.13 |
| poly-2i-3 | 285 | 8 | 32,200 | 52,700 | 1.29 | 0.61 |
| poly-2i-4 | 290 | 24 | 58,200 | 37,400 | 1.35 | 1.56 |
| poly-2i-5 | 300 | 14 | 38,000 | 35,400 | 1.47 | 1.07 |
| poly-2i-6 | 315 | 13 | 34,700 | 34,000 | 1.30 | 1.02 |

[a] Determined by $^1H$ NMR in $CDCl_3$
[b] Determined by SEC-MALS in PBS
[c] Determined by SEC-MALS in $CHCl_3$
[d] Aggregation number in PBS: $N_{agg} = M_{w,MALS}$ (PBS)/$M_{w,MALS}$ (chloroform)

Synthesis of poly[(benzyl)-co-(poly(ethylene acrylateglycol)methyl etheracrylate)-co-(2-carboxyethyl acrylate), $BZA_m$-$PEGA_n$-$CEA_k$

Typical polymerization method

[Chemical Formula 11]

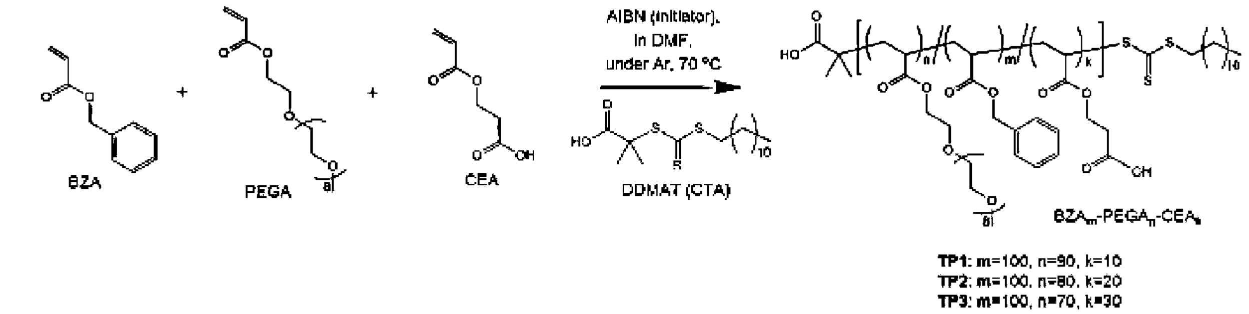


Synthesis of TP1

A monomer solution was prepared by weighing out 146 mg of benzyl acrylate (BZA), 389 mg of poly(ethylene glycol)methyl etheracrylate (PEGA, Mn = 480 g/mol) and 13 mg of 2-carboxyethyl acrylate. Separately, 0.5 mg of 2,2'-azobis(2-methylpropionitrile) (AIBN) and 2.2 mg of 2-(dodecylthiocarbonothioylthio)-2-methylpropionic acid (DDMAT) were weighed out and dissolved in 1 mL of distillation-purified N,N-dimethylformamide (DMF), the resulting solution was added to the monomer solution, and polymerization was carried out in an oil bath at 70°C. After an elapse of 20 minutes, the reaction mixture was cooled with liquid nitrogen to halt the reaction, and then the reaction mixture was dialyzed against methanol and ultrapure water (MWCO = 6-8 kD) to purify the target compound. Finally, freeze-drying was carried out to obtain poly(benzyl acrylate)-co-(poly(ethylene glycol)methyl etheracrylate)-co-(2-carboxyethyl acrylate). The conversion rate, polymerization degree and number-average molecular weight ($M_{nNMR}$) of the obtained copolymer was analyzed by $^1$H-NMR. The conversion rates were BZA = 73.3%, PEGA = 65.7% and CEA = 57.1%, $M_{nNMR}$ = 62,700 g/mol, and measurement using GPC for the molecular weight dispersity ($M_w/M_n$) of the obtained copolymer resulted in $M_w/M_n$ = 1.62. The charging amounts of the monomers (PEGA, BZA, CEA) were changed under the same conditions to synthesize polymers with different compositional ratios (TP1-TP3) and copolymers without BZA ($PEGA_{124}$-$CEA_{10}$, $PEGA_{104}$-$CEA_{30}$).

Measuring apparatus and conditions

(1) $^1$H-NMR measurement

Apparatus: Bruker Biospin AVANCE III (400 MHz)/Bruker
Solvent: Dimethyl sulfoxide-$d_6$ containing 0.03% tetramethylsilane/Kanto Kagaku Co., Ltd. Measuring temperature: room temperature
Results: δ(ppm) = 0.88 (t, 3H, $-S-CH_2-(CH_2)_{10}-CH_3$), 1.10-1.29 (m, 20H, $-S-CH_2-(CH_2)_{10}-CH_3$), 1.33-1.99 (m, 406H, $-(CH_2-CH$ (BZAsegment)$)_m$-, $-(CH_2-CH$ (PEGAsegment)$)_n$-, $-(CH_2-CH$ (CEAsegment)$)_k$-, $(CH_3)_2-C(COOH)-$), 2.19-2.53 (m, 200H, $-(CH_2-CH$ (BZA segment)$)_m$-, $-(CH_2-CH$ (PEGA segment)$)_n$-, $-(CH_2-CH$ (CEA segment)$)_k$-), 2.60-2.75 (m, 20H, $-COO-CH_2-CH_2-COOH$), 3.31-3.40 (m, 272H, $-CH_2-CH_2-O-(CH_2-CH_2-O)_8-CH_3$,$-S-CH_2-(CH_2)_{10}-CH_3$), 3.42-3.74 (m, 3060H, $-CH_2-CH_2-O-(CH_2-CH_2-O)_8-CH_3$), 3.90-4.40 (m, 200H, $-CH_2-CH_2-O-(CH_2-CH_2-O)_8-CH_3$, $-COO-CH_2-CH_2-COOH$), 4.81-5.16 (m, 200H, $-COO-CH_2-C_6H_5$), 7.17-7.40 (m, 500H, $-COO-CH_2-C_6H_5$).

(2) GPC measurement

Apparatus: JASCO Extrema HPLC system (LC-Net II/ADC, Co-4060, AS-4050, PU-4180/JASCO Corp.

Detector: UV-4070/JASCO Corp., RI-4030/JASCO Corp.

Column: TSKgel α-2500 column/Tosoh Corp. (column size: 7.8 mm × 300 mm, pore size: 2.5 nm, particle size: 7 μm, exclusion limit: 1 × $10^4$ g/mol)

TSKgel α-4000 column/Tosoh Corp. (column size: 7.8 mm × 300 mm, pore size: 45 nm, particle size: 10 μm, exclusion limit: 1 × $10^6$ g/mol)

TSKgel Guard Column-α/Tosoh Corp.

Mobile phase: N,N-dimethylformamide (DMF) dissolving 10 mmol/L lithium bromide

Temperature: 40°C

Flow rate: 1 mL/min

Sample concentration : 5 mg/mL

Standard substance: Shodex STANDARD M-75/Showa Denko K.K.

Results: Fig. 9

(5) Synthesis of poly[(benzyl acrylate)-co-(poly(ethylene glycol)methyl etheracrylate)-co-(2-carboxyethyl acrylate)-(Gd-s-2-(4-aminobenzyl)-1,4,7,10-tetraazacyclododecanetetraacetic acid) and $BZA_m$-$PEGA_n$-CEAk (DOTA)j

[Chemical Formula 12]

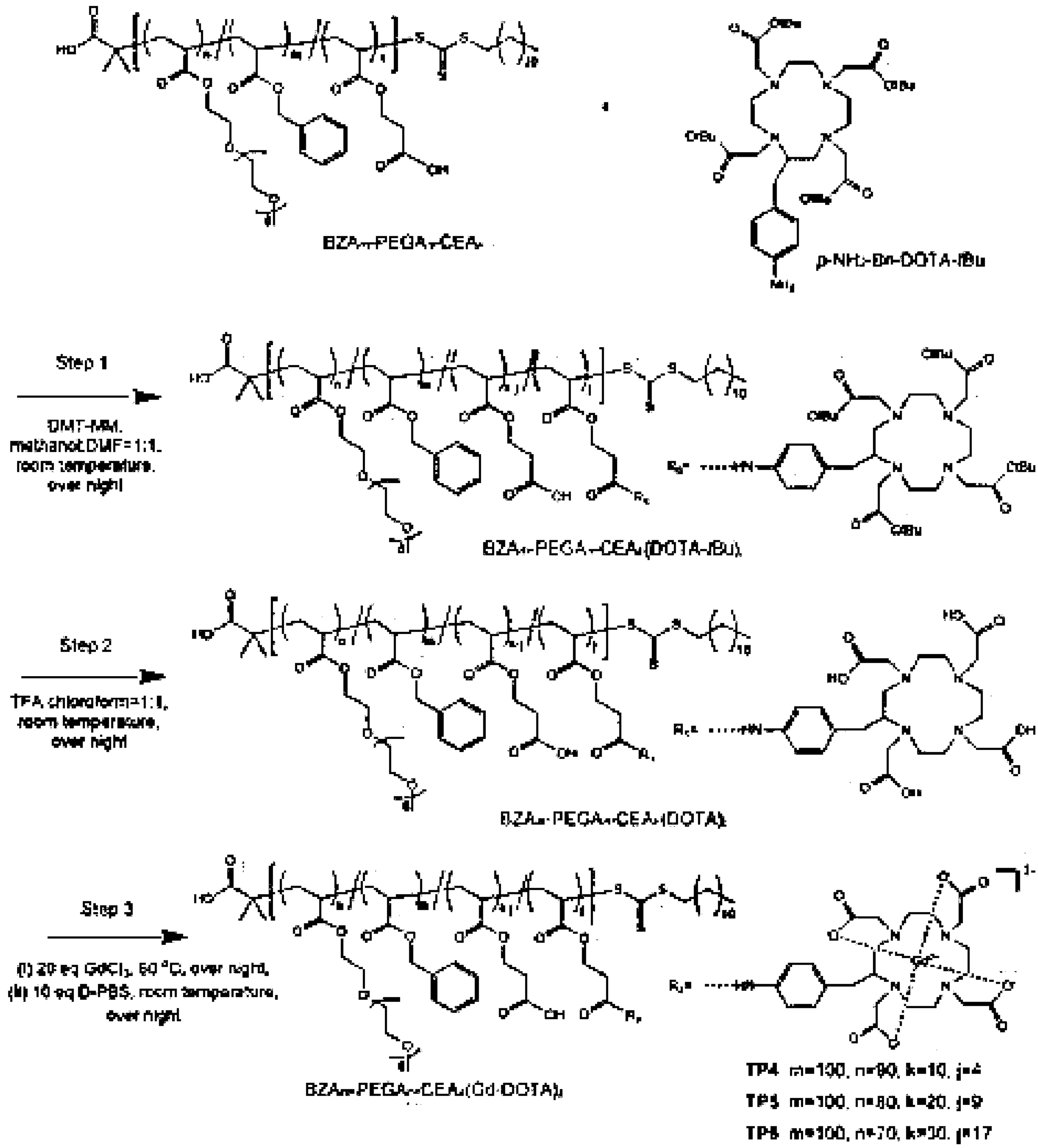


Synthesis of TP4

After dissolving the copolymer (2449 mg, 0.04 mmol) and DMT-MM (132.8 mg, 0.48 mmol) in a methanol/DMF mixture (20 mL, methanol:DMF = 1:1), the mixture was stirred for 1 hour at 0°C. Next, s-2-(4-aminobenzyl)-1,4,7,10-tetraazacyclododecane tetra-tert-butyl acetate (p-$NH_2$-Bn-DOTA-tBu, 339.2 mg, 0.4 mmol) was added to the solution and the mixture was stirred overnight at room temperature. The target compound was purified by dialysis of the reaction mixture against methanol and ultrapure water (MWCO = 6 to 8 kD). Finally, freeze-drying was carried out to obtain $BZA_{100}$-$PEGA_{90}$-$CEA_{10}$(DOTA-tBu)$_4$. The obtained compound was dissolved in a trifluoroacetic acid (TFA)/chloroform mixture (20 mL, TFA:chloroform = 1:1) and stirred overnight at room temperature. The target compound was purified by dialysis of the reaction mixture against methanol and ultrapure water (MWCO = 6 to 8 kD). Finally, freeze-drying was carried out to obtain $BZA_{100}$-$PEGA_{90}$-$CEA_{10}$(DOTA)$_4$. The obtained polymer was analyzed by $^1$H-NMR. Copolymers with different compositions were also reacted and analyzed under the same conditions.

Measuring apparatus and conditions

(1) $^1$H-NMR measurement

Apparatus: Bruker Biospin AVANCE III (400 MHz)/Bruker
Solvent: Dimethyl sulfoxide-$d_6$ containing 0.03% tetramethylsilane/Kanto Kagaku Co., Ltd.
Measuring temperature: room temperature
Results: $\delta$(ppm) = 0.88 (t, 3H, $-S-CH_2-(CH_2)10-CH_3$), 0.93-1.27 (m, 20H, $-S-CH_2-(CH_2)_{10}-CH_3$), 1.30-1.98 (m, 406H, $-(CH_2-CH$ (BZA segment)$)_m$-, $-(CH_2-CH$ (PEGA segment)$)_n$-, - $(CH_2-CH$ (CEA segment)$)_k$-, $(CH_3)_2-C(COOH)-$), 2.05-2.43 (m, 200H,$-(CH_2-CH$ (BZA segment)$)_m$-, $-(CH_2-CH$ (PEGA segment)$)_n$-, $-(CH_2-CH$ (CEA segment)$)_k$-), 2.54-2.68 (m, 20H, $-COO-CH_2-CH_2-COOH$, $-COO-CH_2-CH_2-CO-$DOTA segment), 3.18-3.27 (m, 422H, $-CH_2-CH_2-O-(CH_2-CH_2-O)_8-CH_3$, $-CH_2-CH_2-N(CH_2COOH)-$, $-S-CH_2-(CH_2)_{10}-CH_3$), 3.28-3.71 (m, 3100H, $-CH_2-CH_2-O-(CH_2-CH_2-O)_8-CH_3$, $-CH_2-CH_2-N(CH_2COOH)-$, $-NH-C_6H_4-CH_2-$), 3.85-4.35 (m, 200H, $-CH_2-CH_2-O-(CH_2-CH_2-O)_8-CH_3$, $-COO-CH_2-CH_2-COOH$, $-COO-CH_2-CH_2-CO-$DOTA segment), 4.77-5.15 (m, 200H, $-COO-CH_2-C_6H_5$), 7.00-7.60 (m, 540H, $-COO-CH_2-C_6H_5$, $-NH-C_6H_4-CH_2-$) (Fig. 10)

The copolymers synthesized above and gadolinium chloride hexahydrate (2973.6 mg, 8.0 mmol) were dissolved in ultrapure water and each mixture was stirred overnight in a water bath at 60°C. The reaction mixture was dialyzed against ultrapure water (MWCO = 6 to 8 kD) and then mixed with PBS(-) (100 mL), and the mixture was stirred overnight at room temperature. The reaction mixture was then filtered with a filter and again purified by dialysis against ultrapure water (MWCO = 6 to 8 kD). Finally, freeze-drying was carried out to obtain the target compound. The amount of Gd introduced into each polymer was confirmed by inductively coupled plasma mass spectrometry (ICP-MS). For copolymers without BZA as well, p-$NH_2$-Bn-DOTA-tBu and Gd were introduced and purification was carried out by the same procedure. Five different final products were obtained by the reaction described above. TP4 = $BZA_{100}$-$PEGA_{90}$-$CEA_{10}$$(DOTA)_4$, TP5 = $BZA_{100}$-$PEGA_{80}$-$CEA_{20}$$(DOTA)_9$, TP6 = $BZA_{100}$-$PEGA_{70}$-$CEA_{30}$$(DOTA)_{17}$, PEGA-$Gd_4$ = $PEGA_{124}$-$CEA_{10}$$(DOTA)_4$, $PEGA_{104}$-$CEA_{30}$$(DOTA)_{12}$.

The present invention will now be explained in greater detail by examples. However, it is to be understood that the invention is not limited to these examples.

## EXAMPLES

Example 1: Hydrophobic evaluation of hydrophobic side chains of poly-2a-i

The degree of hydrophobicity of the hydrophobic side chains introduced into poly-2a-i was evaluated by inputting the structure of the introduced compound into molinspiration [https://molinspiration.com].
Results: Table 20

[Table 20]

Table 20

| Entry | Pendant | cLogP [a] |
|---|---|---|
| poly-2a | Benzylamine | 1.12 |
| poly-2b | 1-Naphthylmethylamine | 2.28 |
| poly-2c | 1-Pyrenemethylamine | 4.01 |
| poly-2d | 2-Ethylhexylamine | 2.31 |
| poly-2e | Nerol | 3.20 |
| poly-2f | Farnesol | 5.05 |
| poly-2g | DL-$\alpha$-tocopherol | 9.04 |
| poly-2h | Losartan | 4.87 |
| poly-2i | Fluphenazine | 4.29 |

[a] cLogP calculated by molinspiration [www.molinspiration.com]

Example 2: Determination of unimolecular association boundary conditions in universal poly-2 aqueous solution

The boundary conditions for unimolecular association of poly-2a to poly-2g in aqueous solution were used as the basis for determining the boundary conditions for unimolecular association of poly-2 in aqueous solution to be applied for various hydrophobic side chain structures. Specifically, in three-dimensional space with the cLogP value of the introduced hydrophobic side chains as the first axis, the polymerization degree of the polymer as the second axis and the proportion of hydrophobic side chains in the polymer as the third axis, a curve was drawn linking the boundary conditions for unimolecular association in the solutions of poly-2a to poly-2g. The lower side of the curved surface represents the polymer polymerization degree, proportion of hydrophobic side chains and the cLogP value of the hydrophobic side chains allowing association as single molecules in the poly-2 aqueous solution.

Results: Fig. 11

Example 3: Evaluation of SMDC formation

The foldability in water of $BZA_m$-$PEGA_n$-$CEA_k(DOTA)_j$ obtained by the procedure described above was evaluated by comparing the absolute weight-average molecular weight $M_w$ calculated from size exclusion chromatography in aqueous solution (PBS) and in an organic solvent (chloroform or DMF) and a system utilizing a multi-angle light scattering detector (SEC-MALS). Data obtained from SEC-MALS were analyzed using a Zimm plot, with dn/dc being the value calculated by measuring the refractive index of 4 different samples with different concentrations in each solvent (4, 3, 2 and 1 mg/mL). DA was calculated by the following formula. DA = $M_{wPBS}/M_{wCHCl3}$

(1) GPC measurement (aqueous system)

Apparatus: JASCO Extrema HPLC system (LC-Net II/ADC, Co-4060, AS-4050, PU-4180/JASCO Corp.
Detector: UV-4070/JASCO Corp., RI-4030/JASCO Corp., DAWN 8 MALS detector/Wyatt Technology Corporation
Column: OHpak SB-804HQ column/Showa Denko K.K.
(column size: 8 mm × 300 mm, pore size: 20 nm, particle size: 10 μm, exclusion limit: 1 × $10^6$ g/mol)
OHpak SB-806 MHQ column/Showa Denko K.K.
(column size: 8 mm × 300 mm, pore size: 1.5 μm, particle size: 13 μm, exclusion limit: 2 × $10^7$ g/mol)
OHpak SB-G 6B Guard column/Showa Denko K.K.
Mobile phase: 137 mM NaCl, 2.7 mM KCl, 11.9 mM phosphate buffer (PBS), pH 7.4 Temperature: 40°C
Flow rate: 1 mL/min
Sample concentration : 5 mg/mL
Results: Table 21

(2) GPC measurement (chloroform-based)

Apparatus: TOSOH HLC-8220 GPC system/Tosoh Corp.
Detector: DAWN HELEOS II MALS detector/Wyatt
Column: LF-804 column/Showa Denko K.K.
(column size: 8 mm × 300 mm, pore size: 300 nm, particle size: 6 μm, exclusion limit: 2 × $10^6$ g/mol)
LF-G guard column/Showa Denko K.K.
Mobile phase: chloroform
Temperature: 40°C
Flow rate: 1 mL/min
Sample concentration : 5 mg/mL
Results: Table 21

Table 21. DA calculation for TP4-6

| No. | *dn/dc* in PBS[a] (mL/g) | *dn/dc* in $CHCl_3$[b] (mL/g) | $M_w$ by MALS-PBS[c] (g/mol) | $M_w$ by MALS-$CHCl_3$[d] (g/mol) | DA[e] |
|---|---|---|---|---|---|
| TP4 | 0.1334 | 0.0691 | 82,100 | 89,900 | 0.91 |
| TP5 | 0.1369 | 0.0718 | 75,700 | 86,000 | 0.88 |
| TP6 | 0.1363 | 0.0778 | 74,900 | 142,400 | 0.53 |

[a] Increase in refractive index concentration in PBS

[b] Increase in refractive index concentration in chloroform

[c] Determined by SEC-MALS in PBS

[d] Determined by SEC-MALS in $CHCl_3$

[e] DA (degree of association) in PBS

As mentioned above, DA = 1.0 means intramolecular association, i.e. SMDC formation, a high DA (DA > 1.0) means intermolecular self-assembly, and a low DA (DA < 1.0) means SMDC formation in water and potential micelle formation in organic solvents. Since the DA values for TP4, TP5 and TP6 were 0.91, 0.88 and 0.53, respectively, as shown in the tables above, this suggests SMDC formation in water.

Example 4: Design and property evaluation of SMDC-Gd

For the previously obtained $BZA_m$-$PEGA_n$-$CEA_k(DOTA)_j$ and $PEGA_n$-$CEA_k(DOTA)_j$, the particle size was confirmed by Guinier plot in small angle x-ray scattering (SAXS), and SMDC formation was confirmed by Kratky plot. The particle size was also measured by transmission electron microscopy (TEM) and dynamic light scattering. The surface potential was also measured.

(1) SAXS measurement

Apparatus: nano-viewer SAXS system/Rigaku Corp.
Solvent: Ultrapure water or DMF
Sample concentration : 10 mg/ml
Measuring temperature: room temperature
Results: Fig. 12 and Fig. 13

(2) TEM measurement

Apparatus: JEM-2100F TEM system/JEOL Corp.
Solvent: Ultrapure water
Pretreatment: Stained on formvar/carbon supported-copper grids
Measuring temperature: room temperature
Results: Fig. 14 and Fig. 15

(3) DLS

Apparatus: Malvern Zetasizer Nano ZSP/Malvem Panalytical

Solvent: Ultrapure water
Sample concentration : 10 mg/ml
Measuring temperature: room temperature
Results: Fig. 16 and Fig. 17

The peaks in the Kratky plots (Fig. 12) in aqueous solution indicate that TP4, TP5 and TP6 form particles in aqueous solution. On the other hand, no peaks were observed in the Kratky plots in DMF, indicating that the particles had not formed in DMF. TEM images and particle size analysis of TP4 and TP6 (Fig. 14 and Fig. 15) and SAXS analysis (Fig. 12 and Fig. 13) showed diameters of approximately 5 to 7 nm, with a narrow particle size distribution. This matched the results of DLS analysis. In research using numerous polymeric micelles as drug delivery carriers and comparing polymeric micelles with CMC/CAC with having diameters of 30 to 100 nm, it has been reported that smaller carriers exhibit more satisfactory accumulation, permeability and extravascular infiltration with respect to malignant tumors. However, not many carriers lacking CMC/CAC and with sizes of 10 nm and smaller have been reported. In renal excretion, which is one of the major discharge pathways following administration, a threshold exists for filtration by renal glomeruli. Accumulation in the liver can also be controlled by limiting the particle size to 8 to 9 nm. The TP4 and TP6 analyzed in the Examples herein were slightly larger than the threshold for renal excretion. Upon measuring the zeta potentials for TP4, TP6 and PEGA-$Gd_4$ (Fig. 17), the surface charge was found to be negative due to the effects of carboxy groups remaining on the polymer side chains. This makes it possible to inhibit excretion out of the body by the mononuclear phagocyte system, by preventing nonspecific interaction with plasma proteins.

Example 5: Relaxivity evaluation of SMDC-Gd

Different polymer samples (Gd conc. 1 mM, 0.5 mM, 0.25 mM and 0.125 mM) were prepared, and the longitudinal relaxation time $T_1$ and transverse relaxation time $T_2$ were measured for each concentration. Using the obtained results, a plot of Gd concentration with respect to obtained relaxation time was drawn, and the longitudinal relaxivity $r_1$ and transverse relaxivity $r_2$ were derived from the slope of the fitted curve, based on the following formula. Here, $(1/T_i)_{obs}$ represents the measured relaxation time, and $(1/T_i)_d$ represents the relaxation time of the solvent.

$$(1/T_i)_{obs} = (1/T_i)_d + r_i[Gd].\ i = 1, 2$$

(1) Relaxivity measurement

Apparatus: Bruker minispec 0.47T NMR/Bruker
Solvent: Ultrapure water or BSA (10 mg/mL)-dissolved ultrapure water
Measuring conditions: Temperature: 37°C, receiver gain = 80-82, recycle delay = 2.
Results: Table 22

Table 22. Relaxivity measurement results

| | $r_1$[a] ($s^{-1}mM^{-1}$) | $r_2$[b] ($s^{-1}mM^{-1}$) | $r_1/r_2$[c] | $r_1$ with BSA[d] ($s^{-1}mM^{-1}$) | $r_2$ with BSA[e] ($s^{-1}mM^{-1}$) | $r_1/r_2$ with BSA[f] |
|---|---|---|---|---|---|---|
| Gd-DOTA | 3.7 | 4.7 | 0.79 | - | - | - |
| PEGA-$Gd_4$ | 19.6 | 23.6 | 0.83 | 21.4 | 25.9 | 0.83 |
| PEGA-$Gd_{12}$ | 20.4 | 23.0 | 0.89 | - | - | - |
| SMDC-$Gd_4$ (TP4) | 25.9 | 31.1 | 0.83 | 26.6 | 32.1 | 0.83 |
| SMDC-$Gd_9$ (TP5) | 22.8 | 28.0 | 0.81 | - | - | - |
| SMDC-$Gd_{17}$ (TP6) | 24.7 | 29.6 | 0.83 | 25.5 | 30.8 | 0.83 |

[a] Longitudinal relaxivity measured in water at 37°C

[b] Transverse relaxivity measured in water at 37°C

[c] Positive contrast in water at 37°C

[d] Longitudinal relaxivity at 37°C in water containing 10 mg/ml bovine serum albumin

[e] Transverse relaxivity at 37°C in water containing 10 mg/ml bovine serum albumin

[f] Positive contrast at 37°C in water containing 10 mg/ml bovine serum albumin

The relaxivity measurement results (Table 22) indicated that PEGA-$Gd_4$, PEGA-$Gd_{12}$, TP4, TP5 and TP6 exhibited higher longitudinal relaxivity ($r_1$) and transverse relaxivity ($r_2$) than Gd-DOTA. This is because covalent bonding of Gd-DOTA complexes to the polymer produces a slow tumbling effect, thus improving the relaxivity. However, the effect of the increased amount of Gd-DOTA introduced into each polymer molecule was negligible. This is because of the high degree of mobility of Gd-DOTA in the polymer or mobility of the polymer as a whole. Interestingly, folding of the polymer by SMDC formation produced a more crowded environment which reduced Gd-DOTA movement, thereby improving the relaxivity. This phenomenon has not been reported in the past and has been demonstrated for the first time by this Example. Moreover, since the effect was minimal when the amount of Gd-DOTA introduced into the SMDC was increased, this suggested the possibility that SMDC-Gd has high relaxivity when the Gd supporting weight is low. Since virtually no effect on relaxivity was observed even in the co-presence of bovine serum albumin (BSA), there was virtually no interaction between SMDC-Gd and PEGA-Gd or proteins, and $r_1/r_2$ was also unaffected. These results indicated that SMDC-Gd can be used as a systemically-administered MRI contrast agent.

Example 6: Pharmacokinetics test for SMDC-Gd

A tumor-bearing model was prepared by subcutaneously transplanting CT26 mouse colorectal cancer cells into mice (Balb/c, 6-week-old, female, SLC), and the model was used for pharmacokinetics testing. A cell suspension that had been subcultured (5% $CO_2$, 37°C) using DMEM medium (containing 10% FBS and 1% penicillin streptomycin) was subcutaneously injected into the mouse dorsal region ($1 \times 10^6$ cells/mouse, DMEM). At 10 days after administration, the tumor size was randomized and each sample was administered to the mouse through the caudal vein (Gd conc. 5 mg/kg, n = 6). After administration, blood was collected and the organs (tumors, liver, spleen, kidneys, pancreases, skin, muscles and brains) were dissected after specified time periods (1, 4, 8, 24 and 72 hours). After sampling, the plasma was collected by centrifugal separation of the blood to prepare a sample. The collected organs were pretreated (heated at 70% nitric acid, heating at 90°C for ≥1 hour, followed by dilution with ultrapure water), and the Gd concentration was measured using ICP-MS.

(1) ICP-MS measurement

Apparatus: Agilent 7700x ICP-MS/Agilent
Pretreatment apparatus: EYELA MG-2300/Tokyo Rikakikai Co., Ltd.
Standard solution: Gadolinium standard solution (Gd1000) for ICP analysis/FujiFilm-Wako
Results: Figs. 18 to 25

Based on comparison of blood retentivities (Fig. 18), the SMDC-$Gd_4$ and PEGA-$Gd_4$ blood retentivity and blood clearance were equivalent, but clearance of SMDC-$Gd_{17}$ from the blood was more rapid. In regard to tumor accumulation (Fig. 19), the Gd concentration at 24-48 hours after of administration of SMDC-$Gd_4$ was at least twice that of SMDC-$Gd_{17}$ and PEGA-$Gd_4$. In the tumor models used in this Example, Gd-DOTA was rapidly excreted from the blood and tumor accumulation was low. These results suggest that intramolecular folding provides a PEG layer surrounding SMDC, thereby inhibiting aggregation and binding with the proteins. In regard to accumulation in different organs (Figs. 20 to 25), SMDC-$Gd_4$ was equivalent to typical nanomedicine, but SMDC-$Gd_{17}$ accumulated more abundantly in the liver, spleen and kidneys. This is because the data potential for SMDC-$Gd_{17}$ is lower than SMDC-$Gd_4$ or PEGA-$Gd_4$. Generally speaking, a neutral or slightly negative surface charge can prevent nonspecific interaction with plasma proteins and avoid excretion by the mononuclear phagocyte system. Since the data potential of SMDC-$Gd_{17}$ was low at -37 mV, however, it was taken up by endocytosis via scavenger receptors into liver nonparenchymal cells such as Kupffer cells and macrophages.

<u>Example 7: Toxicity test for SMDC-Gd</u>

The cytotoxicity of SMDC-Gd was evaluated using a cell counting kit-8 (CCK8). CT26 cells subcultured (5% $CO_2$, 37°C) using DMEM medium (containing 10% FBS and 1% penicillin streptomycin) were seeded in a 96-well plate at 50 μL, 10,000 cells/well, and cultured for 24 hours. Each sample was then added and incubated for 48 hours, after which a CCK8 solution (10 μL) was added, the mixture was further incubated for 1 hour, and the absorbance at 450 nm was measured with a plate reader.

(1) Absorbance measurement

Apparatus: iMark microplate reader/Bio-Rad Laboratories
Results: Fig. 26

Mice (Balb/c, 6-week-old, female, SLC) were used for an SMDC-Gd toxicity test. Each sample was administered to the mice through the caudal vein (Gd conc. 0.1 mmol/kg, n = 5). At 4 and 48 hours of administration, blood was collected, mixing 90 μL with 10 μL of an ethylenediaminetetraacetic acid (EDTA) solution (EDTA conc. = 12 mg/mL, solvent: ultrapure water), and the leukocytes (WBC), erythrocytes (RBC), hemoglobin (HGB), hematocrit (HCT) and platelets (PLT) were measured using a multi-item automatic blood cell counter for animals. After obtaining blood plasma by centrifugal separation from the remaining blood, a dry clinical chemical analyzer was used to measure blood urea nitrogen (BUN), creatinine (CRE), glutamate-pyruvate transaminase (GPT), glutamate-oxaloacetate transaminase (GOT), alkaline phosphatase (ALP) and total protein (TP).

(1) WBC, RBC, HGB, HCT and PLT measurement

Apparatus: Automatic multiple blood cell counter pocH-100iV Diff/Sysmex
Results: Figs. 27 to 31

(2) BUN, CRE, GPT, GOT, ALP and TP measurement

Apparatus: Dri-Chem 7000IZ/FujiFilm-Wako
Results: Figs. 32 to 37

Upon evaluating physiological parameters (Figs. 27 to 37) for SMDC-Gd cytotoxicity (Fig. 26), it was found that no acute effect is exhibited at clinically applicable doses (0.1 mmol/kg in terms of Gd groups).

Example 8: MRI experiment for SMDC-Gd

CT26 mouse colorectal cancer cells were subcutaneously grafted into mice (BALB/c nu/nu mice, 5-week-old, female, SLC) to create a tumor-bearing model which was used for MRI. A CT26 cell suspension that had been subcultured (5% $CO_2$, 37°C) using DMEM medium (containing 10% FBS and 1% penicillin streptomycin) was subcutaneously injected into the mouse dorsal region ($1 \times 10^6$ cells/mouse, Hank's balanced salt solution). At 1 week after administration, the tumor size was randomized, and then each sample was administered to the mouse through the caudal vein (Gd conc. 0.1, 0.05, 0.03 and 0.01 mmol/kg). MRI imaging ($R_1$ mapping, $T_1$-weighted image) was carried out under isoflurane anesthesia.

(1) $R_1$ mapping imaging

Apparatus: Horizontal 1.0 T Bruker ICON MRI system/Bruker
Pulse sequence: Rapid acquisition with relaxation enhancement (RARE)-based inversion recovery sequence
Measuring conditions:

$$\text{Echo time (TE)} = 10\ \text{ms};$$

$$\text{Repetition time (TR)} = 10{,}000\ \text{ms};$$

Inversion Time (TI) = 100, 300, 500, 700, 900, 1300, 1700, 1900, 2100, 2500, 2900, 3300, 3700 ms

$$\text{RARE Factor} = 4;$$

$$\text{Field of view (FOV)} = 28.0 \times 16.0\ \text{mm}^2;$$

$$\text{Slice thickness} = 2.0\ \text{mm};$$

$$\text{Pixel count} = 70 \times 40;$$

$$\text{Resolving power (in-plane resolution)} = 0.4 \times 0.4\ \text{mm}^2$$

$$\text{Number of excitations} = 1;$$

Fat suppression mode: OFF;
FOV saturation: ON
Measuring points: Before administration and 20, 40, 60 minutes and 24 hours after administration
Results: Figs. 38 to 42

Quantitative mapping image for the longitudinal relaxation rate (Fig. 38) confirmed the superiority of SMDC-$Gd_4$ over Gd-DOTA. The high $R_1$ value shown as red at the center of the image is adipose tissue, and it can be reduced and removed using the fat suppression technique during MRI imaging. Upon examining the longitudinal relaxation rate of the tumors and comparing the longitudinal relaxation rates for tumors and muscles (Fig. 39 and Fig. 40), SMDC-$Gd_4$ was found to exhibit a high contrast effect even in tumor tissue, and the tumor/muscle ratio (T/M ratio) of the relaxation rate 1 hour after administration was 1.17. On the other hand, the T/M ratio at 1 hour after administration of Gd-DOTA was 0.77, and therefore the T/M ratio for SMDC-$Gd_4$ was significantly higher than for Gd-DOTA. The T/M ratio for SMDC-$Gd_4$ increased to 1.36 within 24 hours after administration. While PEGA-Gd4 also exhibited a clear MR image, the $R_1$ value tended to be dose-dependent, and the $R_1$ value was consistently lower than SMDC-$Gd_4$ during the 24 hours.

(1) $T_1$-weighted image

Apparatus: Permanent magnet type: 1.0T Bruker ICON MRI system/Bruker
Pulse sequence: Spin-echo sequence
Measuring conditions:

$$\text{Echo time (TE)} = 7\ \text{ms};$$

$$\text{Repetition time (TR)} = 400\ \text{ms}$$

$$\text{Field of view (FOV)} = 30.0 \times 30.0\ \text{mm}^2;$$

$$\text{Slice thickness} = 1.0\ \text{mm};$$

$$\text{Number of slices} = 20;$$

$$\text{Slice interval} = 1.0\ \text{mm};$$

$$\text{Pixel count} = 150 \times 150;$$

$$\text{In-plane resolution} = 0.2 \times 0.2\ \text{mm}^2$$

$$\text{Number of excitations} = 4;$$

Fat suppression mode: OFF
FOV saturation: OFF
Measuring points: Before administration and 0-60 minutes after administration
Results: Figs. 43 to 45

Upon evaluating time-dependent change in the MR signal in the kidneys and bladders up to 1 hour after administration (Figs. 43 to 45), the MR signal in the kidneys showed the same tendency as for accumulation amount in the kidneys (Fig. 20), but the difference in clearance time between the kidneys and bladders reflects the difference in permeability of SMDC-$Gd_4$ and PEGA-$Gd_4$ for the negatively charged glomerular basement membrane (GBM). SMDC-$Gd_4$ and PEGA-$Gd_4$ had approximately the same surface charge, but SMDC-$Gd_4$ was designed to have a diameter of about 6 nm by folding, which was within the threshold of particle sizes capable of passing through the GBM. On the other hand, PEGA-$Gd_4$ was in a soft polymer state, since no peak could be confirmed in the Kratky plots of the SAXS analysis example in aqueous solution (Fig. 12). Even within the threshold of particle sizes capable of permeating the GBM, therefore, this property of the single polymer chain can potentially significantly affect behavior at the GBM.

Example 9: Pharmacokinetics test experiment for predicting therapeutic effect of neutron capture therapy (NCT) utilizing Gd

A tumor-bearing model was prepared by subcutaneous grafting of CT26 mouse colorectal cancer cells into mice (Balb/c, 6-week-old, female, SLC) by the same procedure as for the SMDC-Gd pharmacokinetics test, and a pharmacokinetics test was conducted as an experiment for Gd-NCT. In this case, however, each sample was administered once or 3 times through the caudal vein of the mouse (0, 24 and 48 hours afterwards, Gd conc. 0.1 mmol/kg, n = 5), with blood collection and organ dissection and collection (tumors, liver and kidneys) 24 hours after the final administration. Each organ was pretreated by the same procedure as described above, and the Gd concentration was also measured in the same manner using ICP-MS.

(1) ICP-MS measurement

Apparatus: Agilent 7700x ICP-MS/Agilent
Pretreatment apparatus: EYELA MG-2300/Tokyo Rikakikai Co., Ltd.
Standard solution: Gadolinium standard solution (Gd1000) for ICP analysis/FujiFilm-Wako
Results: Fig. 46 and Fig. 47

$^{157}$Gd can be applied for neutron capture therapy, but it has not yet been applied in the clinic for treatment of tumors. One reason for this is that the intratumoral $^{157}$Gd concentration must be high in order to obtain a therapeutic effect. The intratumoral Gd concentration with different numbers of SMDC-$Gd_4$ administrations was therefore evaluated. It was confirmed that with a single dose of SMDC-$Gd_4$, 41.2 ppm of Gd (6.4 ppm in terms of $^{157}$Gd groups) had been delivered into the tumor 24 hours after injection, and that with 3 doses given every 24 hours, the Gd concentration had increased to 91.2 ppm (14.3 ppm in terms of $^{157}$Gd groups) after the final 24 hours.

Example 10: Gd-NCT experiment for SMDC-Gd

A tumor-bearing model prepared by subcutaneously grafting CT26 mouse colorectal cancer cells into mice (Balb/c, 6-week-old, female, SLC) was used in an experiment for Gd-NCT. A CT26 cell suspension was injected into the mouse right femoral region ($2 \times 10^5$ cells/mouse, DMEM). After approximately 2 weeks, each sample was administered 3 times through the caudal vein of the mouse (after 0, 24 and 48 hours, Gd conc. 0.1 mmol/kg, n = 5). A PBS-administered group was used as a comparative control. At 72 hours after the first administration, the holder in which the mouse had been placed was set on a 5 mm-thick thermoplastic sheet containing 40 wt% $^6$LiF (96% $^6$Li) for shielding of thermal neutrons and irradiated with neutron rays for 10 minutes only at the tumor site of the right femoral region (5 MW, Kyoto University Research Reactor, fluence: $2.87 \times 10^{12}$ to $3.29 \times 10^{12}$ thermal neutron/cm$^2$, $5.10 \times 10^{11}$ to $5.86 \times 10^{11}$ epithermal neutron/cm$^2$). The antitumor effect was confirmed by time-dependent change in tumor volume, and toxicity was confirmed by change in mouse body weight.

(1) Gd-NCT

In regard to the antitumor effect and change in body weight due to Gd-NCT (Fig. 48 and Fig. 49), three groups: Gd-DOTA, PBS and no administration + no irradiation, failed to exhibit sufficient antitumor activity, whereas SMDC-Gd4 suppressed tumor growth without body weight loss. This was because SMDC-$Gd_4$ exhibited a therapeutic effect by NCT and delivered a sufficient amount of $^{157}$Gd to the tumor tissue.

All of the publications and patent literature cited herein are incorporated into the present specification in their entirety as reference. The specific embodiments of the invention were explained in the present specification for the purpose of example, and it will be readily appreciated by a person skilled in the art that various modifications may be employed such as are not outside of the spirit and scope of the invention.

References

[Chemical Formula 13]

[1] Stabile, A. et al. Multiparametric MRI for prostate cancer diagnosis: current status and future directions. Nat. Rev. Urol. 17, 41-61 (2020).

[2] Sclafani, F. et al. Comparison between MRI and pathology in the assessment of tumour regression grade in rectal cancer. Br. J. Cancer 117, 1478-1485 (2017).

[3] Wahsner, J. et al. Chemistry of MRI contrast agents: current challenges and new frontiers. Chem. Rev. 119, 957-1057 (2018).

[4] Jeon, M. et al. Iron oxide nanoparticles as T1 contrast agents for magnetic resonance imaging: fundamentals, challenges, applications, and prospectives. Adv. Mater. 33, 1906539 (2021).

[5] Tang, J. et al. Macromolecular MRI contrast agents: structures, properties and applications. Prog. Polym. Sci. 38, 462-502 (2013).

[6] Wang, A. Z., Langer, R. & Farokhzad, O. C. Nanoparticle delivery of cancer drugs. Annu. Rev. Med. 63, 185-198 (2012).

[7] Cheng, C. J. & Saltzman, W. M. Downsizing tumour therapeutics. Nat. Nanotechnol. 7, 346-347 (2012).

[8] Kanasty, R. et al. Delivery materials for siRNA therapeutics. Nat. Mater. 12, 967-977 (2013).

[9] Nishiyama, N., Matsumura, Y. & Kataoka, K. Development of polymeric micelles for targeting intractable cancers. Cancer Sci. 107, 867-874 (2016).

[10] Cho, K. et al. Therapeutic nanoparticles for drug delivery in cancer. Clin. Cancer Res. 14, 1310-1316 (2008).

[11] Verwilst, P. et al. Recent advances in Gd-chelate based bimodal optical/MRI contrast agents. Chem. Soc. Rev. 44, 1791-1806 (2015).

[12] Cabral, H. et al. Accumulation of sub-100 nm polymeric micelles in poorly permeable tumours depends on size. Nat. Nanotechnol. 6, 815-823 (2011).

[13] Lu, Y. et al. Micelles with ultralow critical micelle concentration as carriers for drug delivery. Nat. Biomed. Eng. 2, 318-325 (2018).

[14] Cai, Y. et al. The in vivo fate of polymeric micelles. Adv. Drug Delivery Rev. 188, 114463 (2022).

[15] Yu, M. & Zheng, J. Clearance pathways and tumor targeting of imaging nanoparticles. ACS nano 9, 6655-6674 (2015).

[16] Terashima, T. & Sawamoto, M. Single-Chain Polymer Nanoparticles: Synthesis, Characterization, Simulations, and Applications Ch 8. Single-chain nanoparticles via self-folding amphiphilic copolymers in water. (Wiley-VCH, 2017).

[Chemical Formula 14]

[17] Imai, S. et al. Programmed self-assembly systems of amphiphilic random copolymers into size-controlled and thermoresponsive micelles in water. Macromolecules 51, 398-409 (2018).

[18] Hattori, G. et al. Self-assembly of PEG/dodecyl-graft amphiphilic copolymers in water: consequences of the monomer sequence and chain flexibility on uniform micelles. Polym. Chem. 8, 7248-7259 (2017).

[19] Swaminathan, S. & Shah, S. V. New insights into nephrogenic systemic fibrosis. J. Am. Soc. Nephrol. 18, 2636-2643 (2007).

[20] Robert, P. et al. T1-weighted hypersignal in the deep cerebellar nuclei after repeated administrations of gadolinium-based contrast agents in healthy rats: difference between linear and macrocyclic agents. Invest. Radiol. 50, 473-480 (2015).

[21] Robert, P. et al. Linear gadolinium-based contrast agents are associated with brain gadolinium retention in healthy rats. Invest. Radiol. 51, 73-82 (2016).

[22] Hirai, Y. et al. Precision self-assembly of amphiphilic random copolymers into uniform and self-sorting nanocompartments in water. Macromolecules 49, 5084-5091 (2016).

[23] Dreher, M. R. et al. Tumor vascular permeability, accumulation, and penetration of macromolecular drug carriers. J. Natl. Cancer Inst. 98, 335-344 (2006).

[24] Kobayashi, H. & Brechbiel, M. W. Dendrimer-based nanosized MRI contrast agents. Curr. Pharm. Biotechnol. 5, 539-549 (2004).

[25] Kobayashi, H. & Brechbiel, M. W. Nano-sized MRI contrast agents with dendrimer cores. Adv. Drug Delivery Rev. 57, 2271-2286 (2005).

[26] Zhang, Z. et al. Multilocus binding increases the relaxivity of protein-bound MRI contrast agents. Angew. Chem., Int. Ed. 44, 6766-6769 (2005).

[27] Caravan, P., Farrar, C. T. & Frullano, L. Uppal R. Influence of molecular parameters and increasing magnetic field strength on relaxivity of gadolinium-and manganese-based T1 contrast agents. Contrast Media Mol. Imaging 4, 89-100 (2009).

[28] Tóth, É. et al. The role of water exchange in attaining maximum relaxivities for dendrimeric MRI contrast agents. Chem. - Eur. J. 2, 1607-1615 (1996).

[29] Botta, M. & Tei, L. Relaxivity enhancement in macromolecular and nanosized GdIII-based MRI contrast agents. Eur. J. Inorg. Chem. 2012, 1945-1960 (2012).

[30] Uchino, H. et al. Cisplatin-incorporating polymeric micelles (NC-6004) can reduce nephrotoxicity and neurotoxicity of cisplatin in rats. Br. J. Cancer 93, 678-687 (2005).

[31] Hamaguchi, T. et al. NK105, a paclitaxel-incorporating micellar nanoparticle formulation, can extend in vivo antitumour activity and reduce the neurotoxicity of paclitaxel. Br. J. Cancer 92, 1240-1246 (2005).

[32] Takahashi, A. et al. NC-6300, an epirubicin-incorporating micelle, extends the antitumor effect and reduces the cardiotoxicity of epirubicin. Cancer Sci. 104, 920-925 (2013).

[33] Song, G. et al. Emerging nanotechnology and advanced materials for cancer radiation therapy. Adv. Mater. 29, 1700996 (2017).

## Claims

**1.** A polymeric micelle composed of a self-aggregating single polymer chain, comprising one to several gadolinium complexes, and spontaneously folding in water to form a micelle with a diameter of 10 nm or smaller.

**2.** The polymeric micelle composed of the self-aggregating single polymer chain according to claim 1, wherein:

(A) a side chain containing polyethylene glycol (PEG) or a PEG derivative;
(B) a side chain containing a hydrophobic group; and
(C) a side chain containing a gadolinium complex;

are bonded to the main backbone either via a linker or directly.

**3.** The polymeric micelles composed of the self-aggregating single polymer chain according to claim 2, wherein

(D) a side chain containing a carboxylic acid group
is also bonded to the main backbone either via a linker or directly.

**4.** Polymeric micelles composed of self-aggregating single polymer chain according to claim 2, wherein the PEG or PEG derivative in the side chain (A) has the following structure:

[Chemical Formula 1]

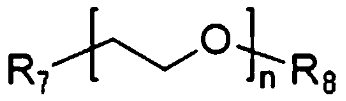

; or

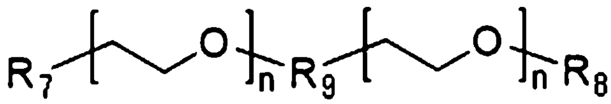

(where

$R_7$ is a methoxy, ethoxy, acetyl or benzyl group;
$R_8$ is an amide bond, ether bond or ester bond, being the portion that binds to the main backbone;
$R_9$ is a biodegradable linker; and
n is 1 to 10).

**5.** The polymeric micelle composed of the self-aggregating single polymer chain according to claim 2, wherein the hydrophobicity (cLogP value) of the hydrophobic groups in the side chain (B) is 1 to 10.

**6.** The polymeric micelle composed of the self-aggregating single polymer chain according to claim 2, wherein the hydrophobic group in the side chain (B) is selected from the group consisting of phenyl, naphthyl, pyrenyl, anthracene, cholesterol, steroid derivatives, 1-pyrenemethylamine, 1-ethyl-3-(3-dimethylaminopropyl)carbodiimide hydrochloride, 1-naphthylmethylamine, DL-α-tocopherol, nerol, farnesol and anticancer agents.

**7.** The polymeric micelle composed of the self-aggregating single polymer chain according to any one of claims 1 to 6, wherein the gadolinium complex is one or more selected from the group consisting of gadoteridol, meglumine gadoterate and gadobutrol.

**8.** The polymeric micelle composed of the self-aggregating single polymer chain according to any one of claims 2 to 6, wherein when the numbers of repeating units of the side chains (A) to (D) in a single polymer chain are represented as a, b, c and d respectively, the following conditions are satisfied:

(I) when the cLogP value is less than 1.5 and the total degree of polymerization (DP) of a + b + c + d is 100 to 500,

a is 50% or greater of the total DP;
b is 40% or lower of the total DP;
c is 10% or lower of the total DP; and
d is 10% or lower of the total DP;

with the proviso that c and d make up 1% ≤ c + d ≤ 20% of the total DP;
(II) when the cLogP value is 1.5 or greater and less than 4.5 and the total degree of polymerization (DP) of a + b + c + d is less than 200,

a is 50% or greater of the total DP;
b is 30% or lower of the total DP;
c is 5% or lower of the total DP; and
d is 15% or lower of the total DP;

with the proviso that c and d make up 1% ≤ c + d ≤ 20% of the total DP;
(III) when the cLogP value is 1.5 or greater and less than 4.5 and the total degree of polymerization (DP) of a + b + c + d is 200 to 500,

a is 50% or greater of the total DP;
b is 40% or lower of the total DP;
c is 5% or lower of the total DP; and
d is 15% or lower of the total DP,

with the proviso that c and d make up 1% ≤ c + d ≤ 20% of the total DP;

(IV) when the cLogP value is 4.5 or greater and less than 5.5 and the total degree of polymerization (DP) of a + b + c + d is 200 to 500,

a is 50% or greater of the total DP;
b is 30% or lower of the total DP;
c is 5% or lower of the total DP; and
d is 15% or lower of the total DP, with the proviso that c and d make up 1% ≤ c + d ≤ 20% of the total DP; or

(V) when the cLogP value is 5.5 or greater and the total degree of polymerization (DP) of a + b + c + d is 200 to 500,

a is 60% or greater of the total DP;
b is 20% or lower of the total DP;
c is 2% or lower of the total DP; and
d is 8% or lower of the total DP, with the proviso that c and d make up 1% ≤ c + d ≤ 10% of the total DP.

**9.** The polymeric micelle composed of the self-aggregating single polymer chain according to any one of claims 1 to 6, wherein the association number of the single polymer chain in water or buffer is less than 1.5, as calculated from the ratio of $M_{w,MALS}$ (PBS)/$M_{w,MALS}$ (chloroform).

**10.** The polymeric micelle composed of self-aggregating single polymer chain according to any one of claims 1 to 6, wherein the main chain of the single polymer chain is selected from the group consisting of polyethers, polyesters, polyalkylenes, polycarbonates, poly(dioxanone), polyacid anhydrides, polyhydroxy acids, polyfumarate, polycaprolactone, polyamides, polyacetals, poly(orthoesters), polyhydroxybutyrate, polyvinyl alcohol, polyurethanes, polyphosphazenes, polyacrylates, polymethacrylates, polycyanoacrylate, polyureas, polystyrene, polyamines, poly(arylates), polycarbonates, poly(propylene fumarate), polyhydroxyalkanoates, polyketals, polyesteramides, polyhydroxyvalerates, polyorthocarbonates, poly(vinylpyrrolidone), polyalkylene oxalates, polyalkylene succinate, polylactic acid, poly(malic acid), poly(methylvinyl ether) and poly(maleic anhydride).

**11.** The polymeric micelle composed of self-aggregating single polymer chain, comprising one to several gadolinium complexes, spontaneously folding in water to form micelles with diameters of 10 nm or smaller, and having the following structure:

[Chemical Formula 2]

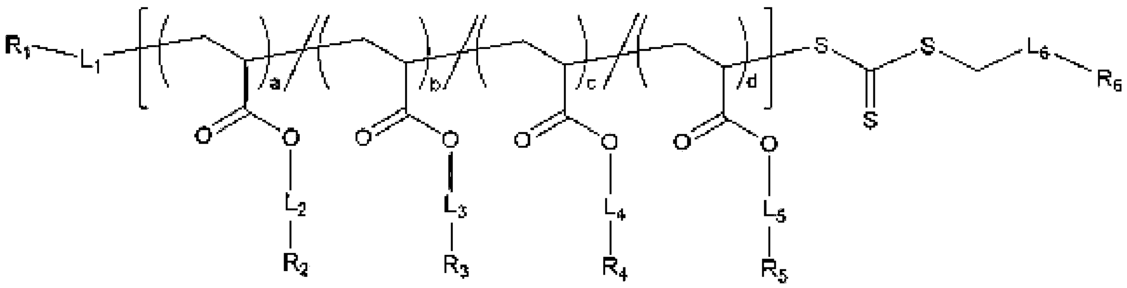

(1)

or

[Chemical Formula 3]

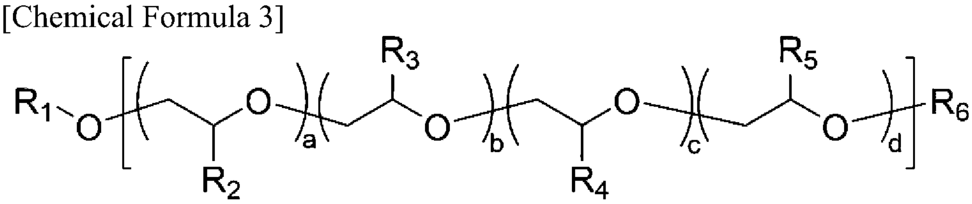

(2)

(where

R1 and R6 are each independently a methoxy, ethoxy, acetyl or benzyl group;

$R_2$ is PEG or a PEG derivative;
$R_3$ is a hydrophobic group;
$R_4$ is a gadolinium complex;
$R_5$ is a carboxylic acid group;
$L_1$ to $L_6$ are each independently not present or a linker; and
a, b, c and d satisfy any of the following conditions (I) to (V):

(I) when the cLogP value is less than 1.5 and the total degree of polymerization (DP) of a + b + c + d is 100 to 500,

a is 50% or greater of the total DP;
b is 40% or lower of the total DP;
c is 10% or lower of the total DP; and
d is 10% or lower of the total DP, with the proviso that c and d make up 1% ≤ c + d ≤ 20% of the total DP;

(II) when the cLogP value is 1.5 or greater and less than 4.5 and the total degree of polymerization (DP) of a + b + c + d is less than 200,

a is 50% or greater of the total DP;
b is 30% or lower of the total DP;
c is 5% or lower of the total DP; and
d is 15% or lower of the total DP, with the proviso that the c and d make up 1% ≤ c + d ≤ 20% of the total DP;

(III) when the cLogP value is 1.5 or greater and less than 4.5 and the total degree of polymerization (DP) of a + b + c + d is 200 to 500,

a is 50% or greater of the total DP;
b is 40% or lower of the total DP;
c is 5% or lower of the total DP; and
d is 15% or lower of the total DP, with the proviso that c and d make up 1% ≤ c + d ≤ 20% of the total DP;

(IV) when the cLogP value is 4.5 or greater and less than 5.5 and the total degree of polymerization (DP) of a + b + c + d is 200 to 500,

a is 50% or greater of the total DP;
b is 30% or lower of the total DP;
c is 5% or lower of the total DP; and
d is 15% or lower of the total DP, with the proviso that c and d make up 1% ≤ c + d ≤ 20% of the total DP;

(V) when the cLogP value is 5.5 or greater and the total degree of polymerization (DP) of a + b + c + d is 200 to 500,

a is 60% or greater of the total DP;
b is 20% or lower of the total DP;
c is 2% or lower of the total DP; and
d is 8% or lower of the total DP, with the proviso that c and d make up 1% ≤ c + d ≤ 10% of the total DP.

**12.** The polymeric micelle composed of self-aggregating single polymer chain according to claim 11, wherein the PEG or PEG derivative has the following structure:

[Chemical Formula 4]

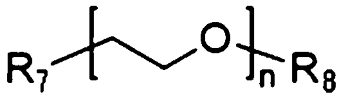

; or

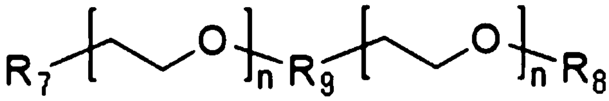

(where

$R_7$ is a methoxy, ethoxy, acetyl or benzyl group;
$R_8$ is an amide bond, ether bond or ester bond, being the portion that binds to the main backbone;
$R_9$ is a biodegradable linker; and
n is 1 to 10).

**13.** The polymeric micelle composed of self-aggregating single polymer chain according to claim 11 or 12, wherein the hydrophobicity (cLogP value) of the hydrophobic groups is 1 to 10.

**14.** The polymeric micelle composed of self-aggregating single polymer chain according to claim 11 or 12, wherein the hydrophobic group is selected from the group consisting of phenyl, naphthyl, pyrenyl, anthracene, cholesterol, steroid derivatives, 1-pyrenemethylamine, 1-ethyl-3-(3-dimethylaminopropyl)carbodiimide hydrochloride, 1-naphthylmethylamine, DL-α-tocopherol, nerol, farnesol and anticancer agents.

**15.** The polymeric micelle composed of self-aggregating single polymer chain according to claim 11 or 12, wherein the gadolinium complex is one or more selected from the group consisting of gadoteridol, meglumine gadoterate and gadobutrol.

**16.** The polymeric micelle composed of self-aggregating single polymer chain according to claim 11 or 12, wherein the association number of single polymer chain in water or buffer is less than 1.5, as calculated from the ratio of $M_w$ (PBS)/Mw (chloroform).

**17.** The polymeric micelle composed of self-aggregating single polymer chain according to any one of claims 1 to 6, 11 and 12, wherein when used in magnetic resonance imaging (MRI) for detection or diagnosis of cancer or a tumor, r1 is 20 $s^{-1}mM^{-1}$ or greater while maintaining longitudinal relaxivity (r1)/transverse relaxivity (r2) ≥ 0.70.

**18.** A pharmaceutical composition for treatment of cancer or tumor by neutron capture therapy, comprising polymeric micelles composed of self-aggregating single polymer chain according to any one of claims 1 to 6, 11 and 12.

Fig. 1

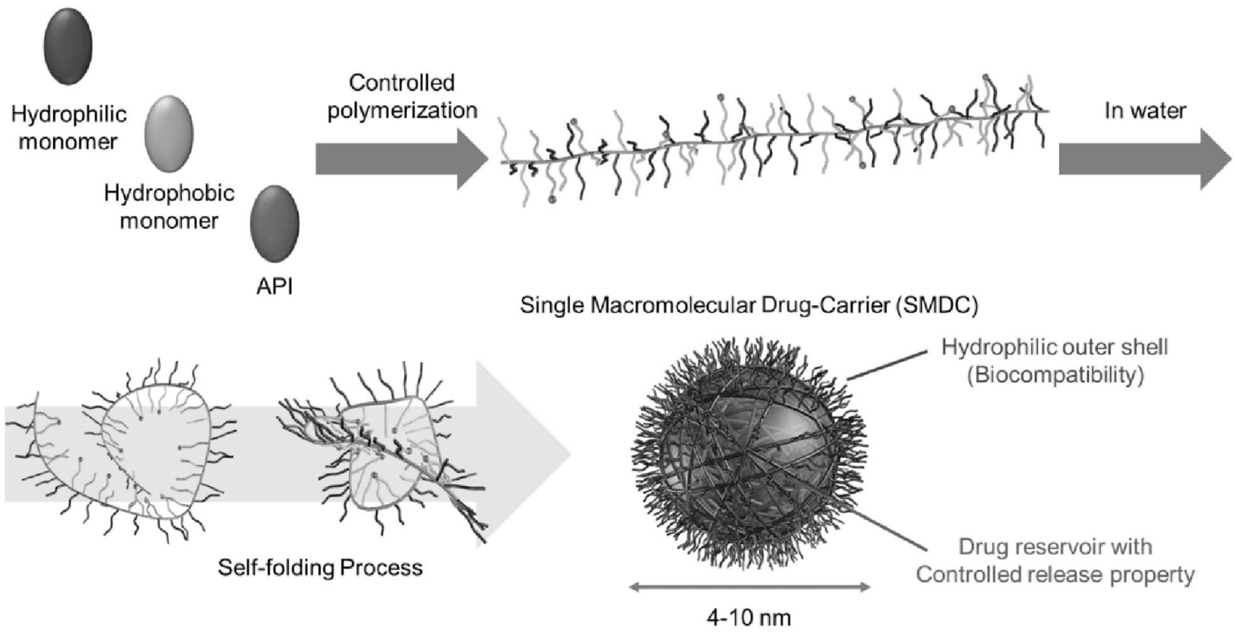
Hydrophilic monomer
Hydrophobic monomer
API
Controlled polymerization
In water
Single Macromolecular Drug-Carrier (SMDC)
Self-folding Process
Hydrophilic outer shell (Biocompatibility)
Drug reservoir with Controlled release property
4-10 nm

Fig. 2

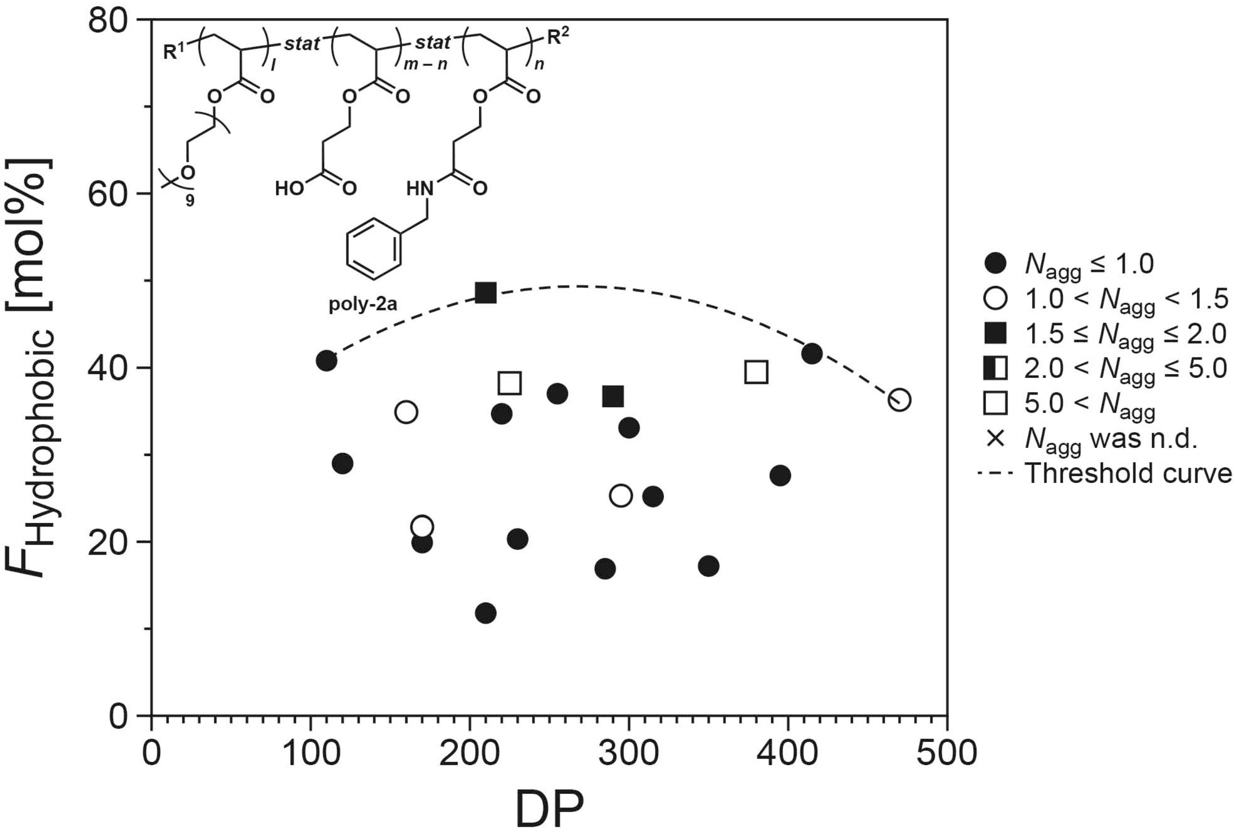
80
60
40
20
0
0
100
200
300
400
500
DP
F Hydrophobic [mol%]
stat
stat
poly-2a
Nagg ≤ 1.0
1.0 < Nagg < 1.5
1.5 ≤ Nagg ≤ 2.0
2.0 < Nagg ≤ 5.0
5.0 < Nagg
Nagg was n.d.
Threshold curve

Fig. 3

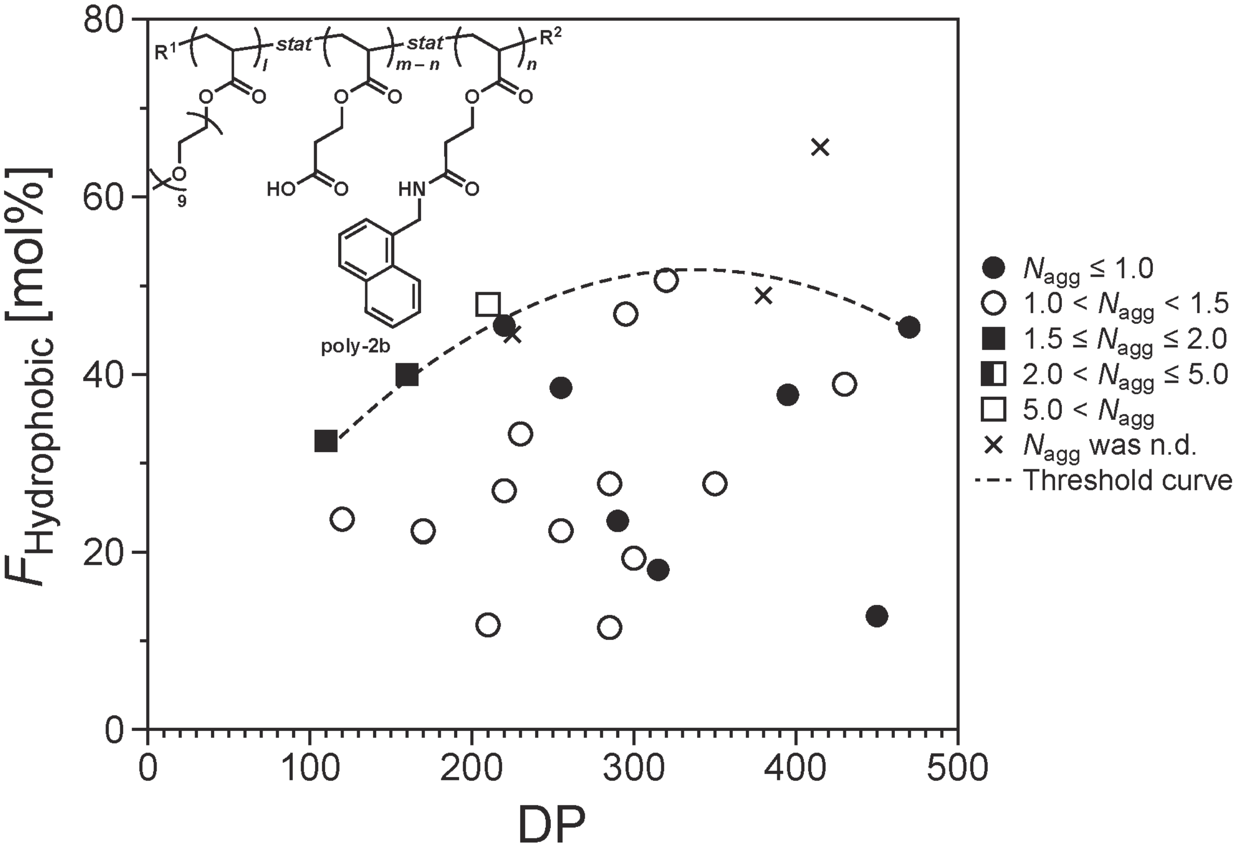
poly-2b
$F_{Hydrophobic}$ [mol%]
0
20
40
60
80
DP
0
100
200
300
400
500
$N_{agg}$ ≤ 1.0
1.0 < $N_{agg}$ < 1.5
1.5 ≤ $N_{agg}$ ≤ 2.0
2.0 < $N_{agg}$ ≤ 5.0
5.0 < $N_{agg}$
$N_{agg}$ was n.d.
Threshold curve

Fig. 4

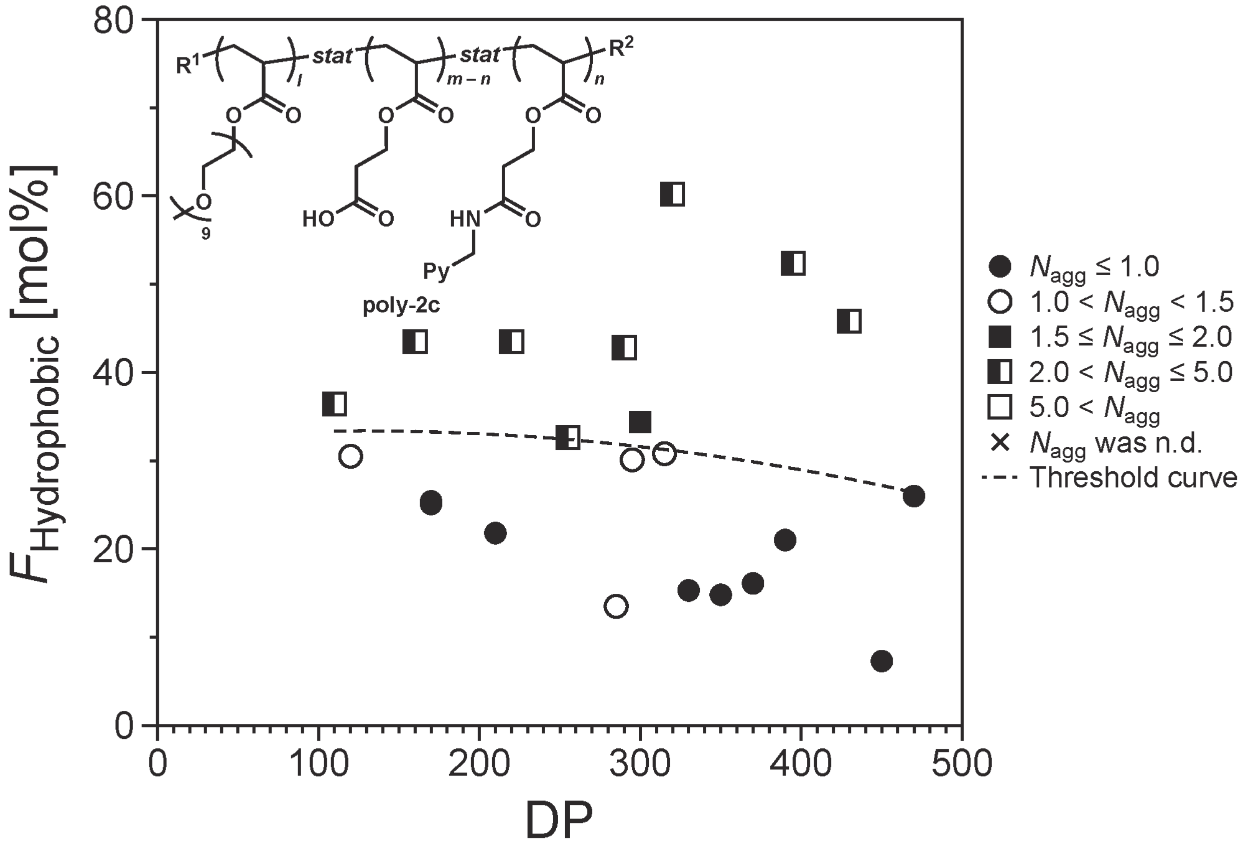
poly-2c
$F_{Hydrophobic}$ [mol%]
DP
0
20
40
60
80
100
200
300
400
500
$N_{agg} \leq 1.0$
$1.0 < N_{agg} < 1.5$
$1.5 \leq N_{agg} \leq 2.0$
$2.0 < N_{agg} \leq 5.0$
$5.0 < N_{agg}$
$N_{agg}$ was n.d.
Threshold curve

Fig. 5

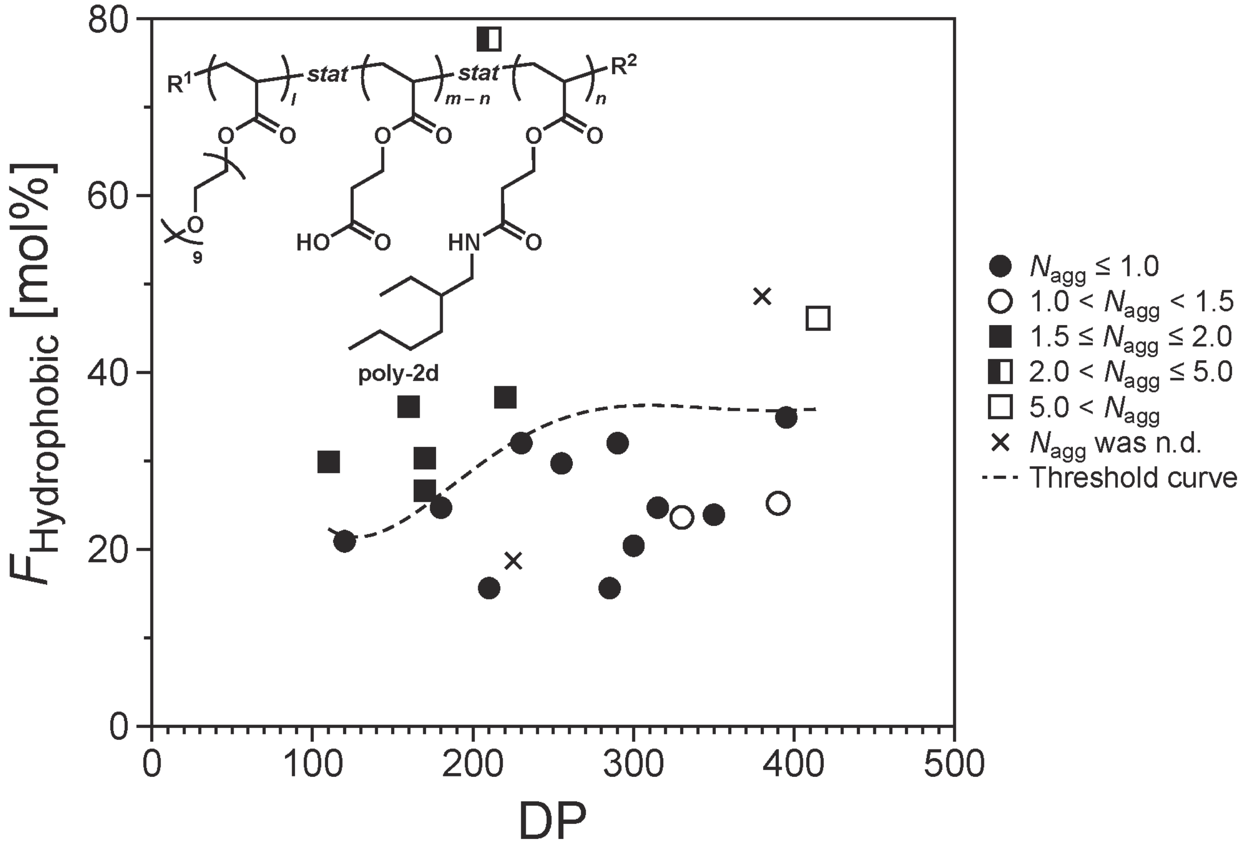
80
60
40
20
0
0
100
200
300
400
500
DP
$F_{Hydrophobic}$ [mol%]
poly-2d
$N_{agg} \leq 1.0$
$1.0 < N_{agg} < 1.5$
$1.5 \leq N_{agg} \leq 2.0$
$2.0 < N_{agg} \leq 5.0$
$5.0 < N_{agg}$
$N_{agg}$ was n.d.
Threshold curve

Fig. 6

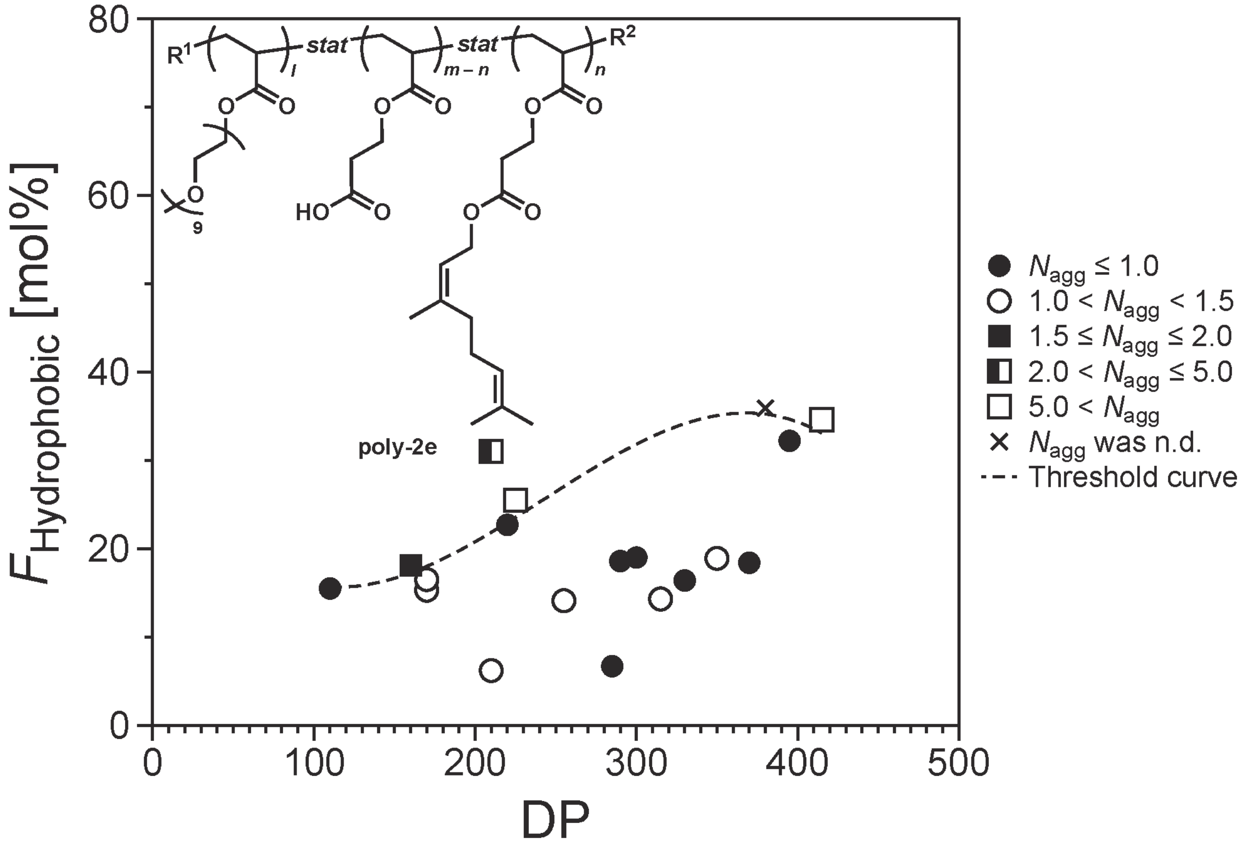

F_Hydrophobic [mol%]
80
60
40
20
0
poly-2e
0
100
200
300
400
500
DP
N_agg ≤ 1.0
1.0 < N_agg < 1.5
1.5 ≤ N_agg ≤ 2.0
2.0 < N_agg ≤ 5.0
5.0 < N_agg
N_agg was n.d.
Threshold curve

Fig. 7

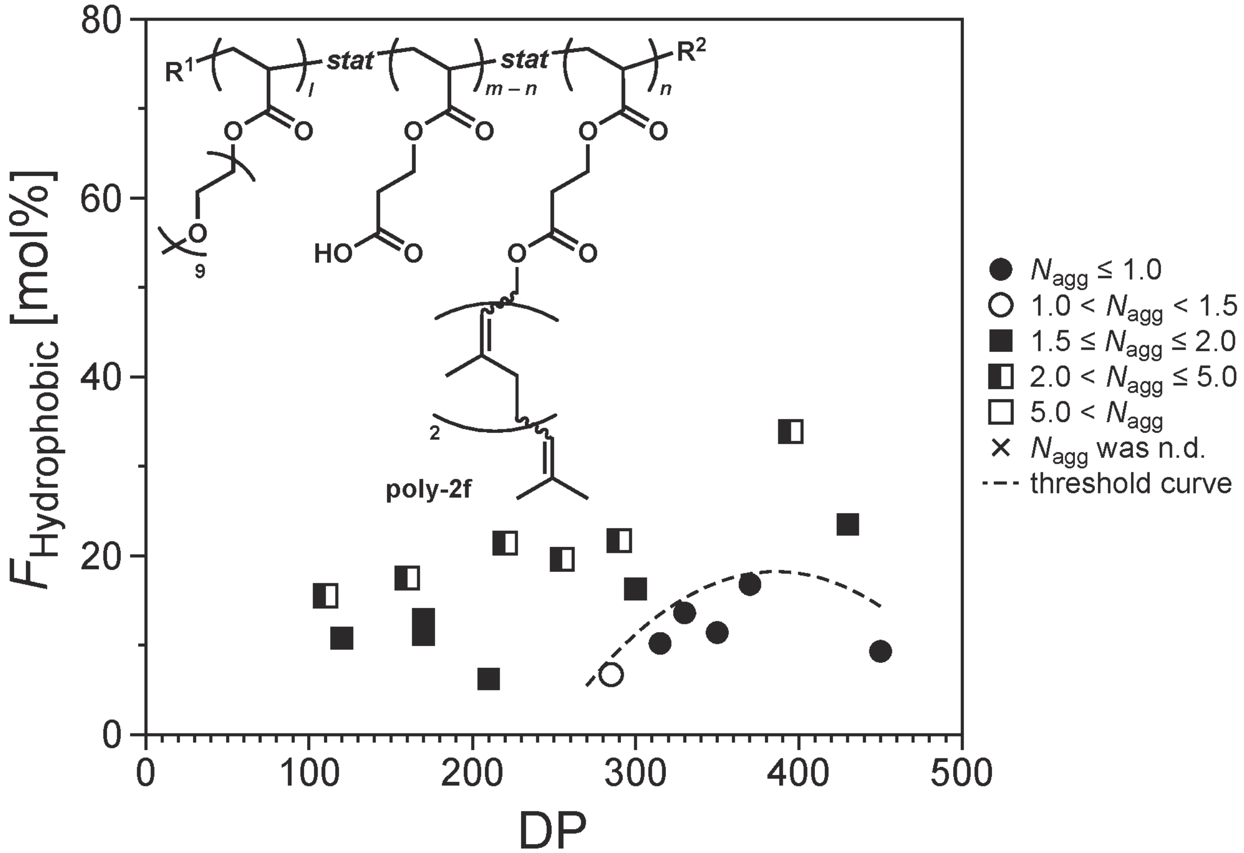

poly-2f
$F_{Hydrophobic}$ [mol%]
DP
0
20
40
60
80
0
100
200
300
400
500
$N_{agg}$ ≤ 1.0
1.0 < $N_{agg}$ < 1.5
1.5 ≤ $N_{agg}$ ≤ 2.0
2.0 < $N_{agg}$ ≤ 5.0
5.0 < $N_{agg}$
$N_{agg}$ was n.d.
threshold curve

Fig. 8

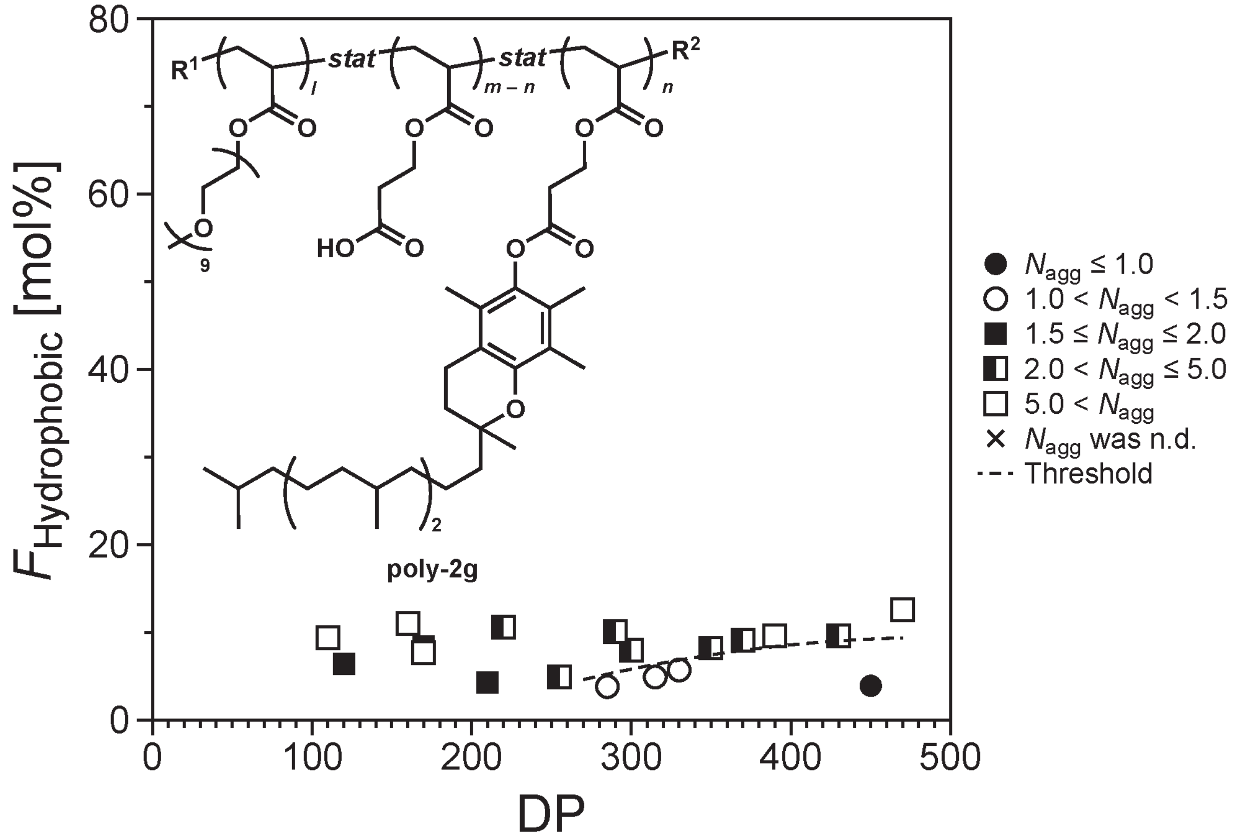
$F_{Hydrophobic}$ [mol%]
80
60
40
20
0
0
100
200
300
400
500
DP
poly-2g
$N_{agg}$ ≤ 1.0
1.0 < $N_{agg}$ < 1.5
1.5 ≤ $N_{agg}$ ≤ 2.0
2.0 < $N_{agg}$ ≤ 5.0
5.0 < $N_{agg}$
$N_{agg}$ was n.d.
Threshold

Fig. 9

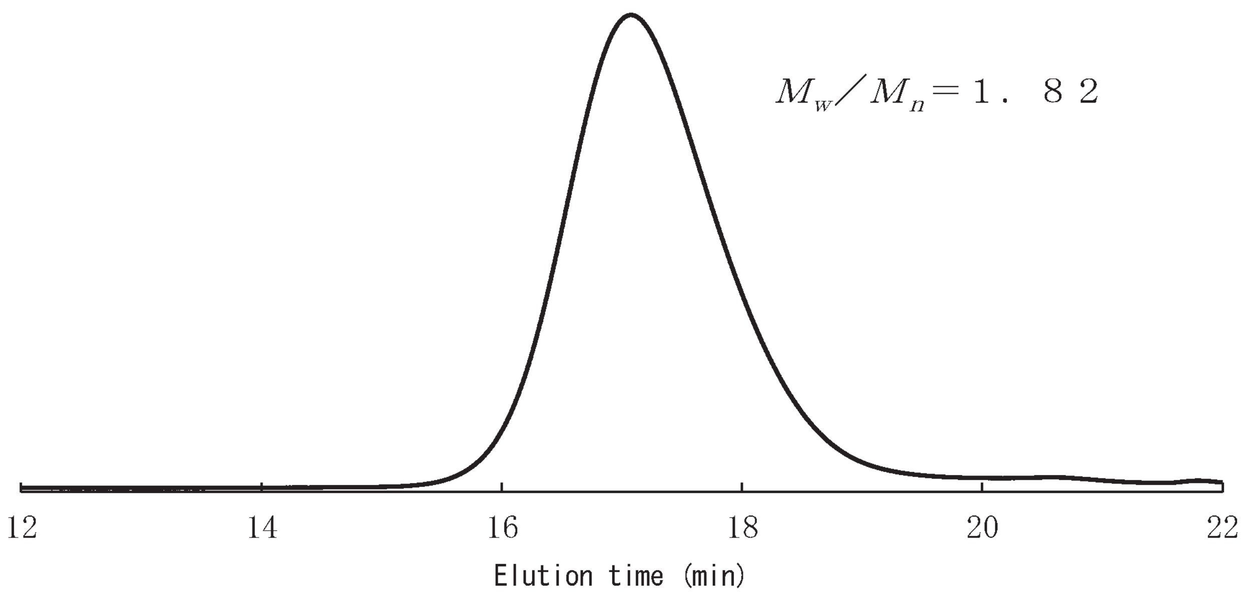
$M_w/M_n$ = 1. 8 2
12
14
16
18
20
22
Elution time (min)

# Fig. 10

BZA100-PEGA90-CEA10(DOTA)4

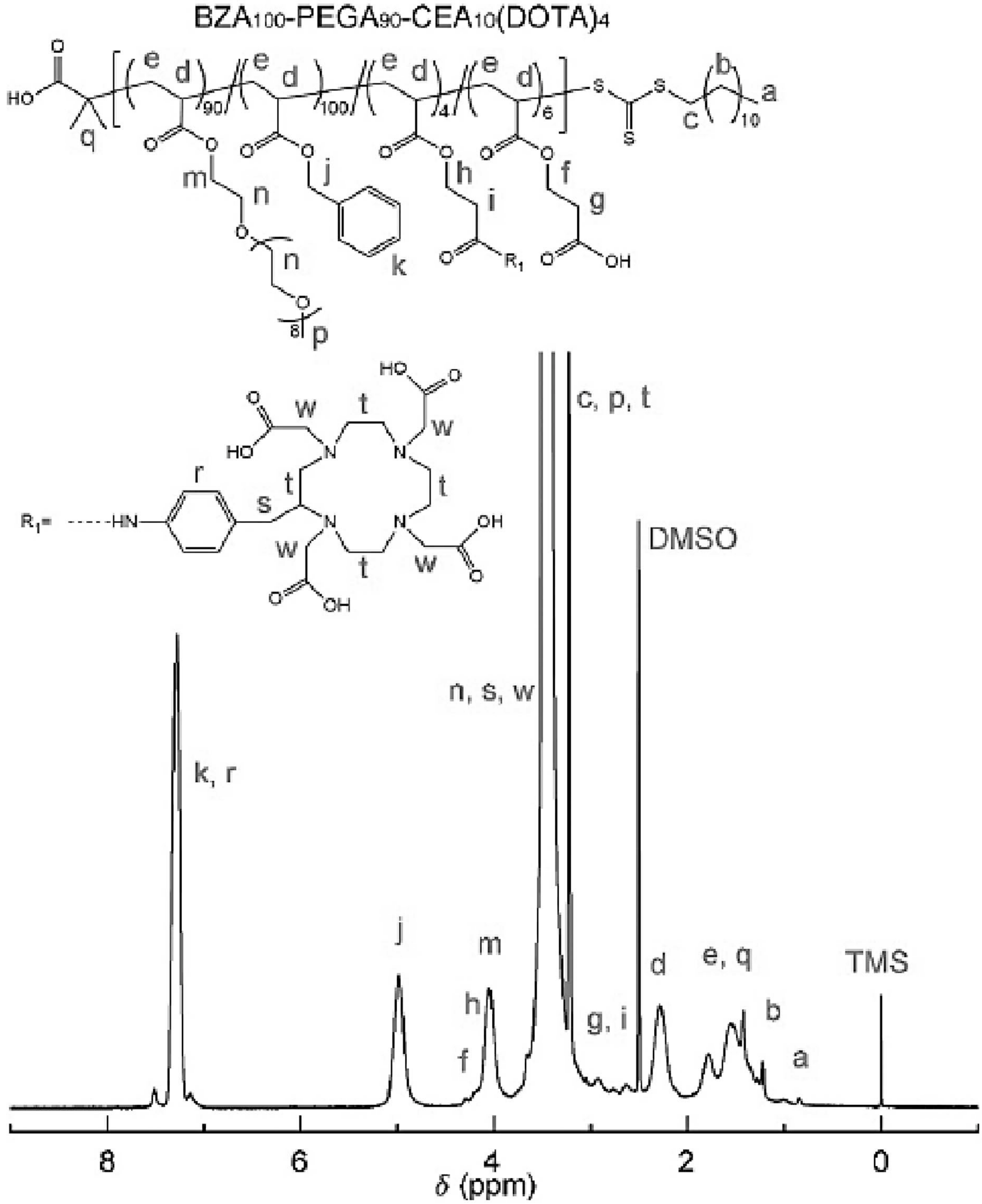

Fig. 11

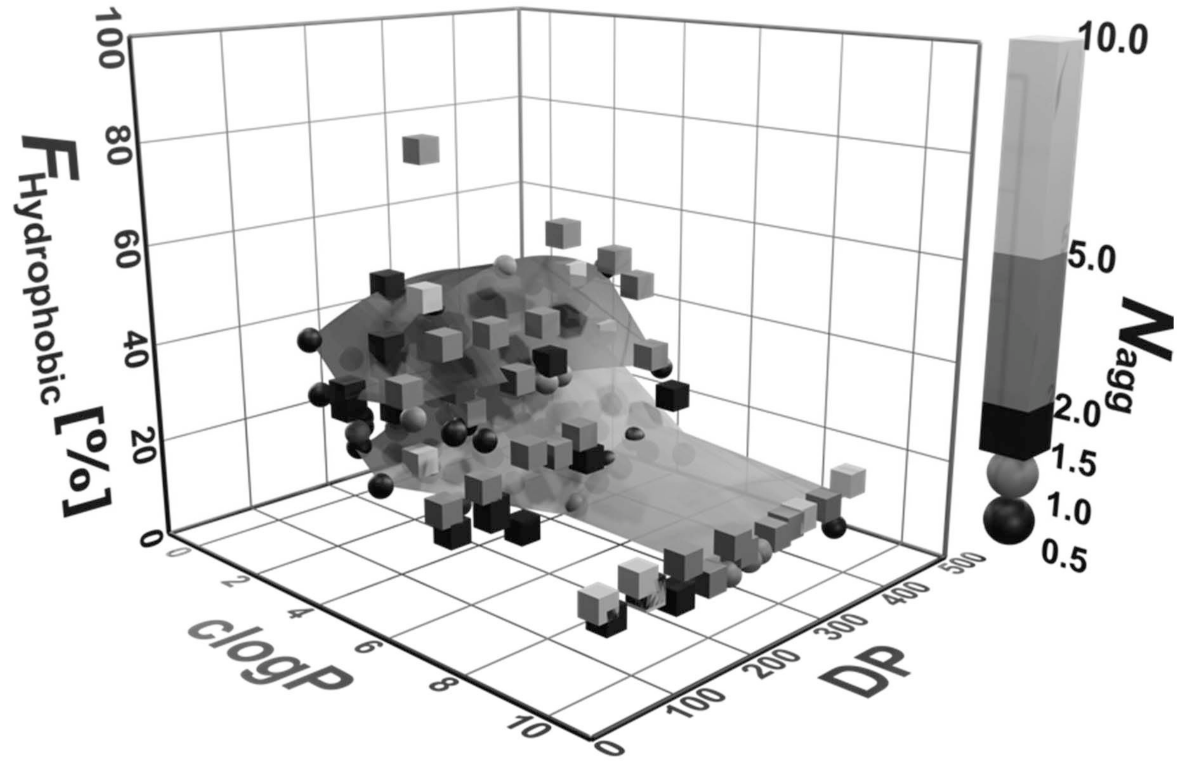
F Hydrophobic [%]
100
80
60
40
20
0
clogP
0
2
4
6
8
10
DP
0
100
200
300
400
500
N agg
10.0
5.0
2.0
1.5
1.0
0.5

Fig. 12

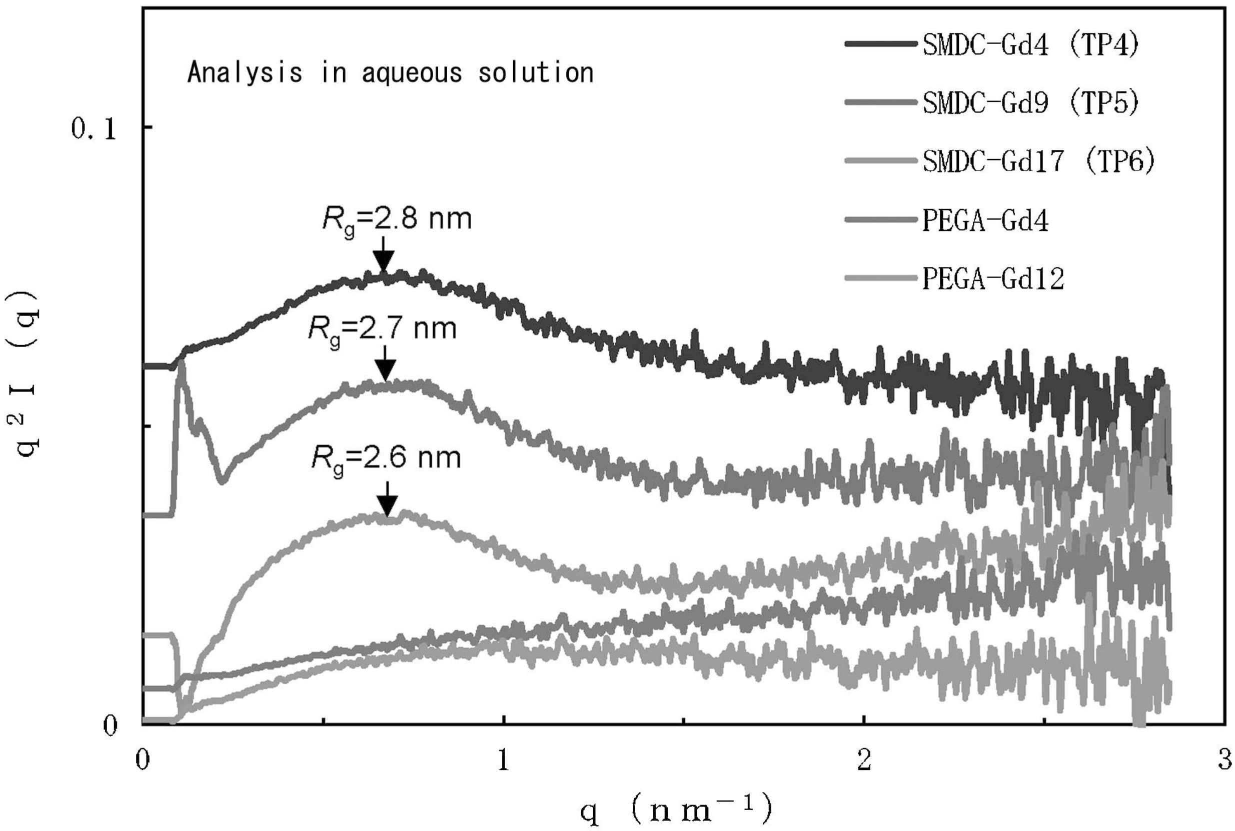
Analysis in aqueous solution
SMDC-Gd4 (TP4)
SMDC-Gd9 (TP5)
SMDC-Gd17 (TP6)
PEGA-Gd4
PEGA-Gd12
Rg=2.8 nm
Rg=2.7 nm
Rg=2.6 nm
0.1
0
q²I (q)
0
1
2
3
q (nm⁻¹)

Fig. 13

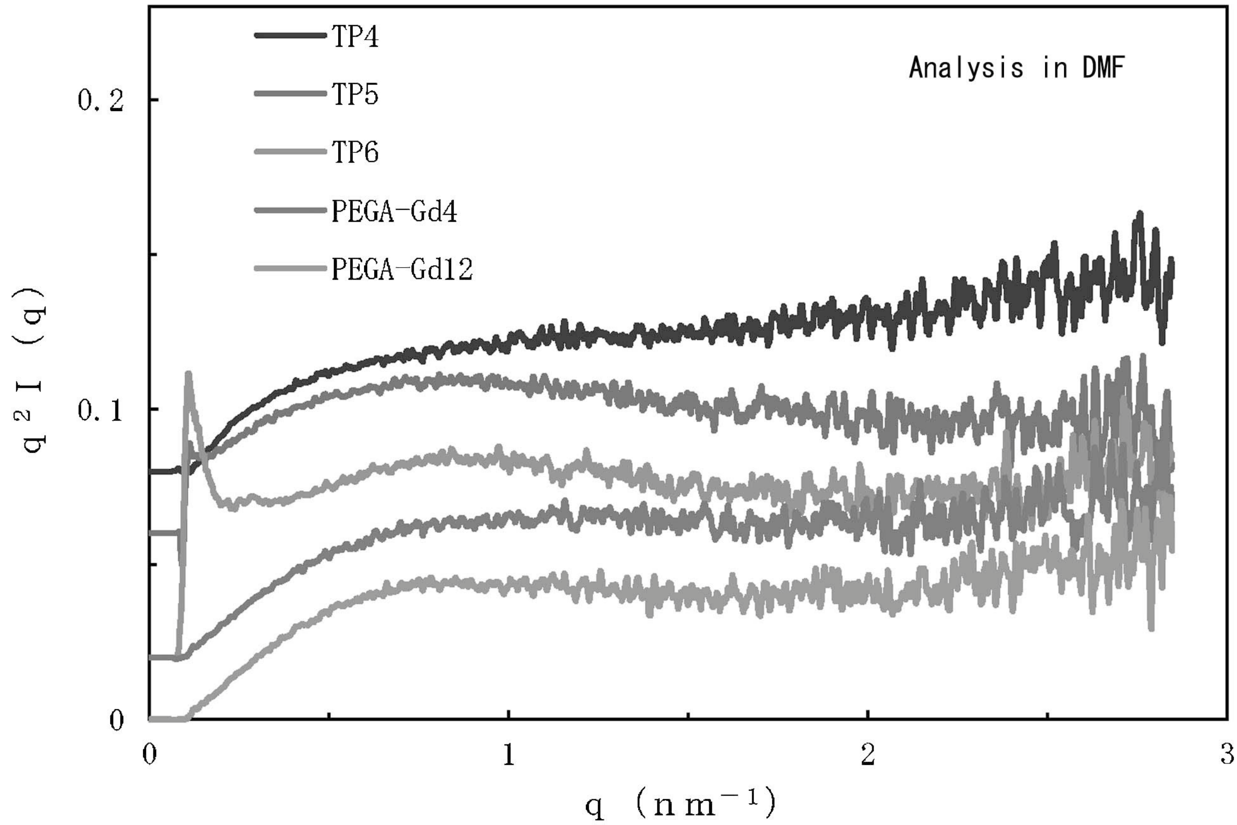


Fig. 14

SMDC-$Gd_4$

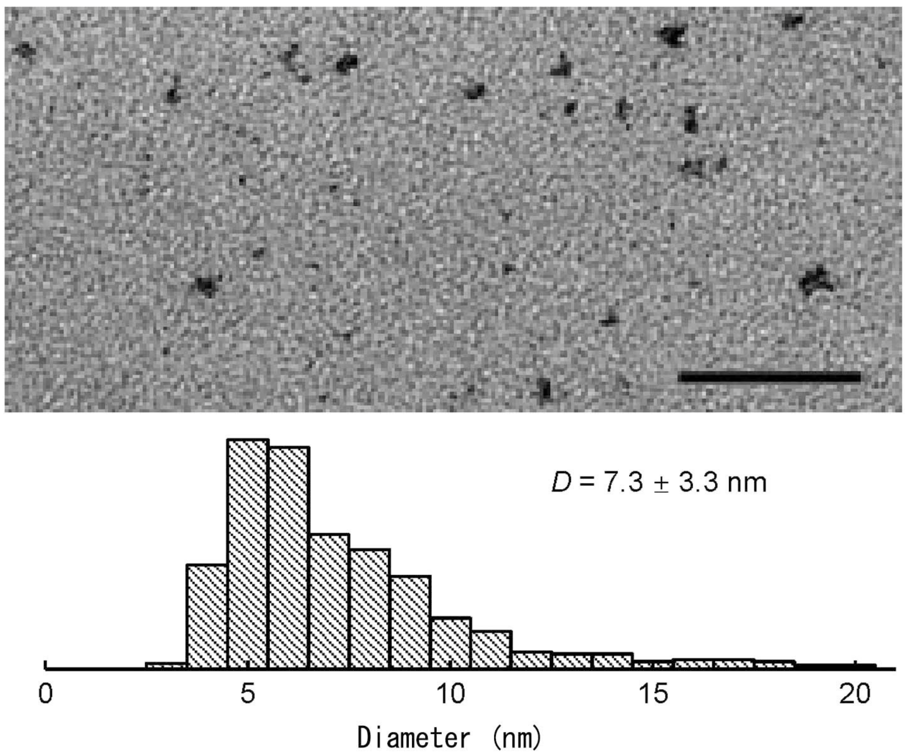

Fig. 15

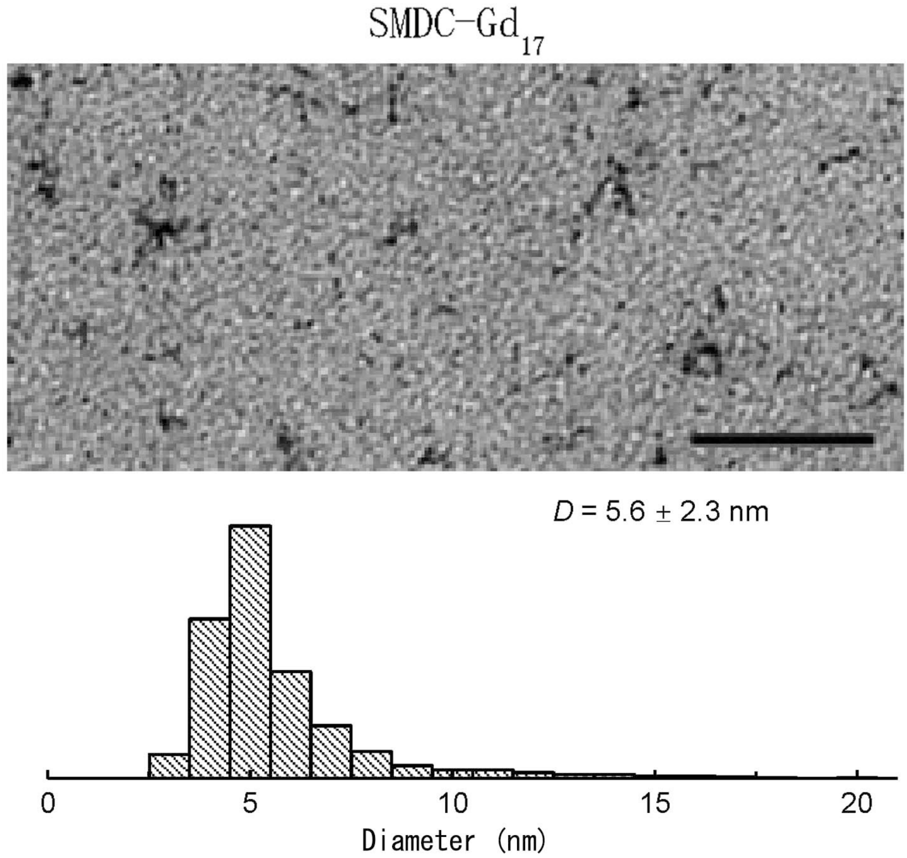
SMDC-Gd17
D = 5.6 ± 2.3 nm
0
5
10
15
20
Diameter (nm)

Fig. 16

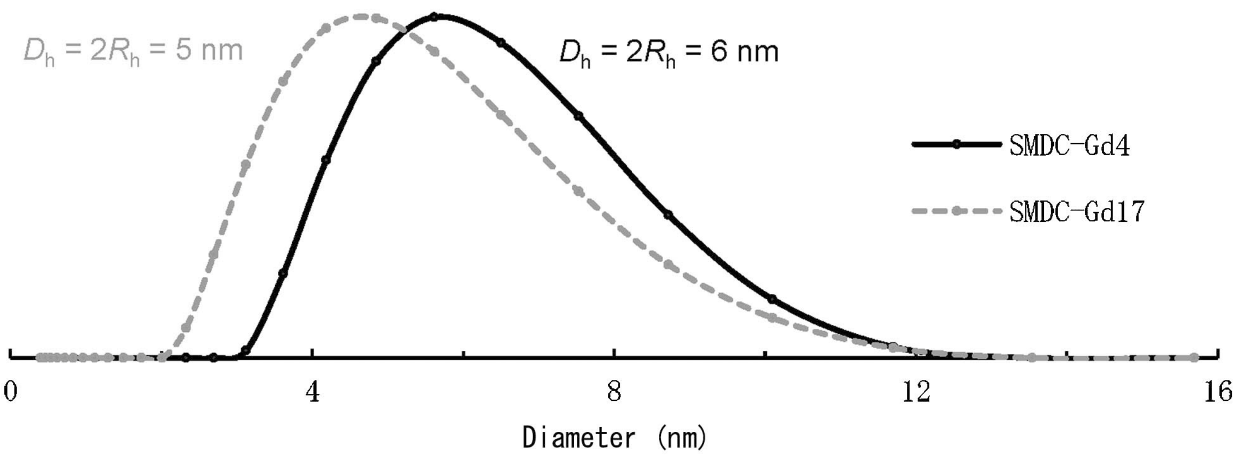
Dh = 2Rh = 5 nm
Dh = 2Rh = 6 nm
SMDC-Gd4
SMDC-Gd17
0
4
8
12
16
Diameter (nm)

Fig. 17

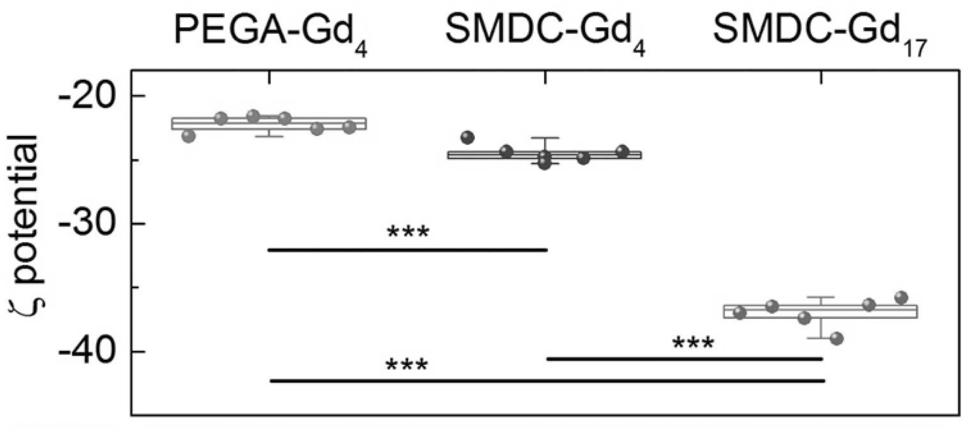


n = 6, *** p < 0. 0 0 1

Fig. 18

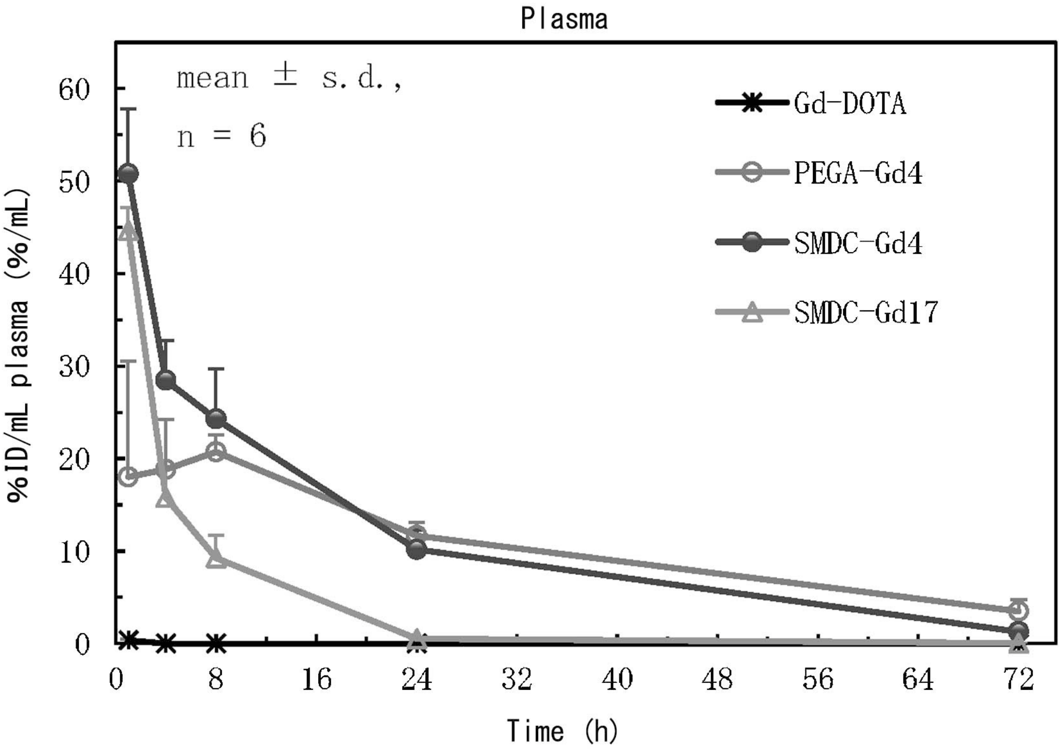

Fig. 19

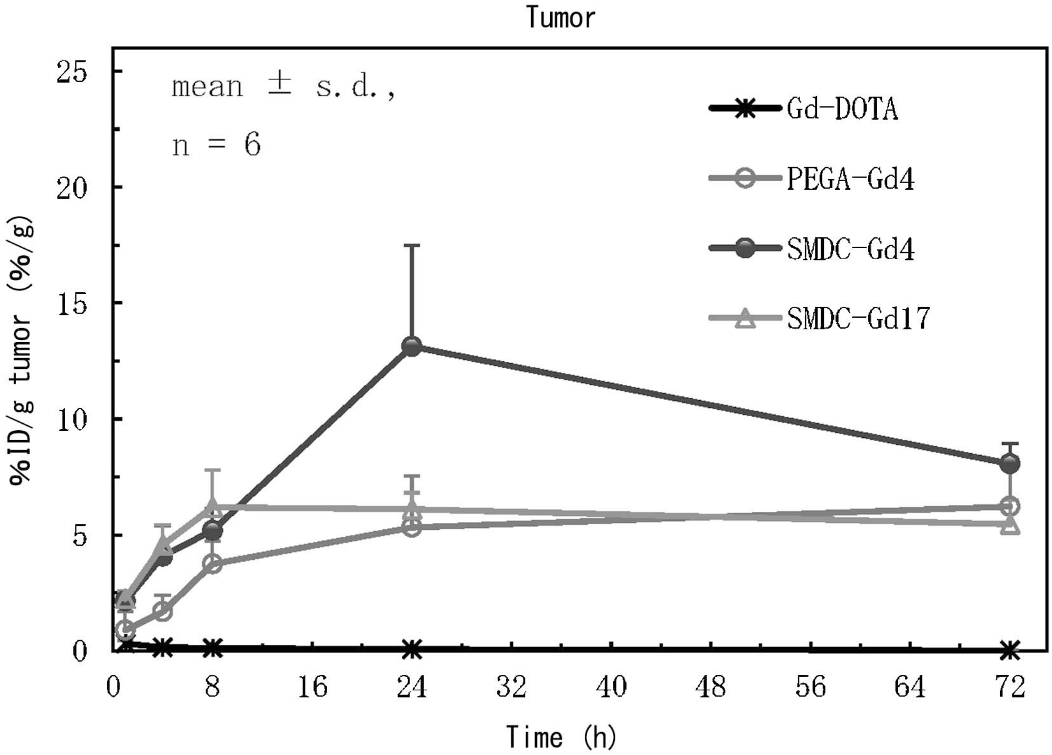
Tumor
mean ± s.d.,
n = 6
Gd-DOTA
PEGA-Gd4
SMDC-Gd4
SMDC-Gd17
%ID/g tumor (%/g)
0
5
10
15
20
25
0
8
16
24
32
40
48
56
64
72
Time (h)

Fig. 20

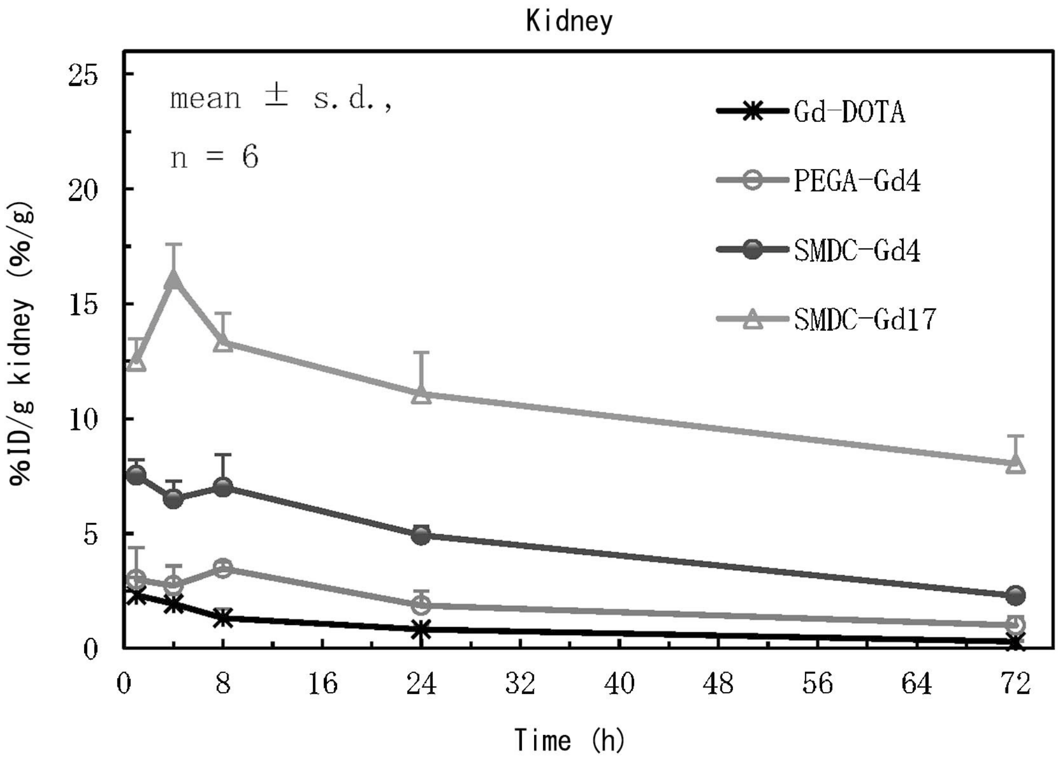
Kidney
mean ± s.d.,
n = 6
Gd-DOTA
PEGA-Gd4
SMDC-Gd4
SMDC-Gd17
%ID/g kidney (%/g)
0
5
10
15
20
25
0
8
16
24
32
40
48
56
64
72
Time (h)

Fig. 21

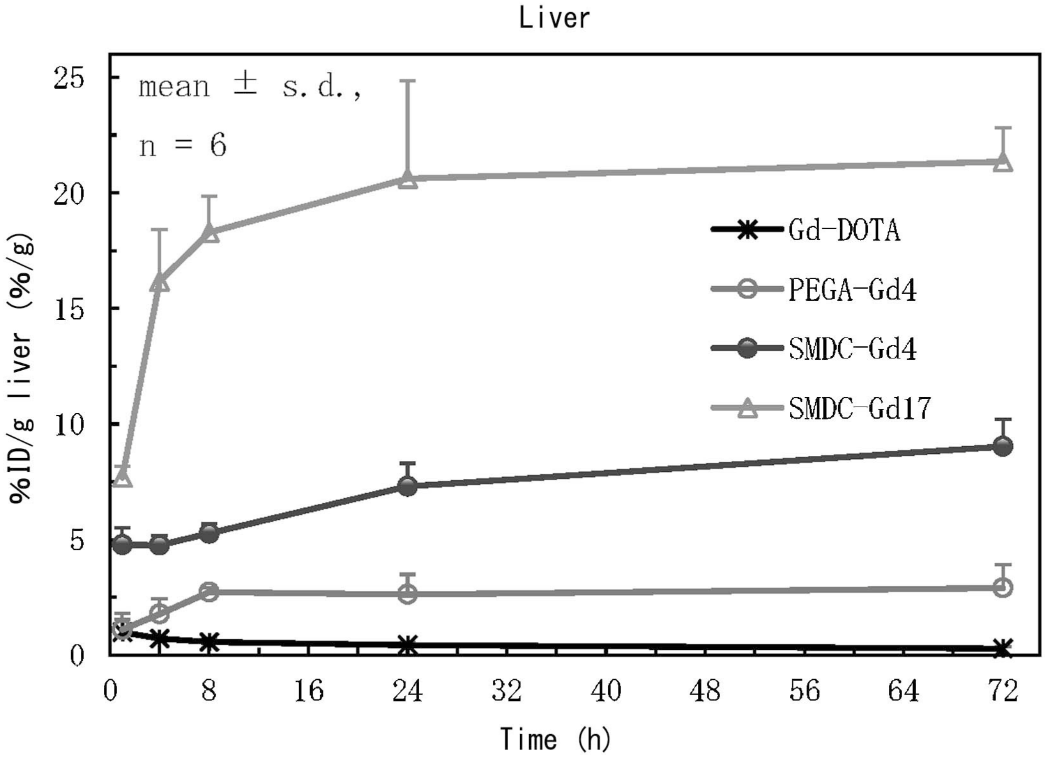
Liver
mean ± s.d.,
n = 6
Gd-DOTA
PEGA-Gd4
SMDC-Gd4
SMDC-Gd17
%ID/g liver (%/g)
0
5
10
15
20
25
0
8
16
24
32
40
48
56
64
72
Time (h)

Fig. 22

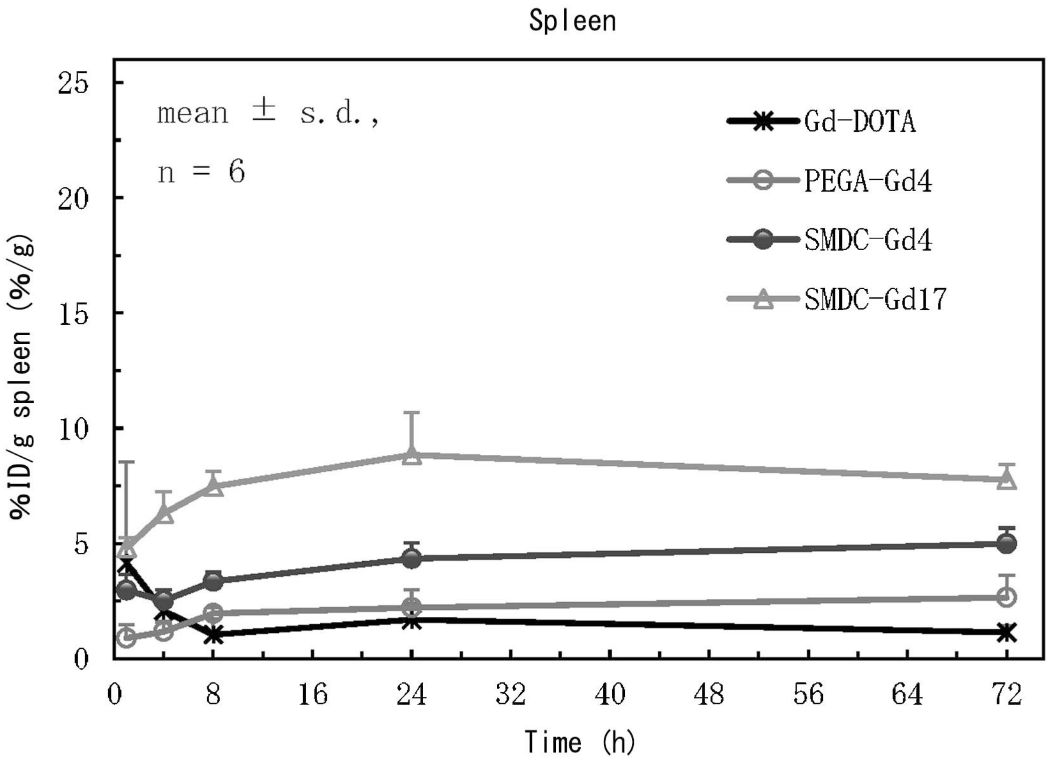
Spleen
mean ± s.d.,
n = 6
Gd-DOTA
PEGA-Gd4
SMDC-Gd4
SMDC-Gd17
%ID/g spleen (%/g)
0
5
10
15
20
25
0
8
16
24
32
40
48
56
64
72
Time (h)

Fig. 23

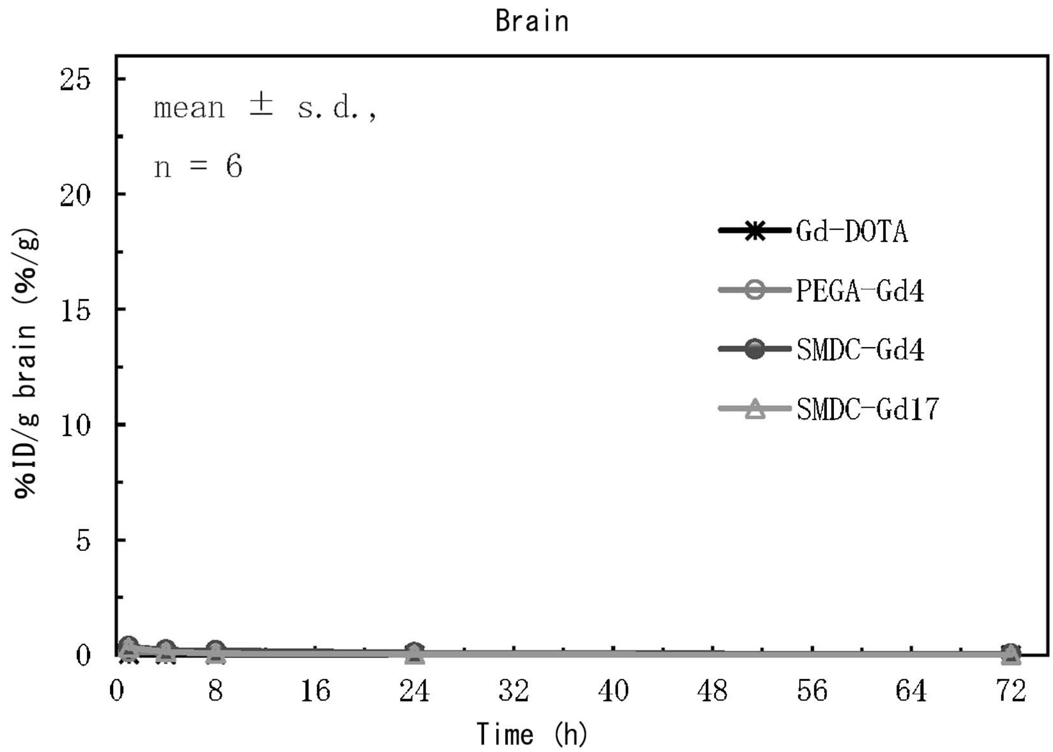
Brain
mean ± s.d.,
n = 6
Gd-DOTA
PEGA-Gd4
SMDC-Gd4
SMDC-Gd17
%ID/g brain (%/g)
25
20
15
10
5
0
0
8
16
24
32
40
48
56
64
72
Time (h)

Fig. 24

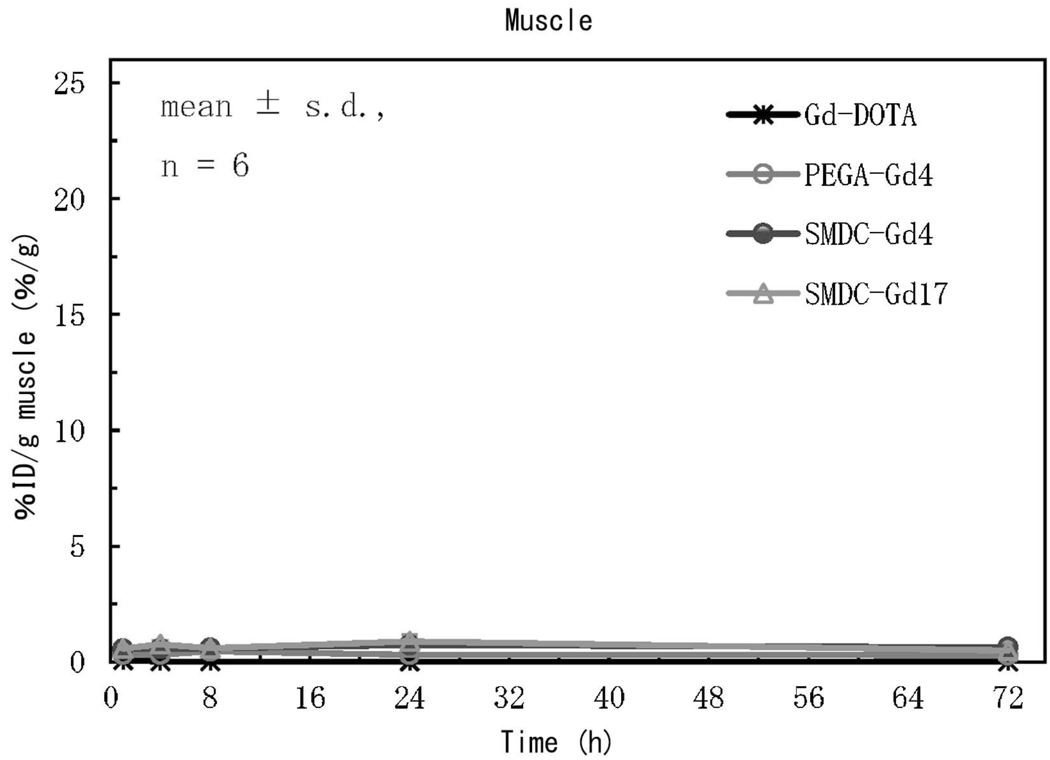
Muscle
mean ± s.d.,
n = 6
Gd-DOTA
PEGA-Gd4
SMDC-Gd4
SMDC-Gd17
%ID/g muscle (%/g)
25
20
15
10
5
0
0
8
16
24
32
40
48
56
64
72
Time (h)

Fig. 25

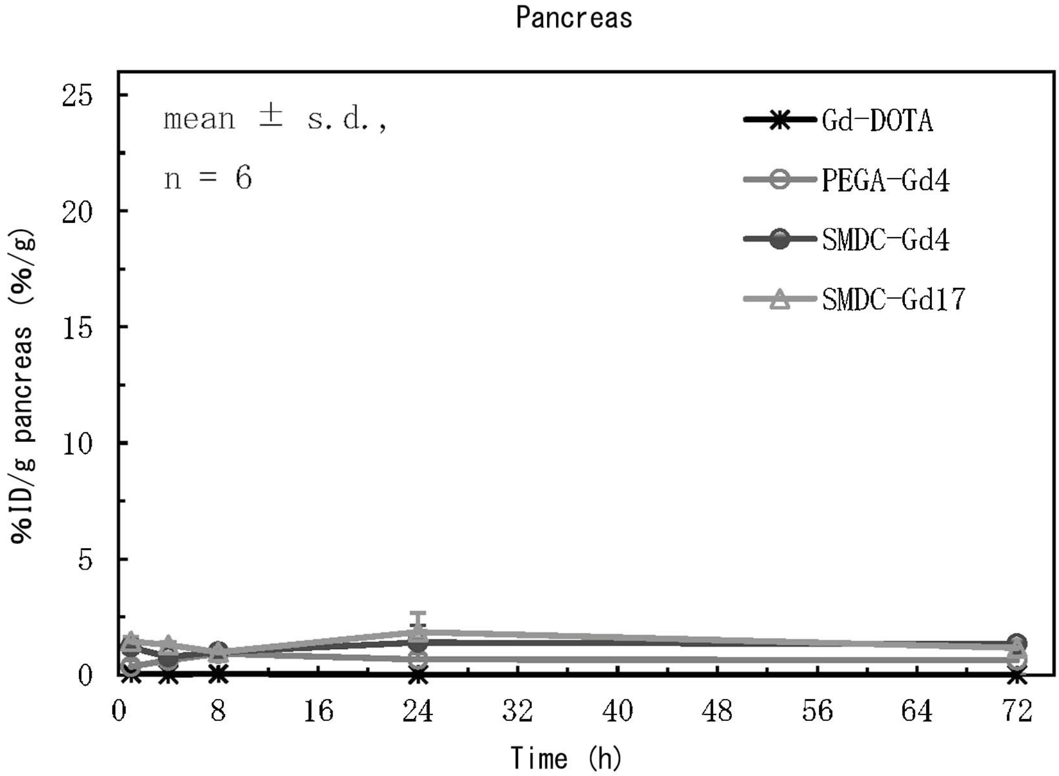
Pancreas
mean ± s.d.,
n = 6
Gd-DOTA
PEGA-Gd4
SMDC-Gd4
SMDC-Gd17
%ID/g pancreas (%/g)
25
20
15
10
5
0
0
8
16
24
32
40
48
56
64
72
Time (h)

Fig. 26

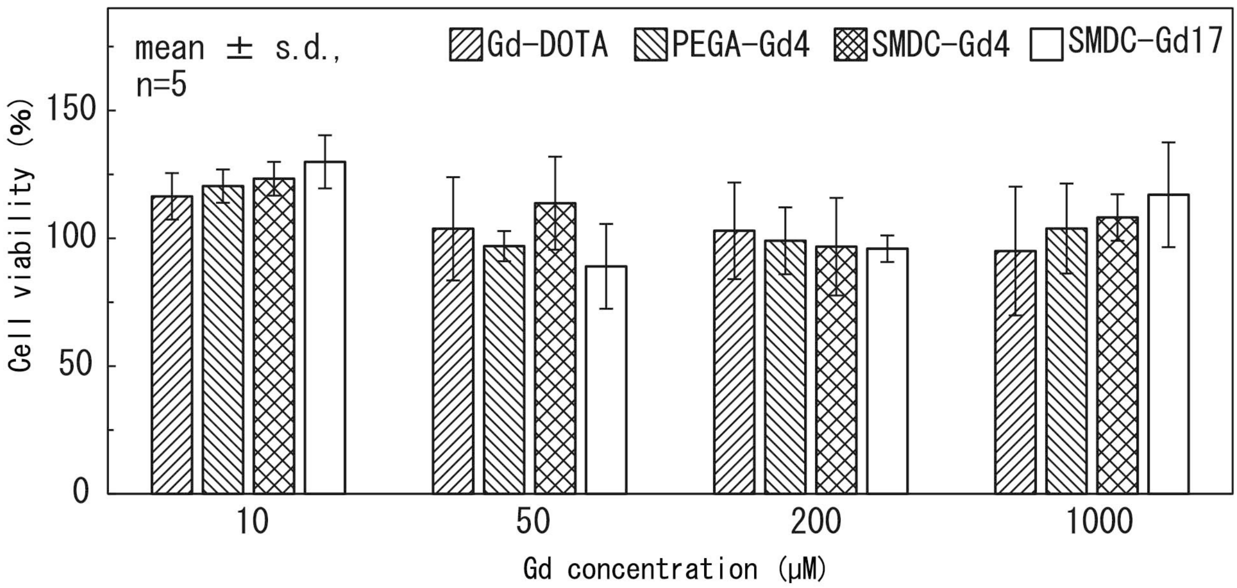
mean ± s.d.,
n=5
Gd-DOTA
PEGA-Gd4
SMDC-Gd4
SMDC-Gd17
Cell viability (%)
150
100
50
0
10
50
200
1000
Gd concentration (μM)

Fig. 27

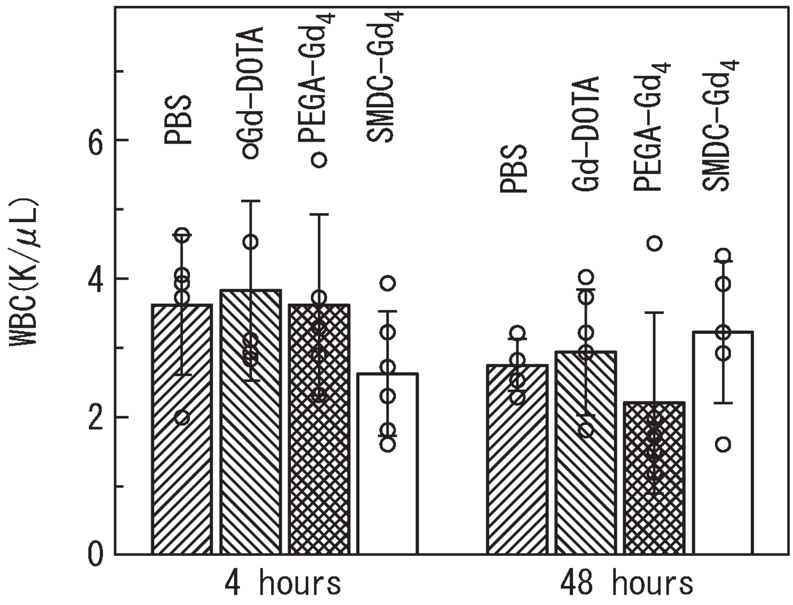
WBC(K/μL)
0
2
4
6
PBS
Gd-DOTA
PEGA-Gd4
SMDC-Gd4
PBS
Gd-DOTA
PEGA-Gd4
SMDC-Gd4
4 hours
48 hours

Fig. 28

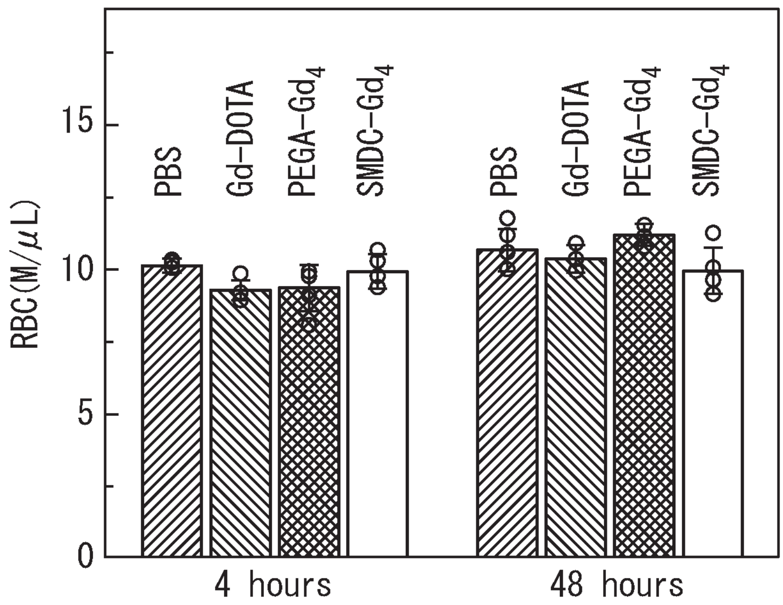
RBC(M/μL)
0
5
10
15
PBS
Gd-DOTA
PEGA-Gd4
SMDC-Gd4
PBS
Gd-DOTA
PEGA-Gd4
SMDC-Gd4
4 hours
48 hours

Fig. 29

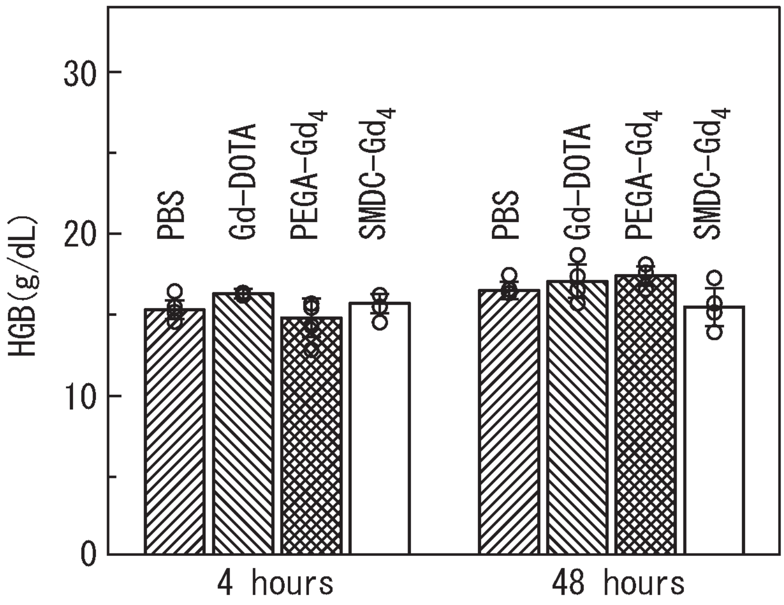
30
20
10
0
HGB(g/dL)
PBS
Gd-DOTA
PEGA-Gd4
SMDC-Gd4
PBS
Gd-DOTA
PEGA-Gd4
SMDC-Gd4
4 hours
48 hours

Fig. 30

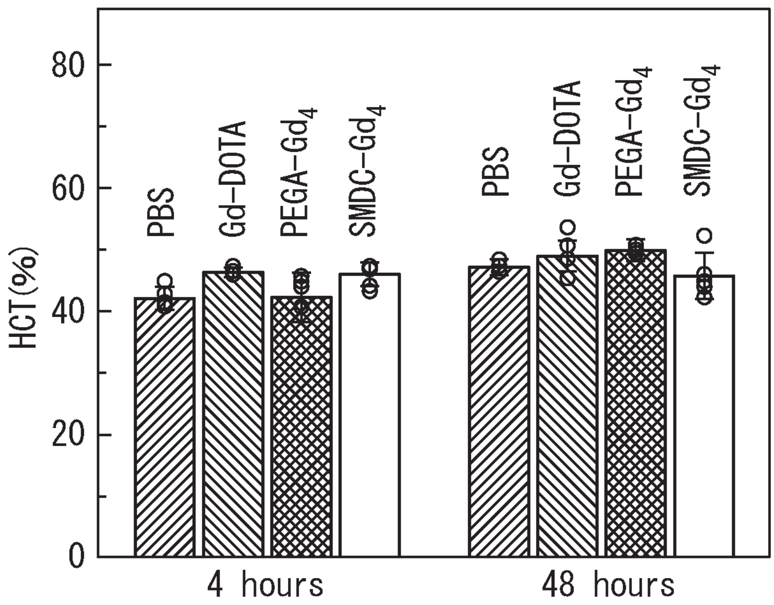
80
60
40
20
0
HCT(%)
PBS
Gd-DOTA
PEGA-Gd4
SMDC-Gd4
PBS
Gd-DOTA
PEGA-Gd4
SMDC-Gd4
4 hours
48 hours

Fig. 31

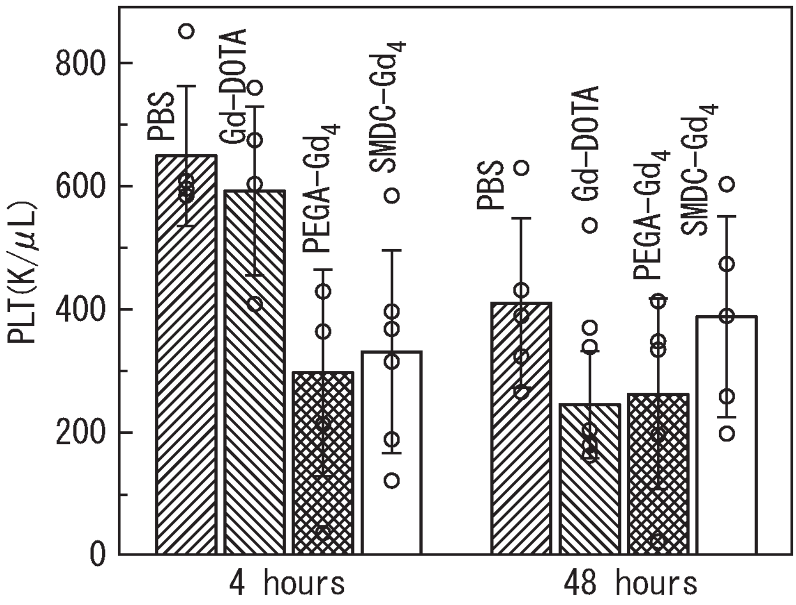
PLT(K/μL)
0
200
400
600
800
PBS
Gd-DOTA
PEGA-Gd4
SMDC-Gd4
4 hours
48 hours

Fig. 32

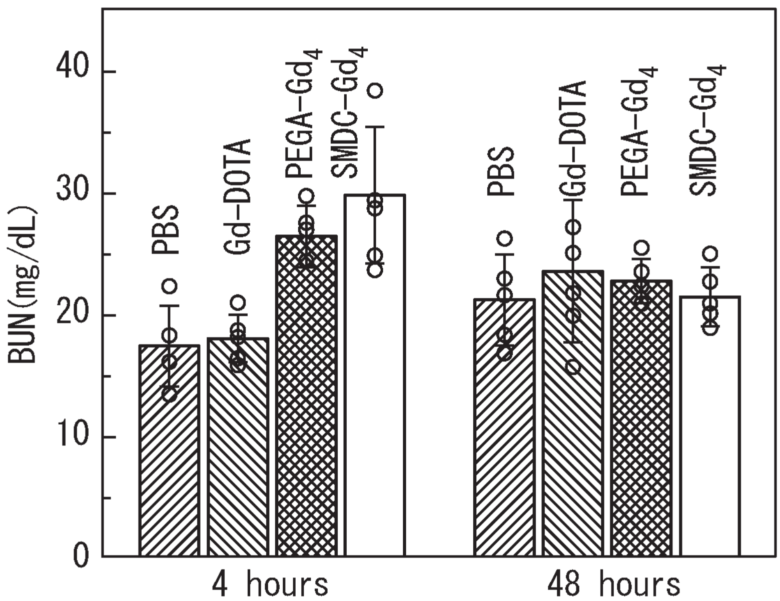
BUN(mg/dL)
0
10
20
30
40
PBS
Gd-DOTA
PEGA-Gd4
SMDC-Gd4
4 hours
48 hours

Fig. 33

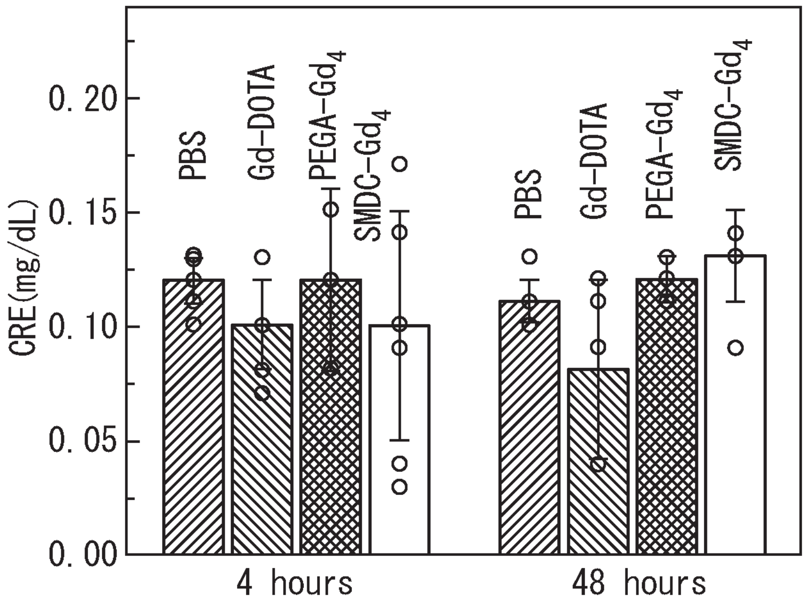
CRE(mg/dL)
0.00
0.05
0.10
0.15
0.20
PBS
Gd-DOTA
PEGA-Gd4
SMDC-Gd4
PBS
Gd-DOTA
PEGA-Gd4
SMDC-Gd4
4 hours
48 hours

Fig. 34

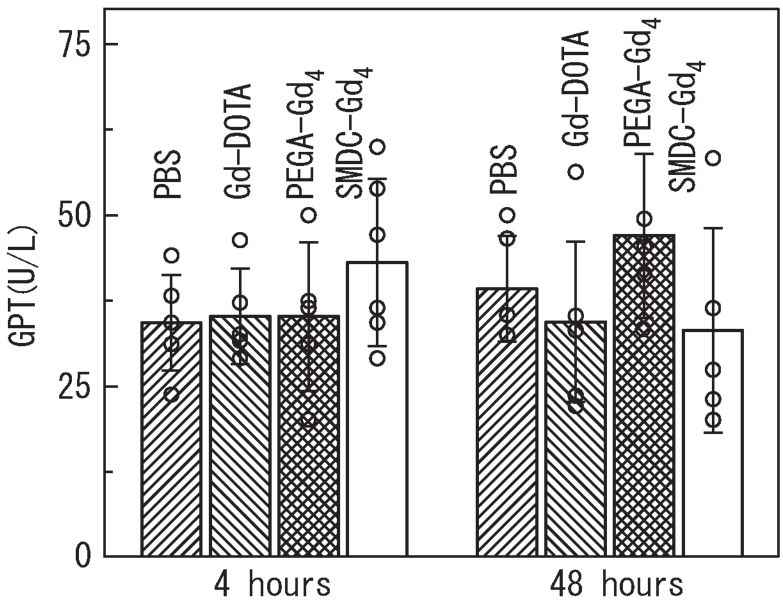
GPT(U/L)
0
25
50
75
PBS
Gd-DOTA
PEGA-Gd4
SMDC-Gd4
PBS
Gd-DOTA
PEGA-Gd4
SMDC-Gd4
4 hours
48 hours

Fig. 35

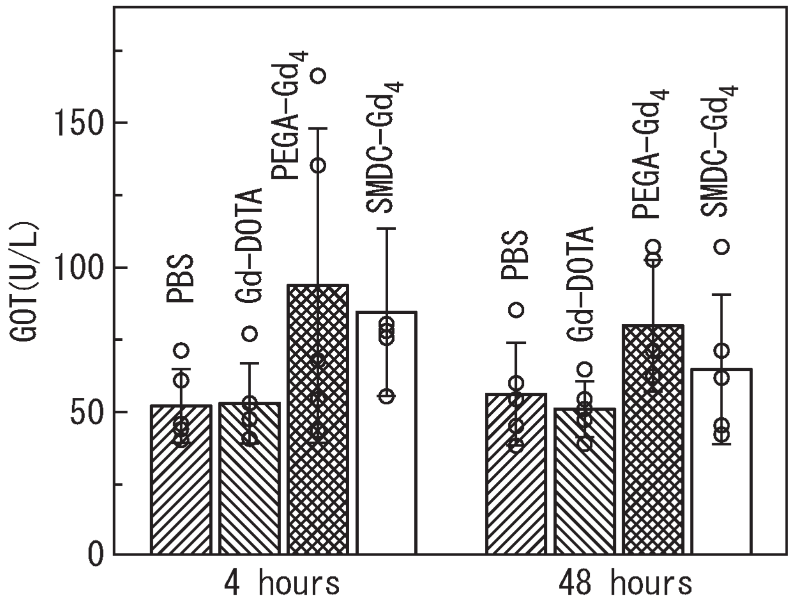
GOT(U/L)
0
50
100
150
PBS
Gd-DOTA
PEGA-Gd4
SMDC-Gd4
PBS
Gd-DOTA
PEGA-Gd4
SMDC-Gd4
4 hours
48 hours

Fig. 36

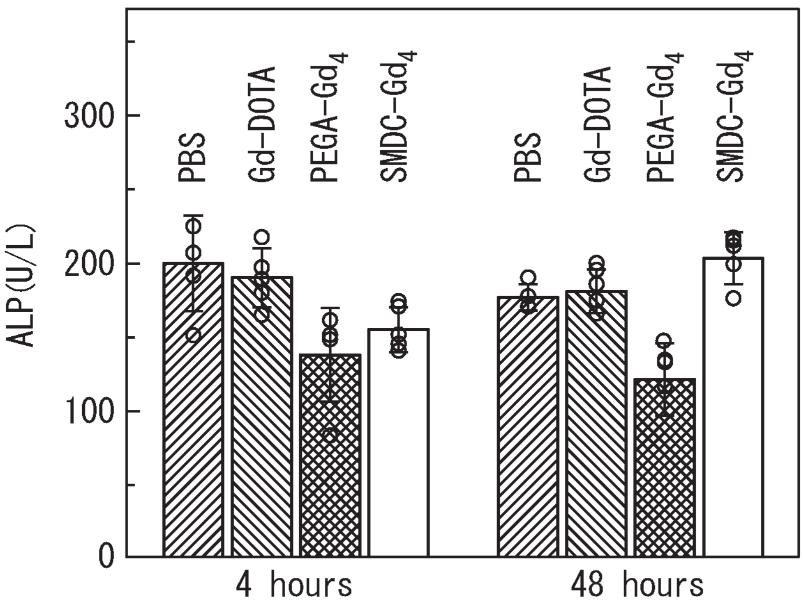
ALP(U/L)
0
100
200
300
PBS
Gd-DOTA
PEGA-Gd4
SMDC-Gd4
PBS
Gd-DOTA
PEGA-Gd4
SMDC-Gd4
4 hours
48 hours

Fig. 37

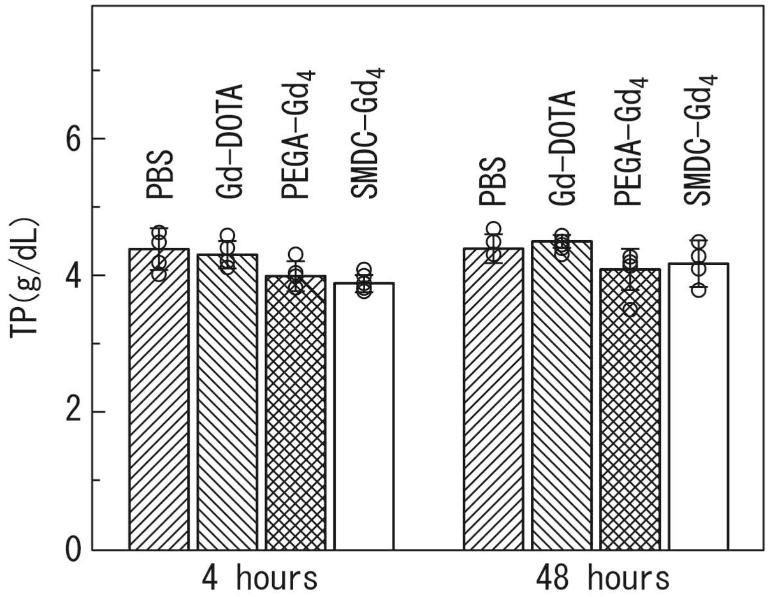
TP(g/dL)
0
2
4
6
PBS
Gd-DOTA
PEGA-Gd4
SMDC-Gd4
4 hours
48 hours

Fig. 38

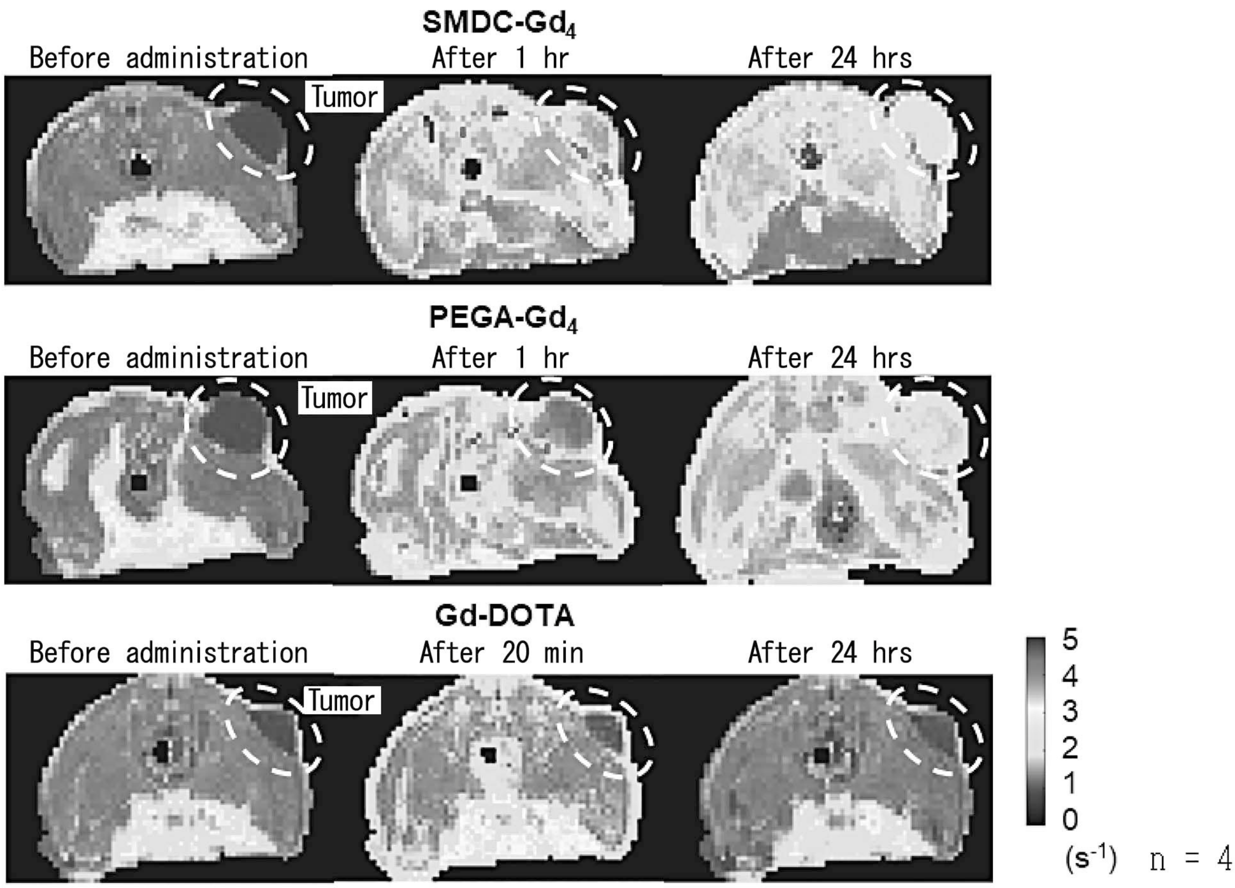
SMDC-Gd4
Before administration
After 1 hr
After 24 hrs
Tumor
PEGA-Gd4
Before administration
After 1 hr
After 24 hrs
Tumor
Gd-DOTA
Before administration
After 20 min
After 24 hrs
Tumor
5
4
3
2
1
0
(s-1)
n = 4

Fig. 39

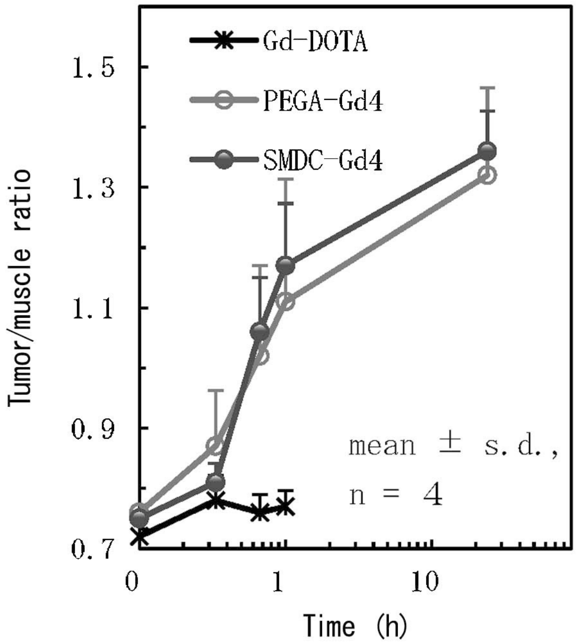
Gd-DOTA
PEGA-Gd4
SMDC-Gd4
1.5
1.3
1.1
0.9
0.7
Tumor/muscle ratio
mean ± s.d.,
n = 4
0
1
10
Time (h)

Fig. 40

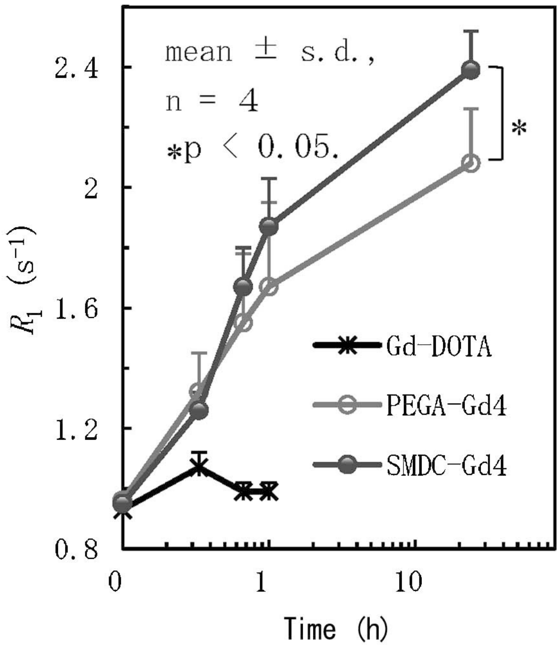
mean ± s.d.,
n = 4
*p < 0.05.
*
2.4
2
1.6
1.2
0.8
R1 (s-1)
Gd-DOTA
PEGA-Gd4
SMDC-Gd4
0
1
10
Time (h)

Fig. 41

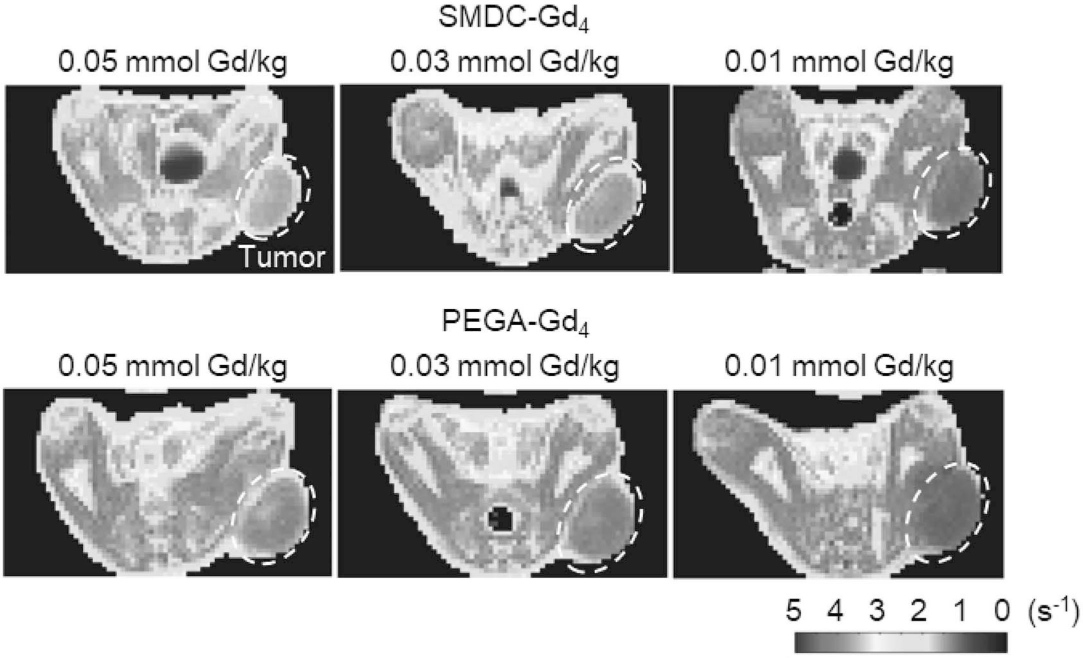
SMDC-Gd4
0.05 mmol Gd/kg
0.03 mmol Gd/kg
0.01 mmol Gd/kg
Tumor
PEGA-Gd4
0.05 mmol Gd/kg
0.03 mmol Gd/kg
0.01 mmol Gd/kg
5 4 3 2 1 0 (s-1)

Fig. 42

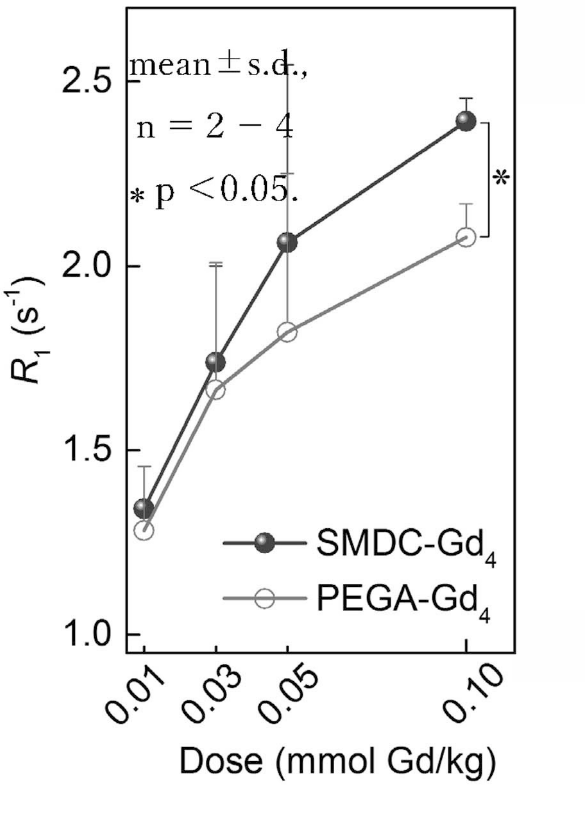
mean ± s.d.,
n = 2 − 4
* p < 0.05.
*
R1 (s-1)
2.5
2.0
1.5
1.0
SMDC-Gd4
PEGA-Gd4
0.01
0.03
0.05
0.10
Dose (mmol Gd/kg)

Fig. 43

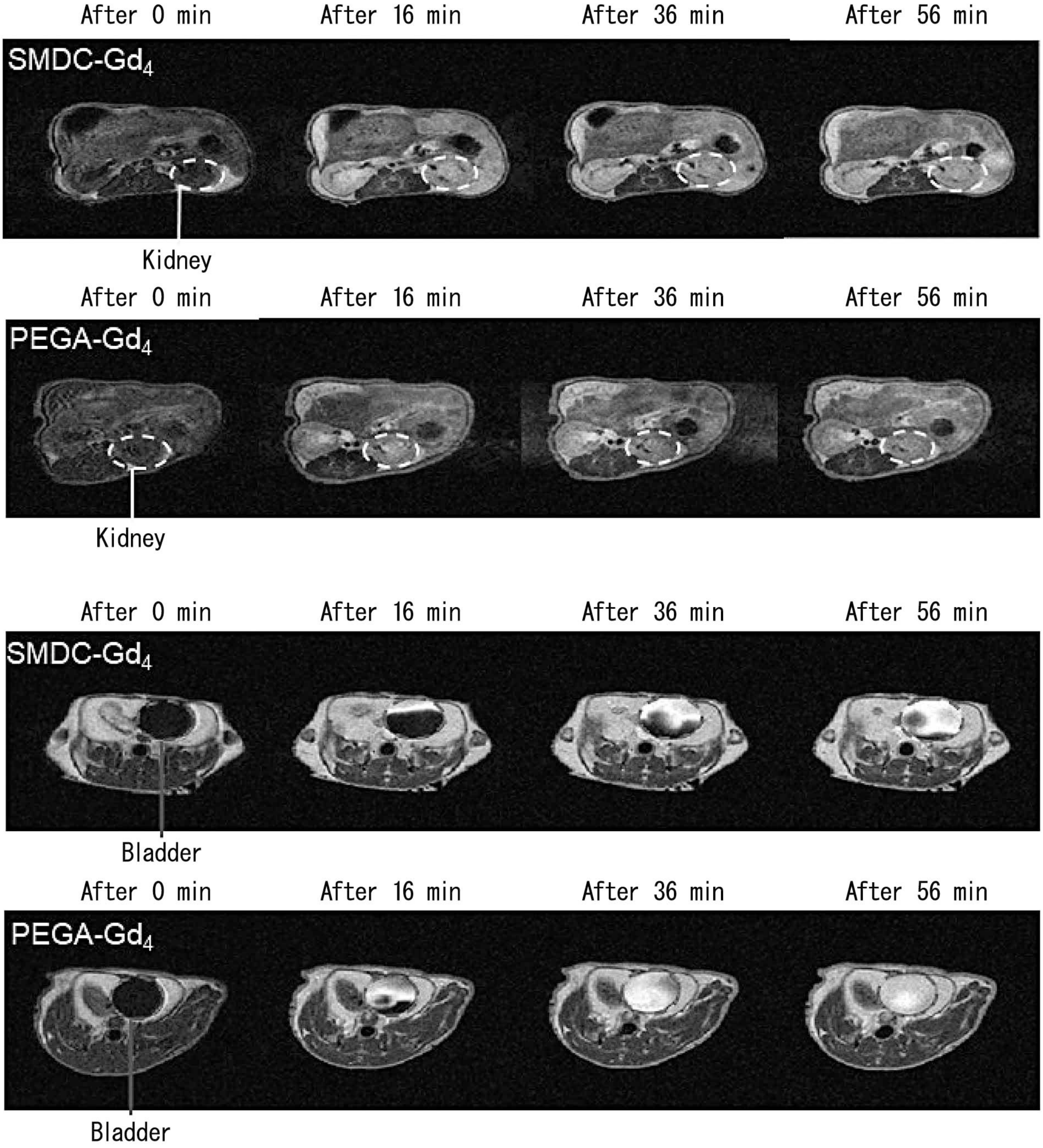
After 0 min
After 16 min
After 36 min
After 56 min
SMDC-Gd4
Kidney
After 0 min
After 16 min
After 36 min
After 56 min
PEGA-Gd4
Kidney
After 0 min
After 16 min
After 36 min
After 56 min
SMDC-Gd4
Bladder
After 0 min
After 16 min
After 36 min
After 56 min
PEGA-Gd4
Bladder

Fig. 44

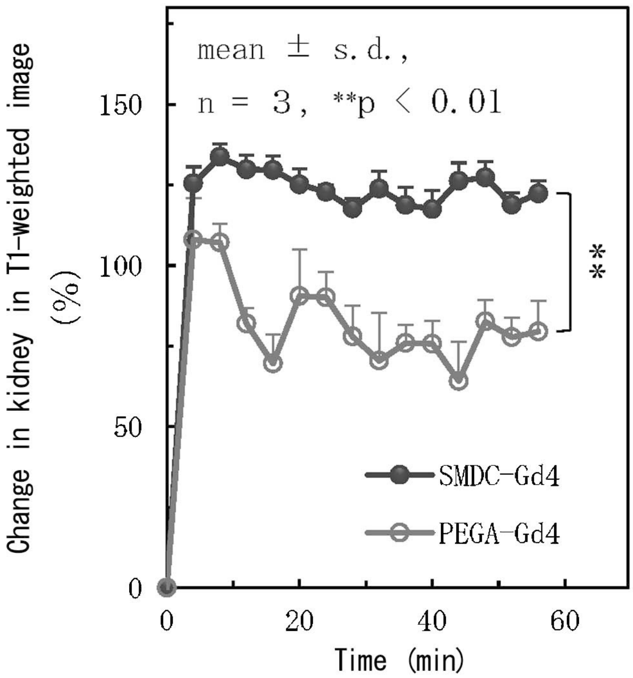
mean ± s.d.,
n = 3, **p < 0.01
Change in kidney in T1-weighted image (%)
150
100
50
0
0
20
40
60
Time (min)
SMDC-Gd4
PEGA-Gd4
**

Fig. 45

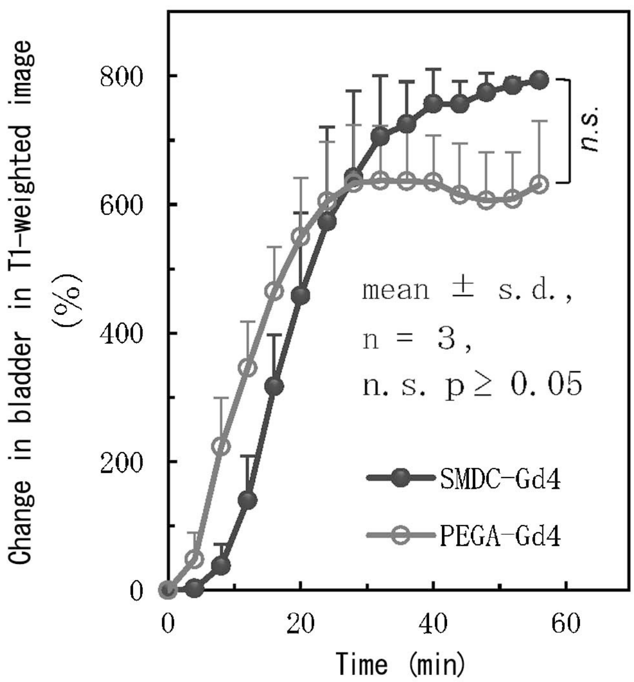
Change in bladder in T1-weighted image (%)
800
600
400
200
0
0
20
40
60
Time (min)
n.s.
mean ± s.d.,
n = 3,
n.s. p≥ 0.05
SMDC-Gd4
PEGA-Gd4

Fig. 46

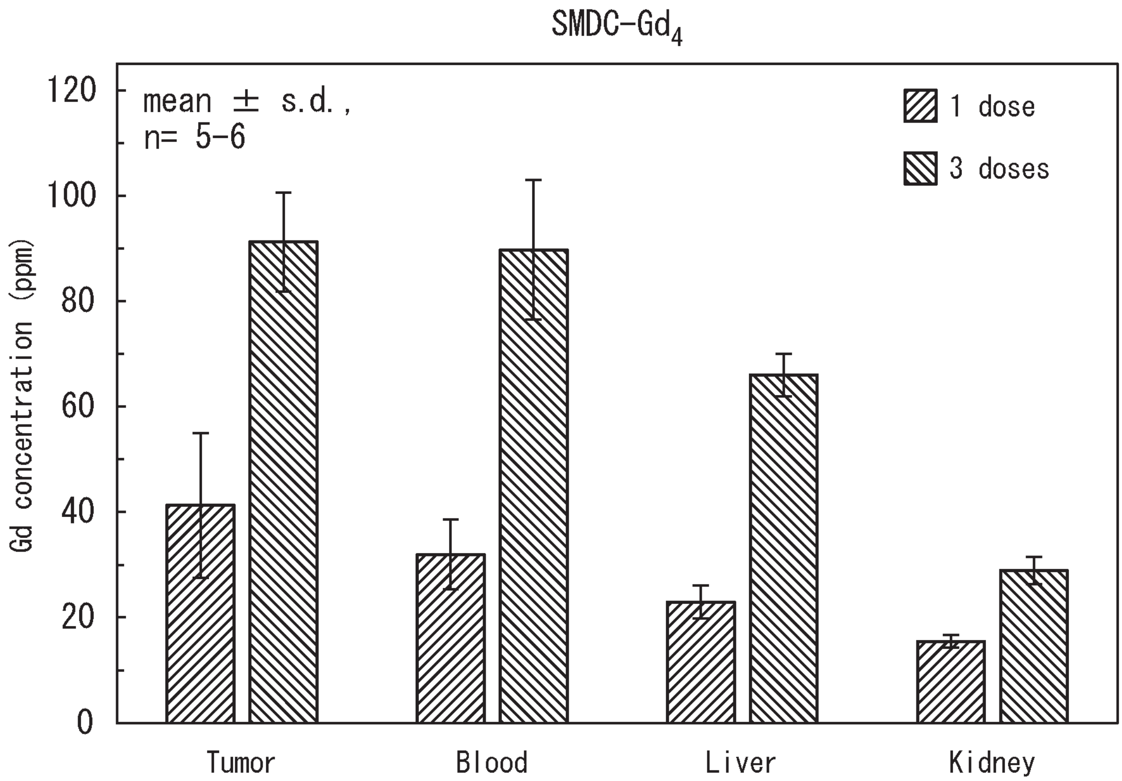
SMDC-Gd4
mean ± s.d.,
n= 5-6
1 dose
3 doses
Gd concentration (ppm)
0
20
40
60
80
100
120
Tumor
Blood
Liver
Kidney

Fig. 47

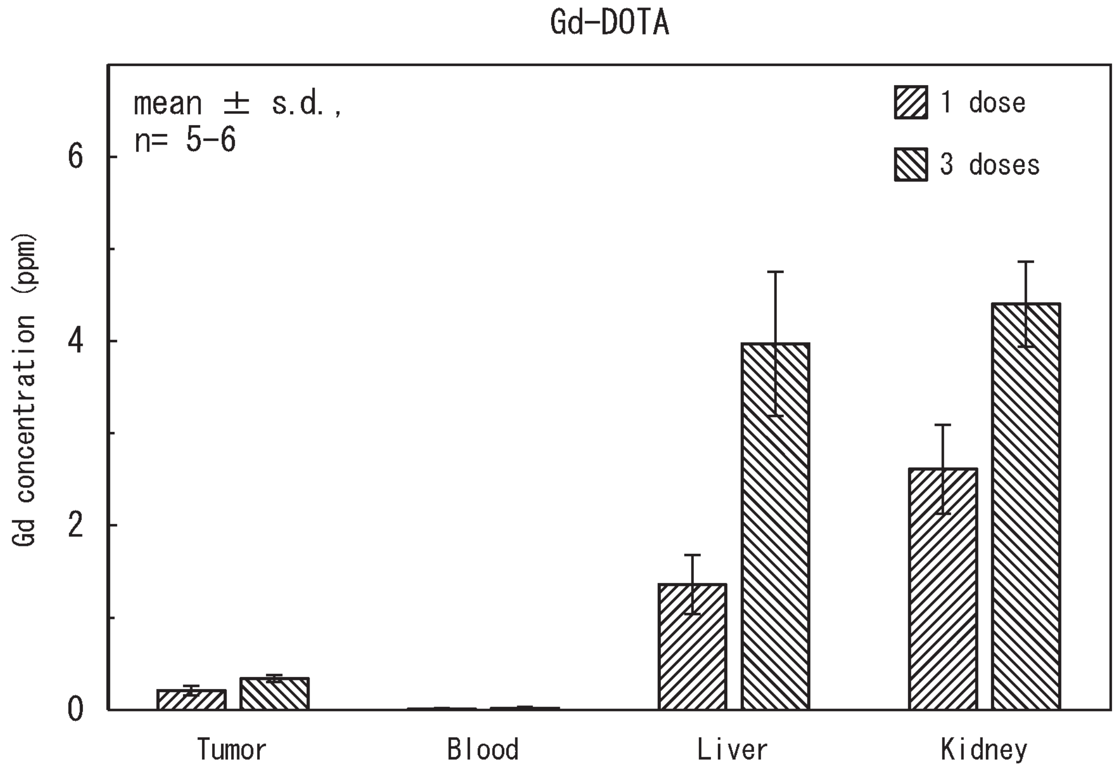
Gd-DOTA
mean ± s.d.,
n= 5-6
1 dose
3 doses
Gd concentration (ppm)
0
2
4
6
Tumor
Blood
Liver
Kidney

Fig. 48

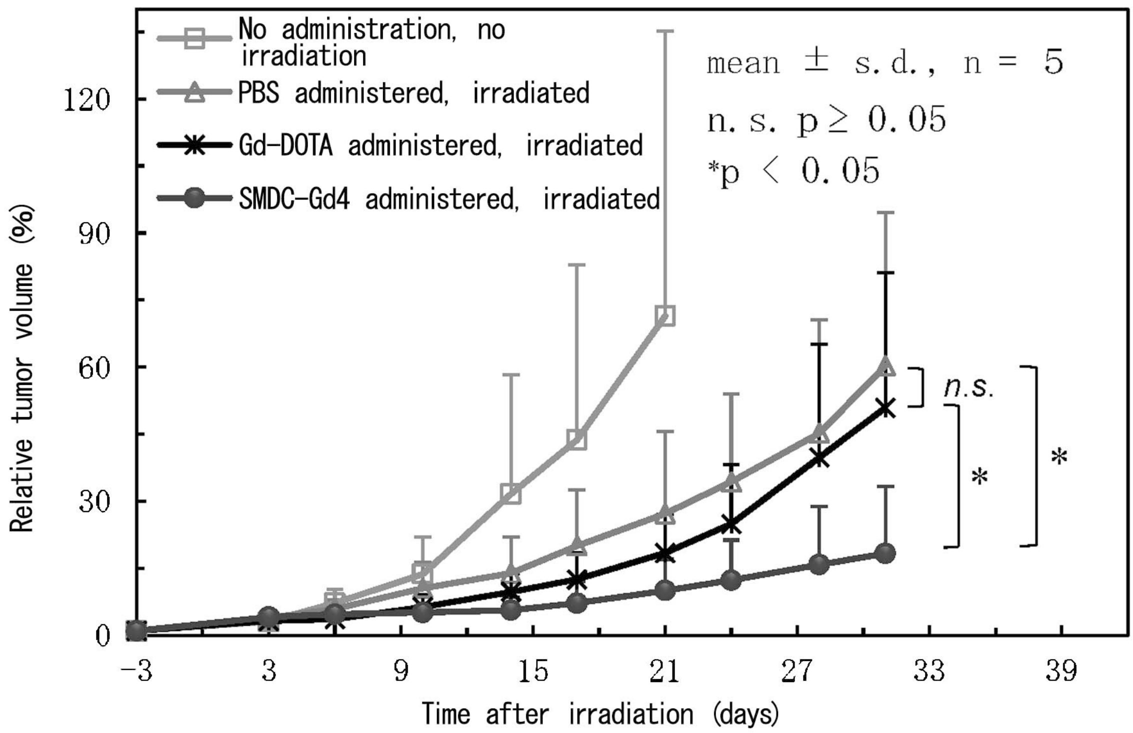
No administration, no irradiation
PBS administered, irradiated
Gd-DOTA administered, irradiated
SMDC-Gd4 administered, irradiated
mean ± s.d., n = 5
n.s. p ≥ 0.05
*p < 0.05
n.s.
*
*
Relative tumor volume (%)
0
30
60
90
120
-3
3
9
15
21
27
33
39
Time after irradiation (days)

Fig. 49

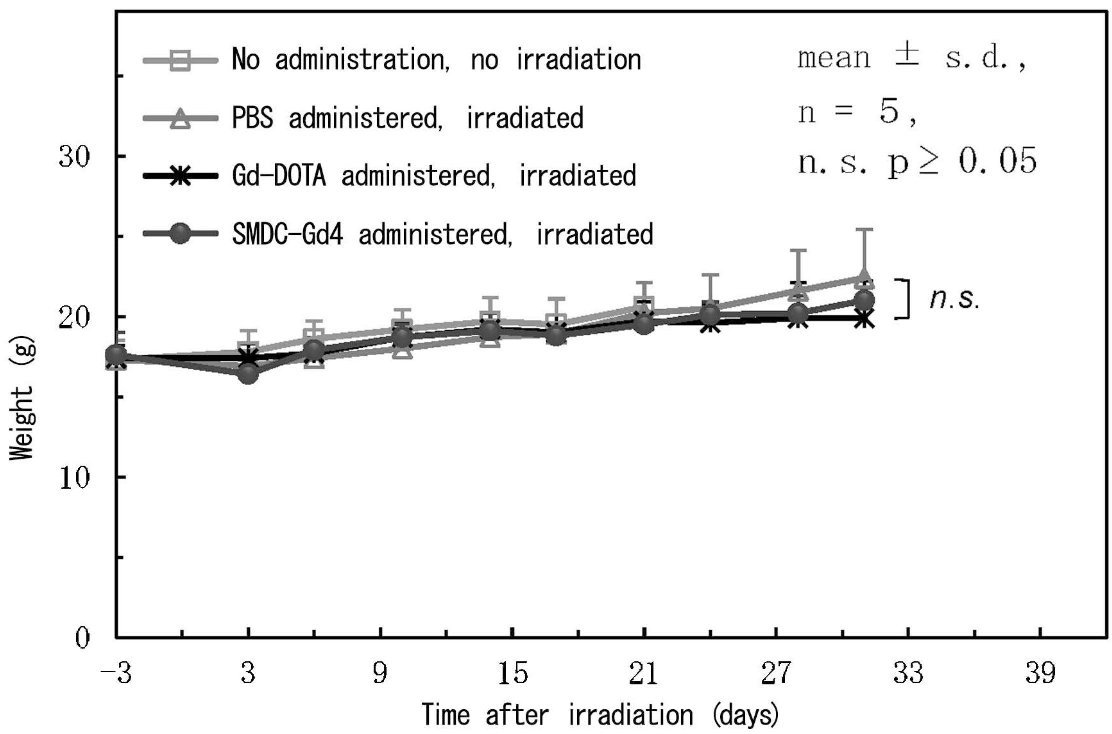
No administration, no irradiation
PBS administered, irradiated
Gd-DOTA administered, irradiated
SMDC-Gd4 administered, irradiated
mean ± s.d.,
n = 5,
n.s. p ≥ 0.05
n.s.
Weight (g)
0
10
20
30
-3
3
9
15
21
27
33
39
Time after irradiation (days)

## INTERNATIONAL SEARCH REPORT

International application No. **PCT/JP2024/027098**

### A. CLASSIFICATION OF SUBJECT MATTER

***A61K 9/10***(2006.01)i; ***A61K 47/34***(2017.01)i; ***A61K 51/12***(2006.01)i; ***A61P 35/00***(2006.01)i; *A61K 103/34*(2006.01)n
FI: A61K9/10; A61K47/34; A61K51/12 100; A61P35/00; A61K51/12 200; A61K103:34

According to International Patent Classification (IPC) or to both national classification and IPC

### B. FIELDS SEARCHED

Minimum documentation searched (classification system followed by classification symbols)

A61K9/10; A61K47/34; A61K51/12; A61P35/00; A61K103/34

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | WO 2009/157561 A1 (JAPAN SCIENCE AND TECHNOLOGY AGENCY) 30 December 2009 (2009-12-30) entire text | 1-18 |
| A | WO 98/41241 A1 (AKAIKE, Toshihiro) 24 September 1998 (1998-09-24) entire text | 1-18 |
| A | SHIRAISHI, K. et al., Preparation and in vivo imaging of PEG-poly(L-lysine)-based polymeric micelle MRI contrast agents, Journal of Controlled Release, 2009, vol. 136, no. 1, pages 14-20 entire text | 1-18 |
| A | LI, H. et al., An advanced micelle-based biodegradable HPMA polymer-gadolinium contrast agent for MR imaging of murine vasculatures and tumors, Polymer Chemistry, 2020, vol. 11, no. 39, pages 6374-6386 entire text | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 October 2024** | **15 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/027098

C. DOCUMENTS CONSIDERED TO BE RELEVANT

| Category* | Citation of document, with indication, where appropriate, of the relevant passages | Relevant to claim No. |
|---|---|---|
| P, X | GAO, S. et al., Self-Folding Macromolecular Drug Carrier for Cancer Imaging and Therapy, Advanced Science, November 2023, vol. 11, Article no. 2304171, pages 1-12<br>abstract, sections 2.1-2.4 | 1-18 |

Form PCT/ISA/210 (second sheet) (July 2022)

**INTERNATIONAL SEARCH REPORT**
**Information on patent family members**

International application No.
**PCT/JP2024/027098**

| Patent document cited in search report | Publication date (day/month/year) | Patent family member(s) | Publication date (day/month/year) |
| --- | --- | --- | --- |
| WO 2009/157561 A1 | 30 December 2009 | US 2011/0123458 A1 | |
| | | EP 2308916 A1 | |
| WO 98/41241 A1 | 24 September 1998 | US 6372194 B1 | |
| | | EP 1034796 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **STABILE, A et al.** *Nat. Rev. Urol.*, 2020, vol. 17, 41-61
- **SCLAFANI, F. et al.** *Br. J. Cancer*, 2017, vol. 117, 1478-1485
- **WAHSNER, J. et al.** *Chem. Rev.*, 2018, vol. 119, 957-1057
- **JEON, M. et al.** *Adv. Mater.*, 2021, vol. 33, 1906539
- **TANG, J. et al.** *Prog. Polym. Sci.*, 2013, vol. 38, 462-502
- **WANG, A. Z. et al.** *Annu. Rev. Med.*, 2012, vol. 63, 185-198
- **CHENG, C. J.** ; **SALTZMAN, W. M.** *Nat. Nanotechnol.*, 2012, vol. 7, 346-347
- **KANASTY, R. et al.** *Nat. Mater.*, 2013, vol. 12, 967-977
- **NISHIYAMA, N. et al.** *Cancer Sci.*, 2016, vol. 107, 867-874
- **CHO, K. et al.** *Clin. Cancer Res.*, 2008, vol. 14, 1310-1316
- **VERSILST, P. et al.** *Chem. Soc. Rev.*, 2015, vol. 44, 1791-1806
- **CABRAL, H. et al.** *Nat. Nanotechnol.*, 2011, vol. 6, 815-823
- **LU, Y. et al.** *Nat. Biomed. Eng.*, 2018, vol. 2, 318-325
- **ICHIKAWA et al.** Frontiers: Creation of gadolinium formulation for neutron capture therapy for malignant tumors. *Pharmacia*, 2020, vol. 56 (5), 396-400
- **STABILE, A. et al.** Multiparametric MRI for prostate cancer diagnosis: current status and future directions. *Nat. Rev. Urol.*, 2020, vol. 17, 41-61
- **SCLAFANI, F et al.** Comparison between MRI and pathology in the assessment of tumour regression grade in rectal cancer.. *Br. J. Cancer*, 2017, vol. 117, 1478-1485
- **WAHSNER, J. et al.** Chemistry of MRI contrast agents: current challenges and new frontiers. *Chem. Rev.*, 2018, vol. 119, 957-1057
- **JEON, M. et al.** Iron oxide nanoparticles as T1 contrast agents for magnetic resonance imaging: fundamentals, challenges, applications, and prospectives.. *Adv. Mater.*, 2021, vol. 33, 1906539
- **TANG, J. et al.** Macromolecular MRI contrast agents: structures, properties and applications. *Prog. Polym. Sci.*, 2013, vol. 38, 462-502
- **WANG, A. Z.** ; **LANGER, R.** ; **FAROKHZAD, O. C.** Nanoparticle delivery of cancer drugs. *Annu. Rev. Med.*, 2012, vol. 63, 185-198
- **CHENG, C. J.** ; **SALTZMAN, W. M**. Downsizing tumour therapeutics. *Nat. Nanotechnol.*, 2012, vol. 7, 346-347
- **KANASTY, R. et al.** Delivery materials for siRNA therapeutics. *Nat. Mater*, 2013, vol. 12, 967-977
- **NISHIYAMA, N** ; **MATSUMURA, Y.** ; **KATAOKA, K**. Development of polymeric micelles for targeting intractable cancers. *Cancer Sci.*, 2016, vol. 107, 867-874
- **CHO, K. et al.** Therapeutic nanoparticles for drug delivery in cancer. *Clin. Cancer Res.*, 2008, vol. 14, 1310-1316
- **VERWILST, P. et al.** Recent advances in Gd-chelate based bimodal optical/MRI contrast agents. *Chem. Soc. Rev.*, 2015, vol. 44, 1791-1806
- **CABRAL, H. et al.** Accumulation of sub-100 nm polymeric micelles in poorly permeable tumours depends on size. *Nat. Nanotechnol.*, 2011, vol. 6, 815-823
- **LU, Y. et al.** Micelles with ultralow critical micelle concentration as carriers for drug delivery. *Nat. Biomed. Eng.*, 2018, vol. 2, 318-325
- **CAI, Y. et al.** The in vivo fate of polymeric micelles. *Adv. Drug Delivery Rev.*, 2022, vol. 188, 114463
- **YU, M** ; **ZHENG, J.** Clearance pathways and tumor targeting of imaging nanoparticles. *ACS nano*, 2015, vol. 9, 6655-6674
- Single-Chain Polymer Nanoparticles: Synthesis, Characterization, Simulations, and Applications Ch 8. **TERASHIMA, T.** ; **SAWAMOTO, M**. Single-chain nanoparticles via self-folding amphiphilic copolymers in water. Wiley-VCH, 2017
- **IMAI, S. et al.** Programmed self-assembly systems of amphiphilic random copolymers into size-controlled and thermoresponsive micelles in water. *Macromolecules*, 2018, vol. 51, 398-409
- **HATTORI, G. et al.** Self-assembly of PEG/dodecyl-graft amphiphilic copolymers in water: consequences of the monomer sequence and chain flexibility on uniform micelles. *Polym. Chem*, 2017, vol. 8, 7248-7259
- **SWAMINATHAN, S.** ; **SHAH, S. V.** New insights into nephrogenic systemic fibrosis. *J. Am. Soc. Nephrol*, 2007, vol. 18, 2636-2643

- **ROBERT, P. et al.** T1-weighted hypersignal in the deep cerebellar nuclei after repeated administrations of gadolinium-based contrast agents in healthy rats: difference between linear and macrocyclic agents. *Invest. Radiol*, 2015, vol. 50, 473-480
- **ROBERT, P. et al.** Linear gadolinium-based contrast agents are associated with brain gadolinium retention in healthy rats. *Invest. Radiol.*, 2016, vol. 51, 73-82
- **HIRAI, Y. et al.** Precision self-assembly of amphiphilic random copolymers into uniform and self-sorting nanocompartments in water. *Macromolecules*, 2016, vol. 49, 5084-5091
- **DREHER, M. R. et al.** Tumor vascular permeability, accumulation, and penetration of macromolecular drug carriers. *J. Natl. Cancer Inst.*, 2006, vol. 98, 335-344
- **KOBAYASHI, H** ; **BRECHBIEL, M. W**. Dendrimer-based nanosized MRI contrast agents. *Curr. Pharm. Biotechnol.*, 2004, vol. 5, 539-549
- **KOBAYASHI, H** ; **BRECHBIEL, M. W**. Nano-sized MRI contrast agents with dendrimer cores. Adv. *Drug Delivery Rev*, 2005, vol. 57, 2271-2286
- Multilocus binding increases the relaxivity of protein-bound MRI contrast agents. **ZHANG, Z. et al.** Angew. Chem., Int. Ed.. 2005, vol. 44, 6766-6769
- **CARAVAN, P** ; **FARRAR, C. T.** ; **FRULLANO, L.** ; **UPPAL R**. Influence of molecular parameters and increasing magnetic field strength on relaxivity of gadolinium-and manganese-based T1 contrast agents. *Contrast Media Mol. Imaging*, 2009, vol. 4, 89-100
- **TÓTH, É. et al.** The role of water exchange in attaining maximum relaxivities for dendrimeric MRI contrast agents. *Chem. - Eur. J.*, 1996, vol. 2, 1607-1615
- **BOTTA, M.** ; **TEI, L**. Relaxivity enhancement in macromolecular and nanosized GdIII-based MRI contrast agents. *Eur. J. Inorg. Chem.*, 2012, vol. 2012, 1945-1960
- **UCHINO, H. et al.** Cisplatin-incorporating polymeric micelles (NC-6004) can reduce nephrotoxicity and neurotoxicity of cisplatin in rats. *Br. J. Cancer*, 2005, vol. 93, 678-687
- **HAMAGUCHI, T. et al.** NK105, a paclitaxel-incorporating micellar nanoparticle formulation, can extend in vivo antitumour activity and reduce the neurotoxicity of paclitaxel. *Br. J. Cancer*, 2005, vol. 92, 1240-1246
- **TAKAHASHI, A. et al.** NC-6300, an epirubicin-incorporating micelle, extends the antitumor effect and reduces the cardiotoxicity of epirubicin. *Cancer Sci*, 2013, vol. 104, 920-925
- **SONG, G. et al.** Emerging nanotechnology and advanced materials for cancer radiation therapy. *Adv. Mater.*, 2017, vol. 29, 1700996